(19) Europäisches Patentamt
European Patent Office
Office européen des brevets

(11) EP 4 271 163 A2

(12) EUROPÄISCHE PATENTANMELDUNG

(43) Veröffentlichungstag:
**01.11.2023 Patentblatt 2023/44**

(21) Anmeldenummer: **23197286.0**

(22) Anmeldetag: **13.11.2017**

(51) Internationale Patentklassifikation (IPC):
***H10K 85/60*** *(2023.01)*

(52) Gemeinsame Patentklassifikation (CPC):
**C09K 11/06; H10K 85/636; H10K 85/654; H10K 85/6572;** H10K 50/11; Y02E 10/549

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.11.2016 EP 16198684**

(62) Dokumentnummer(n) der früheren Anmeldung(en) nach Art. 76 EPÜ:
**17803843.6 / 3 538 623**

(71) Anmelder: **Merck Patent GmbH**
**64293 Darmstadt (DE)**

(72) Erfinder:
- **Stoessel, Philipp**
**64293 DARMSTADT (DE)**
- **Ehrenreich, Christian**
**64293 DARMSTADT (DE)**
- **Harbach, Philipp**
**64293 DARMSTADT (DE)**

Bemerkungen:
Diese Anmeldung ist am 14-09-2023 als Teilanmeldung zu der unter INID-Code 62 erwähnten Anmeldung eingereicht worden.

# (54) VERBINDUNGEN MIT EINER AKZEPTOR- UND EINER DONORGRUPPE

(57) Die vorliegende Erfindung beschreibt Verbindungen mit einer Akzeptor- und einer Donorgruppe, insbesondere zur Verwendung in elektronischen Vorrichtungen. Die Erfindung betrifft ferner ein Verfahren zur Herstellung der erfindungsgemäßen Verbindungen sowie elektronische Vorrichtungen, enthaltend diese.

EP 4 271 163 A2

## Beschreibung

Die vorliegende Erfindung beschreibt Verbindungen mit einer Akzeptor-und einer Donorgruppe, insbesondere zur Verwendung in elektronischen Vorrichtungen. Die Erfindung betrifft ferner ein Verfahren zur Herstellung der erfindungsgemäßen Verbindungen sowie elektronische Vorrichtungen enthaltend diese Verbindungen.

Lichtemittierende elektronische Bauteile halten zunehmend als Lichtquellen in den Alltag Einzug. Sie weisen als Vorteile einen niedrigen Energieverbrauch, eine kompakte Bauweise und eine im Vergleich zu herkömmlichen Leuchtmitteln deutlich längere Lebenszeit auf. Waren es zunächst anorganische lichtemittierende Dioden, die als Leuchtmittel - eingesetzt wurden, finden in jüngerer Zeit auch organische lichtemittierende Dioden (OLED) Anwendung in Gegenständen des täglichen Gebrauchs, wie beispielsweise in Displays für Mobiltelefone. Vorteile der OLEDs gegenüber ihren anorganischen Äquivalenten bestehen in ihrer einfacheren Verarbeitbarkeit. Die in OLEDs eingesetzten Materialien lassen sich in geeigneten Lösungsmitteln lösen. Die einzelnen Schichten der Bauelemente lassen sich beispielsweise mittels Druckverfahren auf ein Substrat aufbringen. Es ist daher vergleichsweise einfach, gebogene Displays herzustellen. Da die Schichten nicht kristallin ausgebildet sind, sind OLEDs im Vergleich zu LEDs auf der Basis anorganischer Verbindungen flexibel und lassen sich daher auch auf flexiblen Substraten für die Herstellung flexibler Bildschirme aufbringen. OLEDs emittieren selbst Licht und benötigen daher keine Hintergrundbeleuchtung, wie sie etwa bei Flüssigkristallanzeigen (CD) erforderlich ist. Dadurch lassen sich Bildschirme auf der Basis von OLEDs sehr dünn ausgestalten. Als weiterer Vorteil zeigen Displays auf der Basis von OLEDs einen sehr hohen Kontrast, da nicht angeregte OLEDs kein Licht aussenden, also vollkommen schwarz sind. Bei der Lichtausbeute konnten große Fortschritte erreicht werden, sodass es nun möglich ist, die zum Anregen elektronischer Zustände der OLED eingesetzte Energie nahezu vollständig in Licht umzuwandeln.

OLEDs sind aus mehreren Schichten aufgebaut. Auf einem Substrat, welches vorzugsweise transparent ist, beispielsweise einer Glasplatte oder einer transparenten Kunststofffolie, wird zunächst eine Anode aufgebracht. Hier wird meist Indium-Zinn-Oxid (ITO) verwendet, um eine transparente Anode zu erhalten. Auf der Anode wird dann eine Lochleitungsschicht (HTL = "hole transport layer") aufgebracht. Um den Übertritt von Löchern aus der Anode in die Lochleitungsschicht zu erleichtern, d.h. die Injektionsbarriere für Löcher zu erniedrigen, wird oft zunächst eine Zwischenschicht aus PEDOT/PSS (Poly(3,4-ethylendioxythiophen) dotiert mit Polystyrolsulfonat) auf die Anode aufgebracht, ehe dann die Lochtransportschicht aufgebracht wird. Auf die Lochtransportschicht folgt dann eine Emitterschicht (EL = "emitter layer"), welche einen Farbstoff in einem Anteil von beispielsweise 5 bis 10 Gew.-% enthält oder aus diesem aufgebaut ist. Auf diese kann dann eine Elektronenleitungsschicht (ETL = "electron transport layer") aufgebracht werden. Zum Abschluss wird im Hochvakuum eine Kathode aufgedampft, welche aus einem Metall oder einer Legierung mit geringer Elektronenaustrittsarbeit besteht, wie zum Beispiel Calcium, Aluminium, Barium, Ruthenium, Magnesium-Silber-Legierung. Als Schutzschicht und zur Verringerung der Injektionsbarriere für Elektronen kann zwischen Kathode und ETL noch eine sehr dünne Schicht aus Lithiumfluorid, Cäsiumfluorid oder Silber aufgedampft werden. Der Aufbau von OLEDs ist also relativ komplex und erfordert mehrere Arbeitsschritte. Ferner sind die verwendeten Materialien empfindlich gegenüber Feuchtigkeit und Sauerstoff, sodass der Aufbau verkapselt werden muss, um seine Funktionsfähigkeit der OLED über einen längeren Zeitraum zu gewährleisten.

Von der Kathode werden Elektronen und von der Anode Löcher als Ladungsträger in den Schichtaufbau injiziert. Elektronen und Löcher driften aufeinander zu und treffen sich in der Emitterschicht um einen gebundenen Zustand zu bilden, welcher als Exciton bezeichnet wird. Bei der Rekombination der Ladungsträger werden Singulett- und Triplett-Excitonen im Verhältnis 1 : 3 gebildet. Durch den Zerfall des Excitons wird die Energie für die Anregung des Farbstoffmoleküls bereitgestellt. Der angeregte Zustand kann dann wieder in den Grundzustand übergehen und dabei ein Photon aussenden. Bei einer Emission aus dem Singulett-Zustand, die als Fluoreszenz bezeichnet wird, führen nur maximal 25 % der erzeugten Excitonen zur Emission, während die aus dem Triplett-Zustand erfolgenden strahlungslosen Übergänge in Form von Wärme verloren gehen. Der Übergang aus dem Triplett Zustand $T_1$ in den Singulett-Zustand $S_0$ ist stark Spin-verboten und steht daher für eine Emission von Licht nicht zur Verfügung.

Bei einer Triplett-Emission, die als Phosphoreszenz bezeichnet wird, können durch Triplett-Harvesting theoretisch sämtliche Excitonen genutzt und als Licht emittiert werden. Dabei wird der mitangeregte und energetisch über dem Triplett-Zustand liegende Singulett-Zustand durch Inter-System-Crossing vollständig in den Triplett-Zustand relaxiert.

Als Triplett-Emitter werden meist Übergangsmetallkomplexverbindungen eingesetzt, in denen das Metall aus der dritten Periode der Übergangsmetalle gewählt wird, beispielsweise Iridium, Platin oder auch Gold. Durch die hohe Spin-Bahn-Kopplung der Edelmetall-Zentralionen wird der für optische Übergänge strikt verbotene Triplett-Singulett-Übergang erlaubt und die für eine Anwendung in OLEDs geeignete Emissionslebensdauer von wenigen $\mu$s erreicht. Bei wachsenden Stromdichten und der dadurch resultierenden Besetzung eines Großteils oder aller Triplettzustände $T_1$ der Emittermoleküle ergeben sich jedoch Sättigungseffekte und in der Folge können Ladungsträgerströme nicht mehr vollständig zur Besetzung angeregter Zustände genutzt werden, d.h. es ergeben sich ohmsche Verluste und die Effizienz des Emitters sinkt ("Roll-off"-Verhalten).

Singulett-Emitter weisen eine deutlich geringere Emissionslebensdauer im Bereich von Nanosekunden auf, sodass Roll-off-Effekte in deutlich geringerem Maß bzw. nicht auftreten. Die Effizienz der Emitter bzw. die Quantenausbeute lässt sich steigern, indem auch die Triplett-Zustände für die Emission von Licht genutzt werden. Dazu muss der Energieunterschied zwischen dem höchsten besetzten Triplett-Zustand $T_1$ und dem untersten angeregten Singulett-Zustand $S_1$ allerdings klein sein, sodass bei Raumtemperatur eine thermische Rückbesetzung des Singulett-Zustandes $S_1$ aus dem Triplett-Zustand $T_1$ erfolgen kann. Ferner muss eine starke Spin-Bahn-Kopplung ermöglicht werden, sodass durch Inter-System-Crossing der an sich spin-verbotene Übergang $T_1 \rightarrow S_1$ möglich wird. Die Emissionslebensdauer für diesen Übergang sollte im Bereich von weniger als 1 $\mu$s liegen, beispielsweise bei etwa 100 bis 600 ns.

Beim Singulett-Harvesting erfolgt also wie beim Triplett-Harvesting eine Besetzung des niedrigsten angeregten Triplett-Zustands. Die Emission erfolgt jedoch nicht aus dem tiefsten Triplett-Zustand $T_1$, sondern über eine thermische Rückbesetzung aus dem niedrigsten angeregten Singulett-Zustand $S_1$, sodass die sonst verlorene Anregungsenergie für den Triplett-Zustand nahezu vollständig für die Lichtemission zur Verfügung steht.

Dieser Vorgang wird als thermisch aktivierte verzögerte Fluoreszenz (TADF = "thermally activated delayed fluorescence") bezeichnet und wird beispielsweise von B. H. Uoyama et al., Nature 2012, Vol. 492, 234 beschrieben. Um diesen Prozess zu ermöglichen, ist im Emitter ein vergleichsweise kleiner Singulett-Triplett-Abstand $\Delta E(S_1 - T_1)$ von zum Beispiel weniger als etwa 2000 $cm^{-1}$ nötig. Um den an sich spinverbotenen Übergang $T_1 \rightarrow S_1$ zu öffnen, kann neben dem Emitter eine weitere Verbindung in der Matrix vorgesehen werden, die eine starke Spin-Bahn-Kopplung aufweist, sodass über die räumliche Nähe und die damit mögliche Wechselwirkung zwischen den Molekülen ein Inter-System-Crossing ermöglicht wird, oder die Spin-Bahn-Kopplung wird über ein im Emitter enthaltenes Metallatom erzeugt.

Als Emitter eignen sich vor allem Moleküle, die einen hohen Charge-Transfer-Charakter zeigen, wie z.B. Kupferverbindungen. Eine stark lumineszierende Verbindung, die eine thermisch aktivierte verzögerte Fluoreszenz zeigen, muss also so gestaltet werden, dass sie einen sehr kleinen Abstand $\Delta E(S_1 - T_1)$ bei relativ kurzen Abklingraten von > $10^6$ $s^{-1}$ aufweist, sodass konkurrierende Abklingwege, bei welchen keine Emission von Licht erfolgt, zurücktreten.

Als weiteres lichtemittierendes elektronisches Bauelement wurden organische lichtemittierende elektrochemische Zellen (OLEC = "organic light emitting electrochemical cell, auch LEC oder LEEC) entwickelt. OLECs wurden erstmals von Ouibing Pei et al., Science, 1995, 296, 1086 - 1088 beschrieben, wo auch die grundlegenden Prinzipien erläutert sind.

OLECs zeigen im Vergleich zu OLEDs einen einfacheren Aufbau und lassen sich auch einfacher herstellen. So können OLECs im Vergleich zu OLEDs eine geringere Anzahl von Schichten aufweisen. Ferner kann die aktive Schicht bei OLECs dicker ausgeführt werden, sodass einfachere Verfahren zur Herstellung der aktiven, lichtemittierenden Schicht eingeetzt werden können. Die aktive Licht emittierende Schicht kann bei OLECs in einer Stärke von mehreren Mikrometern bis hin zu mehreren 10 Mikrometern ausgeführt werden. Zum Aufbringen der aktiven Schicht können daher Verfahren wie Ink-jet-printing, Screen-printing oder Sprühbeschichten eingesetzt werden, die sich auch für eine kostengünstige Massenproduktion eignen. OLECs zeigen eine im Vergleich zu OLEDs geringere Empfindlichkeit gegenüber Unregelmäßigkeiten der Substratoberfläche sowie gegenüber Defekten in der aktiven Schicht. OLECs sollten daher eine bessere Eignung zur Herstellung großflächiger lichtemittierender Vorrichtungen aufweisen.

Eine OLEC umfasst zwei Elektroden sowie eine zwischen Kathode und Anode angeordnete aktive Schicht. Die aktive Schicht umfasst einen Licht emittierenden organischen Halbleiter sowie einen Elektrolyt, welcher bewegliche Ionen zur Verfügung stellt. Um einen Lichtaustritt zu ermöglichen, sollte zumindest eine der Elektroden transparent sein.

Als Licht emittierende organische Halbleiter können konjugierte Copolymere oder ionische Übergangsmetallkomplexe eingesetzt werden, wie sie beispielsweise auch in OLEDs verwendet werden. Beispielhafte Übergangsmetallkomplexverbindungen sind Tris(2-phenylpyridin)iridium ($Ir(ppy)_3$) oder Tris(8-hydroxychinolin)aluminium (Alqs). Kleine Moleküle bieten den Vorteil, dass sie sich leichter verarbeiten lassen, da sie leichter in einem geeigneten Lösungsmittel gelöst werden können. Allerdings kann bei der Verarbeitung dieser Lösungen die Schwierigkeit schlechter Filmbildungseigenschaften auftreten.

Die Fähigkeit des organischen Halbleiters, Licht zu emittieren, wird von der Energie des höchsten besetzten Molekülorbitals (HOMO), des niedrigsten freien Molekülorbital (LUMO) sowie deren relative Lage bestimmt. Bei organischen Halbleitern lassen sich HOMO und LUMO jedoch in ihrer Energie einstellen, indem am Molekül Seitenketten eingeführt werden oder Copolymere aus verschiedenen organischen Halbleitern hergestellt werden, sodass in der Copolymerkette verschiedene organische Halbleiter vorhanden sind. Auf diese Weise lässt sich auch die Löslichkeit des organischen Halbleiters sowie dessen Filmbildungseigenschaften beeinflussen. Die Wechselwirkung zwischen den Orbitalen, durch welche die Lage der Energiezustände bestimmt wird, wird jedoch auch durch die Geometrie des Polymers sowie die relative Lage zu anderen Molekülen beeinflusst. Bei Filmen, die aus Lösungen hergestellt werden, kann daher ein Wechsel des Lösungsmittels die Lage des HOMO und des LUMO beeinflussen, sodass die Emission eines organischen, Licht-emittierenden Bauteils wegen des amorphen Charakters des Films ein relativ breites Spektrum aufweist.

Die meisten Metalle weisen eine relativ hohe Austrittsarbeit auf, weshalb an den Elektroden ein erheblicher Energieaufwand besteht, um Elektronen aus dem Elektrodenmaterial in den organischen Halbleiter zu injizieren. Bei Verwendung von Alkali- oder Erdalkalimetallen weisen diese Metalle zwar eine niedrige Austrittsarbeit auf, sodass die

Energiedifferenz zwischen der Kathode und dem LUMO des organischen Halbleiters verringert wird. Diese Metalle sind gegenüber Sauerstoff und Wasser jedoch sehr reaktiv, sodass die Herstellung der elektronischen Bauteile aufwändig ist.

Bei organischen lichtemittierenden elektrochemischen Zellen werden bewegliche Ionen genutzt, um den Ladungsübertritt von der Elektrode in die Aktivschicht zu ermöglichen. Wird zwischen Kathode und Anode eine Spannung angelegt, reichern sich an der Kathode positive Ionen und an der Anode negative Ionen an, sodass sich eine elektrische Doppelschicht ausbildet. Die elektrischen Doppelschichten sind sehr dünn, wodurch sich unabhängig von der Schichtdicke der Aktivschicht an der Grenzfläche zur Elektrode ein starker elektrischer Feldgradient aufbaut. Besteht zwischen den Elektroden eine ausreichend große Potentialdifferenz, ermöglicht die elektrische Doppelschicht eine effiziente Injektion von Ladungsträgern in das HOMO bzw. LUMO des organischen Halbleiters. Die Injektion von Ladungsträgern wird durch eine gegenläufige Bewegung geladener Ionen kompensiert. Die Injektion von Elektronen an der Kathode bewirkt daher eine Anreicherung positiver Ionen und verursacht damit eine n-Dotierung. Die Extraktion von Elektronen an der Anode, bzw. die Injektion von Löchern an der Anode wird entsprechend von negativ geladenen Ionen kompensiert und bewirkt eine p-Dotierung. Durch die elektrochemische Dotierung wird der Transport von Elektronen bzw. Löchern im organischen Halbleiter erleichtert. Auch bei dickeren Aktivschichten, die einen größeren Elektrodenabstand bewirken, kann daher bei niedriger Potentialdifferenz ein wirksamer Ladungstransport bewirkt werden.

Die elektrochemisch dotierten Bereiche wachsen in Richtung aufeinander zu bis sie schließlich aufeinandertreffen und in einer sehr dünnen undotierten Schicht eine pn-Rekombination bewirkt wird. Der Mechanismus des Ladungstransports in OLECs und OLEDs unterscheidet sich jedoch maßgeblich. Während die Ladungen in OLECs mit Hilfe von mobilen Ionen transportiert werden, erfolgt der Ladungstransport in OLEDs durch Hopping von Elektronen/Löchern von mehr oder weniger stationären Molekülen. Ferner enthalten OLEDs oft weitere Schichten (bspw. Elektronen- und Lochtransportschichten) mit unterschiedlichen Materialien. Die OLEC erfordert bei der Herstellung hingegen nur die Bereitstellung einer einzelnen homogenen Aktivschicht, da sich die Schichten für den Elektronen- bzw. Lochtransport nach Anlegen einer Potentialdifferenz zwischen den Elektroden von selbst in der Aktivschicht ausbilden.

Im Vergleich zu OLEDs hängt daher die Funktion der OLEC nicht von der Austrittsarbeit der Elektroden ab. Somit können beide Elektroden aus demselben Material hergestellt werden. Es ist somit auch möglich, eine komplett metallfreie OLEC herzustellen.

OLEDs sind Dioden mit einer Vorwärts- und einer Rückwärtsrichtung beim Ladungstransport, d.h. die Strom-Spannungskurven sind unsymmetrisch. OLECs sind im Wesentlichen eine elektrolytische Zelle. Nach Anlegen einer Potentialdifferenz wird der Elektrolyt an der Anode oxidiert und an der Kathode reduziert.

OLECs werden typischerweise mit einer einzigen Lösung hergestellt, die Licht emittierende und Ladung transportierende konjugierte Polymere enthält, wie beispielsweise Polyphenylen-vinylen-polymere, Polythiophen-Polymere oder Polyfluoren-Polymere, sowie ein Elektrolytsystem, das bewegliche ionische Dotiermittel, wie beispielsweise Lithiumtriflate, sowie den Elektrolyten bildende Verbindungen enthält, wie beispielsweise Polyethylenoxid.

Licht emittierende elektrochemische Zellen sind beispielsweise aus der WO 2013/173845 bekannt.

Verwendet man als Farbstoff einen Singulett-Emitter, geht analog zu den Prozessen, wie sie oben für OLEDs beschrieben wurden, die bei Triplett-Übergängen freiwerdende Energie in Form von Wärme verloren. Da nur etwa 25 % der Rekombinationen von Ladungsträgern zu Singulett-Excitonen führen, bedeutet dies dass 75 % aller Ladungsträger, die in die aktive Schicht injiziert werden, für die Lichterzeugung verloren gehen. Die Effizienz in Bezug auf die Lichtausbeute ist bei derartigen LECs also nicht zufriedenstellend. Im Stand der Technik ist bekannt, dass OLEDs den OLECs hinsichtlich Lebensdauer und Effizienz deutlich überlegen sind.

Der Erfindung lag daher die Aufgabe zu Grunde, organische Elektrolumineszenzvorrichtungen (OLEDs, PLEDs) oder organische lichtemittierende elektrochemische Zellen (OLECs) zur Verfügung zu stellen, welche mit der eingesetzten elektrischen Energie eine hohe Lichtausbeute erzielen und die aus dem Stand der Technik bekannten Nachteile überwinden.

Eine weitere Aufgabe der vorliegenden Erfindung ist die Bereitstellung von Verbindungen, welche sich für den Einsatz in einer organischen elektronischen Vorrichtung, insbesondere in einer organischen Elektrolumineszenzvorrichtung oder einer organischen elektrochemischen Zellen eignen, und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung Verbindungen zur Verfügung zu stellen, die zu hoher Lebensdauer, guter Effizienz und geringer Betriebsspannung führen. Gerade auch die Eigenschaften der Matrixmaterialien haben einen wesentlichen Einfluss auf die Lebensdauer und die Effizienz der organischen Elektrolumineszenzvorrichtung.

Eine weitere Aufgabe der vorliegenden Erfindung kann darin gesehen werden, Verbindungen bereitzustellen, welche sich für den Einsatz in einer phosphoreszierenden oder fluoreszierenden OLED eignen, insbesondere als Matrixmaterial. Insbesondere ist es eine Aufgabe der vorliegenden Erfindung, Matrixmaterialien bereitzustellen, welche sich für rot, gelb und grün phosphoreszierende OLEDs und gegebenenfalls auch für blau phosphoreszierende OLEDs eignen.

Weiterhin sollten sich die Verbindungen möglichst einfach verarbeiten lassen, insbesondere eine gute Löslichkeit und Filmbildung zeigen. Beispielsweise sollten die Verbindungen eine erhöhte Oxidationsstabilität und eine verbesserte Glasübergangstemperatur zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, nachfolgend näher beschriebene Verbindungen diese Aufgaben lösen und den Nachteil aus dem Stand der Technik beseitigen. Die Verwendung der Verbindungen führt zu sehr guten Eigenschaften organischer elektronischer Vorrichtungen, insbesondere von organischen Elektrolumineszenzvorrichtungen, insbesondere hinsichtlich der Lebensdauer, der Effizienz und der Betriebsspannung. Elektronische Vorrichtungen, insbesondere organische Elektrolumineszenzvorrichtungen oder organische lichtemittierende elektrochemische Zellen, welche derartige Verbindungen enthalten, sowie die entsprechenden bevorzugten Ausfürhungsformen sind daher Gegenstand der vorliegenden Erfindung.

Gegenstand der vorliegenden Erfindung ist daher eine Verbindung, umfassend mindestens eine Struktur gemäß der folgenden Formel (I),

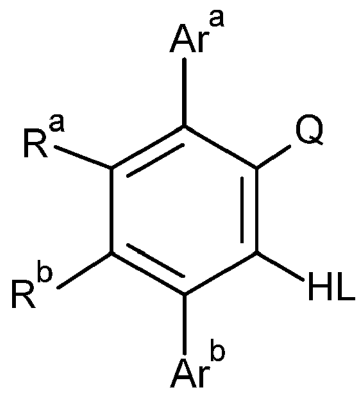

Formel (I)

wobei für die verwendeten Symbole gilt:

Q ist eine Akzeptorgruppe, umfassend ein aromatisches oder heteroaromatisches Ringsystem mit 5 bis 60 aromatischen Ringatomen, welches durch einen oder mehrere Reste $R^1$ substituiert sein kann;

HL ist eine Donorgruppe;

$Ar^a$, $Ar^b$ ist gleich oder verschieden ein aromatisches oder heteroaromatisches Ringsystem mit 5 bis 60 aromatischen, vorzugsweise 5 bis 40 aromatischen Ringatomen, welches durch einen oder mehrere Reste $R^1$ substituiert sein kann;

$R^a$, $R^b$ ist gleich oder verschieden H, D, F, Cl, Br, I, $B(OR^1)_2$, CHO, $C(=O)R^1$, $CR^1=C(R^1)_2$, CN, $C(=O)OR^1$, $C(=O)N(R^1)_2$, $Si(R^1)_3$, $N(R^1)_2$, $NO_2$, $P(=O)(R^1)_2$, $OSO_2R^1$, $OR^1$, $S(=O)R^1$, $S(=O)_2R^1$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^1$ substituiert sein kann, wobei eine oder mehrere nicht benachbarte $CH_2$-Gruppen durch $-R^1C=CR^1-$, -C=C-, $Si(R^1)_2$, $Ge(R^1)_2$, $Sn(R^1)_2$, C=O, C=S, C=Se, $C=NR^1$, $NR^1$, $P(=O)(R^1)$, -C(=O)O-, $-C(=O)NR^1-$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen, vorzugsweise 5 bis 40 aromatischen Ringatomen, das jeweils durch einen oder mehrere Reste $R^1$ substituiert sein kann, oder eine Aryloxy- oder Heteroaryloxygruppe mit 5 bis 60 aromatischen, vorzugsweise 5 bis 40 aromatischen Ringatomen, die durch einen oder mehrere Reste $R^1$ substituiert sein kann, oder eine Aralkyl- oder Heteroaralkylgruppe mit 5 bis 60 aromatischen, vorzugsweise 5 bis 40 aromatischen Ringatomen, die durch einen oder mehrere Reste $R^1$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^1$ substituiert sein kann; oder eine Kombination dieser Systeme, dabei können die Reste

$R^a$ und $R^b$ miteinander oder mit der Gruppe $Ar^a$ oder $Ar^b$ ein mono- oder polycyclisches, aliphatisches, heteroaliphatisches, aromatisches oder heteroaromatisches Ringsystem bilden;

$R^1$ ist bei jedem Auftreten gleich oder verschieden H, D, F, Cl, Br, I, $B(OR^2)_2$, CHO, $C(=O)R^2$, $CR^2=C(R^2)_2$, CN, $C(=O)OR^2$, $C(=O)N(R^2)_2$, $Si(R^2)_3$, $N(R^2)_2$, $NO_2$, $P(=O)(R^2)_2$, $OSO_2R^2$, $OR^2$, $S(=O)R^2$, $S(=O)_2R^2$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, -C=C-, $Si(R^2)_2$, $Ge(R^2)_2$, $Sn(R^2)_2$, C=O, C=S, C=Se, $C=NR^2$, $NR^2$, $P(=O)(R^2)$, -C(=O)O-, $-C(=O)NR^2-$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen, vorzugsweise 5 bis 40 aromatischen Ringatomen, das jeweils durch einen oder mehrere Reste $R^2$ substituiert sein kann, oder eine Aryloxy- oder Heteroaryloxygruppe mit 5 bis 60 aromatischen, vorzugsweise 5 bis 40 aromatischen Ringatomen, die durch einen oder mehrere Reste $R^2$ substituiert sein kann, oder eine Aralkyl- oder Heteroaralkylgruppe mit 5 bis 60 aromatischen, vorzugsweise 5 bis 40 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann; oder eine Kombination dieser Systeme; dabei können zwei oder mehrere, vorzugsweise benachbarte Reste $R^1$ miteinander ein mono- oder polycyclisches, aliphatisches, heteroaliphatisches, aromatisches oder heteroaromatisches Ringsystem bilden;

$R^2$ ist bei jedem Auftreten gleich oder verschieden H, D, F, Cl, Br, I, $B(OR^3)_2$, CHO, $C(=O)R^3$, $CR^3=C(R^3)_2$, CN, $C(=O)OR^3$, $C(=O)N(R^3)_2$, $Si(R^3)_3$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, -C=C-, $Si(R^3)_2$, $Si(R^3)_2$, $Ge(R^3)_2$, $Sn(R^3)_2$, C=O, C=S, C=Se, $C=NR^3$, $NR^3$, $P(=O)(R^3)$, -C(=O)O-, $-C(=O)NR^3-$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen, vorzugsweise 5 bis 40 aromatischen Ringatomen, das jeweils durch einen oder mehrere Reste $R^3$ substituiert sein kann, oder eine Aryloxy- oder Heteroaryloxygruppe mit 5 bis 60 aromatischen, vorzugsweise 5 bis 40 aromatischen Ringatomen, die durch einen oder mehrere Reste $R^3$ substituiert sein kann, oder eine Aralkyl- oder Heteroaralkylgruppe mit 5 bis 60 aromatischen, vorzugsweise 5 bis 40 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere, vorzugsweise benachbarte Substituenten $R^2$ auch miteinander ein mono- oder polycyclisches, aliphatisches, heteroaliphatisches, aromatisches oder heteroaromatisches Ringsystem bilden;

$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere, vorzugsweise benachbarte Substituenten $R^3$ auch miteinander ein mono- oder polycyclisches, aliphatisches, heteroaliphatisches, aromatisches oder heteroaromatisches Ringsystem bilden.

Benachbarte Kohlenstoffatome im Sinne der vorliegenden Erfindung sind Kohlenstoffatome, die direkt miteinander verknüpft sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

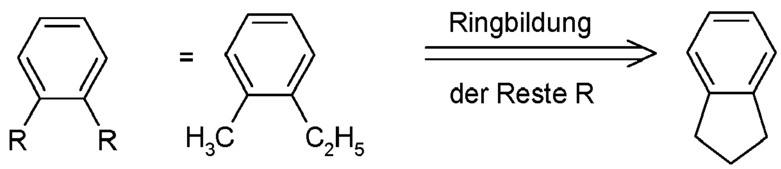


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

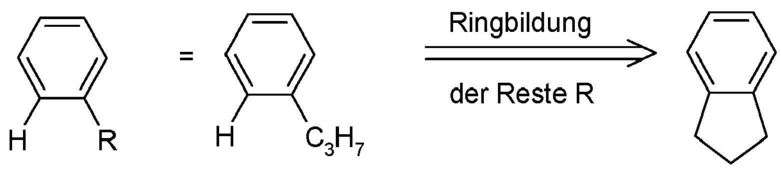


Eine kondensierte Arylgruppe, ein kondensiertes aromatisches Ringsystem oder ein kondensiertes heteroaromatisches Ringsystem im Sinne der vorliegenden Erfindung ist eine Gruppe, in der zwei oder mehr aromatische Gruppen über eine gemeinsame Kante aneinander ankondensiert, d. h. anelliert, sind, so dass beispielsweise zwei C-Atome zu den mindenstens zwei aromatischen oder heteroaromatischen Ringen zugehören, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen. Entsprechende Definitionen gelten für Heteroarylgruppen sowie für kondensierte Ringsysteme, die auch Heteroatome enthalten können, jedoch nicht müssen.

Eine Arylgruppe im Sinne dieser Erfindung enthält 6 bis 60 C-Atome, vorzugsweise 6 bis 40 C-Atome; eine Heteroarylgruppe im Sinne dieser Erfindung enthält 2 bis 60 C-Atome, vorzugsweise 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl-oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60, vorzugsweise 6 bis 40 C-Atome im Ringsystem. Ein heteroaromatisches Ringsystem im Sinne dieser Erfindung enthält 1 bis 60, vorzugsweise 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nichtaromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{20}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 bis 60. vorzugsweise 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Gemäß einer bevorzugten Ausgestaltung sind erfindungsgemäße Verbindungen durch Strukturen gemäß Formel (I) darstellbar. Vorzugsweise weisen Verbindungen, umfassend Strukturen gemäß Formel (I) ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Die Gruppe stellt HL eine Donorgruppe dar, wobei diese in der Fachwelt, insbesondere in Bezug auf TADF-Materialien, bekannt ist.

Unter der Donorgruppe D (auch Donorsubstituent genannt) wird vorliegend eine Gruppe verstanden, die eine Elektronendonorgruppe darstellt. Was unter einer Donorgruppe verstanden wird, ist dem Fachmann gut bekannt. Es ist bevorzugt, wenn die Donorgruppe einen positiven induktiven Effekt (+I) und/oder einen positiven mesomeren Effekt (+M) aufweist. Die Bestimmung der Parameter mit Hilfe der Hammett-Gleichung ist dem Fachmann gut bekannt. Geeignete Donorsubstituenten sind insbesondere Diaryl- bzw. -heteroarylaminogruppen sowie Carbazolgruppen bzw. Carbazolderivate, wie Indenocarbazole oder Indolocarbazole. Dabei können diese Gruppen auch weiter substituiert sein.

Gemäß einer weiteren Ausführungsform ist die Donorgruppe ausgewählt aus Arylaminogruppen, vorzugsweise Di- oder Triarylaminogruppen, Heteroarylaminogruppen, vorzugsweise Di- oder Triheteroarylaminogruppen, Carbazolgruppen, wobei Carbazolgruppen bevorzugt sind.

Vorzugsweise kann vorgesehen sein, dass die Donorgruppe HL eine Gruppe umfasst, bevorzugt für eine Gruppe steht, die ausgewählt ist aus den Formeln (H-1) bis (H-3)

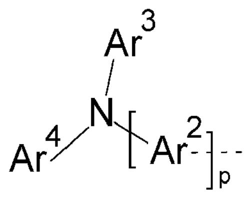

Formel (H-1)

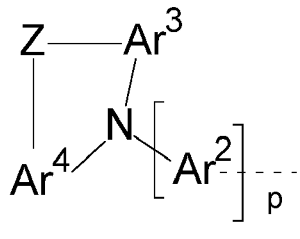

Formel (H-2)

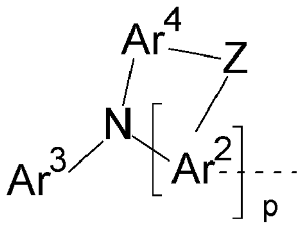

Formel (H-3)

, wobei die gestrichelte Bindung die Anbindungsposition markiert und $Ar^2$, $Ar^3$, $Ar^4$ jeweils unabhängig eine Arylgruppe mit 6 bis 40 C-Atomen oder eine Heteroarylgruppe mit 3 bis 40 C-Atomen ist, welche jeweils durch einen oder mehrere Reste $R^1$ substituiert sein kann;

p 0, 1, 2, 3, 4, 5 oder 6, vorzugsweise 0, 1, 2 oder 3 und besonders bevorzugt 0, 1 oder 2 ist und

Z für $CR^1_2$, $SiR^1_2$, C=O, N-$Ar^1$, $BR^1$, $PR^1$, $POR^1$, SO, $SO_2$, Se, O oder S, vorzugsweise $CR^1_2$, N-$Ar^1$, O oder S steht, wobei der Rest $R^1$ die in Anspruch 1 genannte Bedeutung aufweist und $Ar^1$ ein aromatisches oder heteroaromatisches Ringsystem mit 5 bis 60 aromatischen, vorzugsweise 5 bis 40 aromatischen Ringatomen, das jeweils mit einem oder mehreren Resten $R^1$ substituiert sein kann, eine Aryloxygruppe mit 5 bis 60 aromatischen, vorzugsweise 5 bis 40 aromatischen Ringatomen, die mit einem oder mehreren Resten $R^1$ substituiert sein kann, oder einer Aralkylgruppe mit 5 bis 60 aromatischen, vorzugsweise 5 bis 40 aromatischen Ringatomen, die jeweils mit einem oder mehreren Resten $R^1$ substituiert sein kann, darstellt, wobei optional zwei oder mehr, vorzugsweise benachbarte Substituenten $R^1$ ein mono- oder polycyclisches, aliphatisches, heteroaliphatisches, aromatisches oder heteroaromatisches Ringsystem bilden können, das mit einem oder mehreren Resten $R^2$ substituiert sein kann.

Weiterhin kann vorgesehen sein, dass die Donorgruppe HL eine Gruppe umfasst, bevorzugt für eine Gruppe steht, die ausgewählt ist aus den Formeln (H-4) bis (H-26)

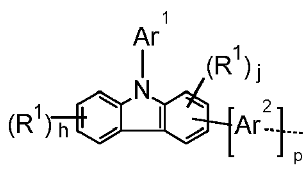

Formel (H-4)

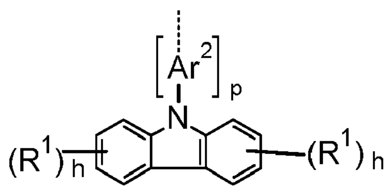

Formel (H-5)

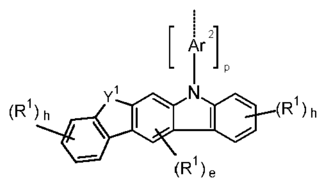

Formel (H-6)

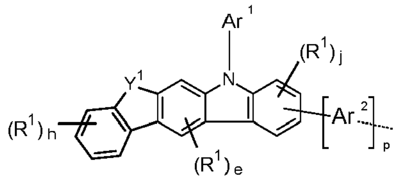

Formel (H-7)

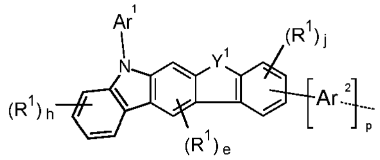

Formel (H-8)

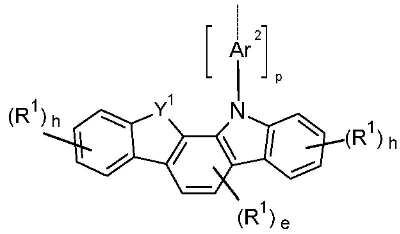

Formel (H-9)

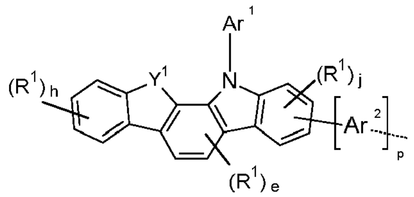

Formel (H-10)

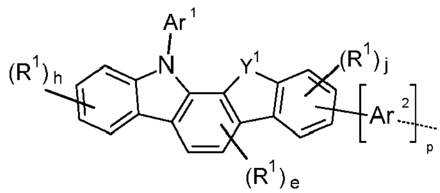

Formel (H-11)

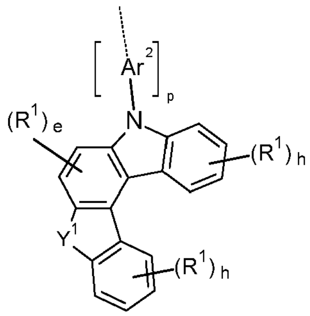

Formel (H-12)

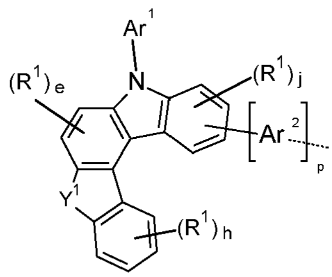

Formel (H-13)

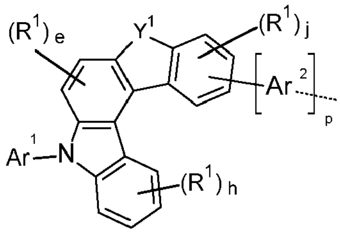

Formel (H-14)

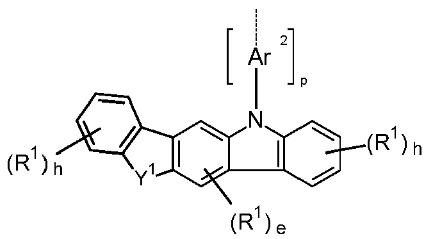

Formel (H-15)

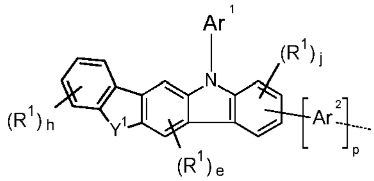

Formel (H-16)

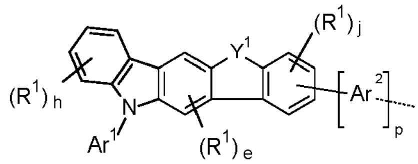

Formel (H-17)

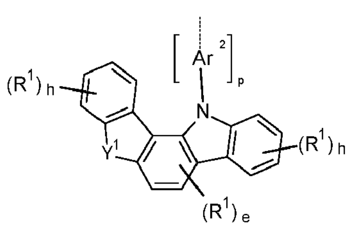

Formel (H-18)

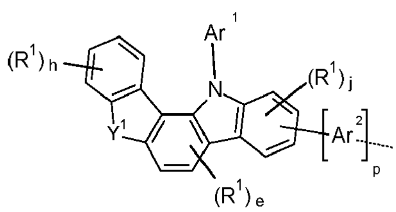

Formel (H-19)

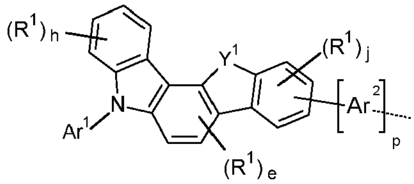

Formel (H-20)

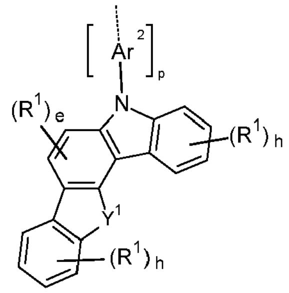

Formel (H-21)

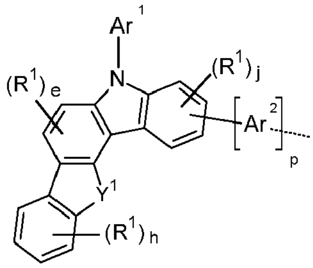

Formel (H-22)

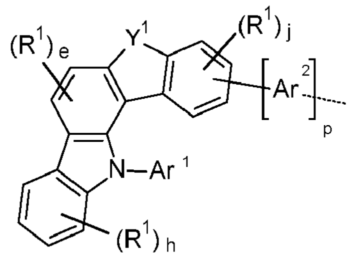

Formel (H-23)

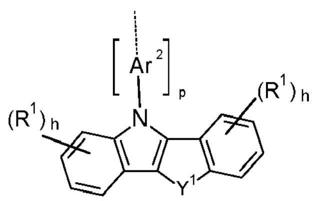

Formel (H-24)

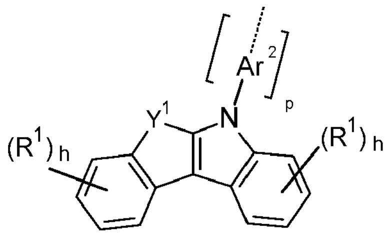

Formel (H-25)

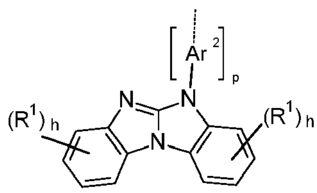

Formel (H-26)

, wobei $Y^1$ O, S, $C(R^1)_2$ oder $NAr^1$ darstellt, die gestrichelte Bindung die Anbindungsposition markiert, e 0, 1 oder 2 ist, j 0, 1, 2 oder 3 ist, h 0, 1, 2, 3 oder 4 ist, p 0, 1, 2, 3, 4, 5 oder 6, vorzugsweise 0, 1, 2 oder 3 und besonders bevorzugt 0, 1 oder 2 ist, $Ar^1$ und $Ar^2$ die zuvor, insbesondere für Formel (H-1) oder (H-2) und $R^1$ die zuvor, insbesondere für Formel (I) genannte Bedeutung aufweist.

Von den Gruppen (H-1) bis (H-26) sind Carbazolgruppen, insbesondere die Gruppen (H-4) bis (H-26) bevorzugt.

Vorzugsweise kann die Gruppe $Ar^2$ in den Formeln (H-1) bis (H-26) für eine verbindende Struktur der Formel (LAr-1) stehen

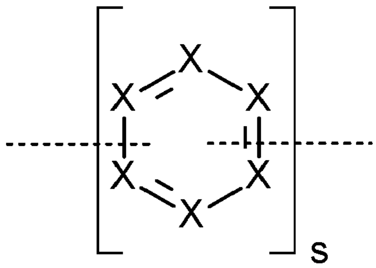

Formel (LAr-1)

wobei X bei jedem Auftreten gleich oder verschieden N oder $CR^1$, vorzugsweise $CR^1$, oder C ist falls an X eine Gruppe bindet; die gestrichelte Bindung die Anbindungsposition markiert und s für 0, 1, 2, 3, 4, 5 oder 6, vorzugsweise für 0, 1, 2 oder 3, besonders bevorzugt für 0, 1 oder 2 und speziell bevorzugt für 0 oder 1 steht, wobei $R^1$ die zuvor, insbesondere für Formel (I) genannte Bedeutung aufweist. Bevorzugt können die zwei Anbindungspositionen der in Formel (LAr-1) dargestellten Gruppe in para-Position stehen. Weiterhin ist bevorzugt, dass der Index p in Formel (H-1) bis (H-26) 1 ist und der Index s in Formel (LAr-1) für 0, 1, 2, 3, 4, 5 oder 6, vorzugsweise für 0, 1, 2 oder 3, besonders bevorzugt für 0, 1 oder 2 und speziell bevorzugt für 0 oder 1 steht.

Bevorzugt stehen höchstens zwei Gruppen X in Formel (LAr-1) pro Ring für N. Besonders bevorzugt umfasst die verbindende Struktur der Formel (LAr-1) höchstens zwei, besonders bevorzugt höchstens ein und speziell bevorzugt kein Stickstoffatom. Darüber hinaus sind Verbindungen bevorzugt, die dadurch gekennzeichnet sind, dass in Formel (LAr-1) pro Ring, mindestens vier, vorzugsweise alle X für $CR^1$ stehen, wobei vorzugsweise höchstens 4, besonders bevorzugt höchstens 3 und speziell bevorzugt höchstens 2 der Gruppen $CR^1$ für die X steht, ungleich der Gruppe CH ist. Besonders bevorzugt umfasst die verbindende Struktur der Formel (LAr-1) höchstens zwei, besonders bevorzugt höchstens ein und speziell bevorzugt keinen Rest $R^1$, der ungleich H ist.

Die Gruppe Q in Formel (I) stellt eine Akzeptorgruppe, umfassend ein aromatisches oder heteroaromatisches Ringsystem mit 5 bis 60 aromatischen Ringatomen, welches durch einen oder mehrere Reste $R^1$ substituiert sein kann, dar. Akzeptorgruppen mit diesen Eigenschaften sind in der Fachwelt, insbesondere in Bezug auf TADF-Materialien, weithin bekannt. Es ist bevorzugt, wenn die Akzeptorgruppe einen negativen induktiven Effekt (-I) und/oder einen negativen mesomeren Effekt (-M) aufweist. Die Bestimmung der Parameter mit Hilfe der Hammett-Gleichung ist dem Fachmann auch gut bekannt. Geeignete Akzeptorsubstituenten sind insbesondere elektronenarme Heteroarylgruppen und Arylgruppen, die mit elektronenziehenden Substituenten substituiert sind, wobei diese Gruppen auch weiter substituiert sein können. Beispiele bevorzugter elektronenarmer Heteroarylgruppen sind ausgewählt aus der Gruppe bestehend aus Triazinen, Pyrimidinen, Phosphinoxiden und Ketonen.

Weiterhin zeigen Verbindungen, umfassend mindestens eine Struktur gemäß Formel (I) oder deren bevorzugte Ausführungsformen überraschende Vorteile, bei denen die Akzeptorgruppe Q mindestens eine Struktur umfasst, die aus der Gruppe Pyridine, Pyrimidine, Pyrazine, Pyridazine, Triazine, Chinazoline, Chinoxaline, Chinoline, Isochinoline, Imidazole und/oder Benzimidazole ausgewählt ist, wobei Pyrimidine, Triazine und Chinazoline besonders bevorzugt sind.

In einer bevorzugten Ausgestaltung der vorliegenden Erfindung kann vorgesehen sein, dass die Akzeptorgruppe für eine Gruppe steht, die durch die Formel (QL) darstellbar ist,

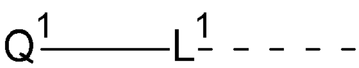

Formel (QL)

worin L' eine Bindung oder ein aromatisches oder heteroaromatisches Ringsystem mit 5 bis 60 aromatischen, vorzugsweise 5 bis 40 aromatischen Ringatomen darstellt, welches durch einen oder mehrere Reste $R^1$ substituiert sein kann, und $Q^1$ eine elektronenziehende Gruppe ist, wobei $R^1$ die zuvor, insbesondere für Formel (I) genannte Bedeutung aufweist.

Vorzugsweise steht die Gruppe L' für eine verbindende Struktur der Formel (LAr-2)

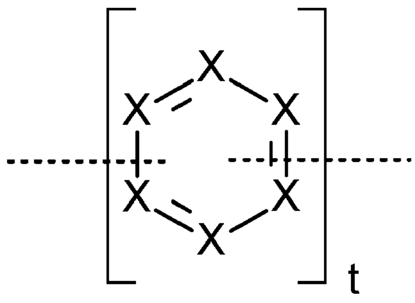

Formel (LAr-2)

wobei X bei jedem Auftreten gleich oder verschieden N oder $CR^1$, vorzugsweise $CR^1$, oder C ist falls an X eine Gruppe bindet;

die gestrichelte Bindung die Anbindungsposition markiert und t für 0, 1, 2, 3, 4, 5 oder 6, vorzugsweise für 0, 1, 2 oder 3, besonders bevorzugt für 0, 1 oder 2 und speziell bevorzugt für 0 oder 1 steht, wobei $R^1$ die zuvor, insbesondere für Formel (I) dargelegte Bedeutung aufweist. Vorzugsweise können die zwei Anbindungspositionen der in Formel (LAr-2) dargestellten verbindenden Struktur in para-Position stehen.

Bevorzugt stehen höchstens zwei Gruppen X in Formel (LAr-2) pro Ring für N. Besonders bevorzugt umfasst die verbindende Struktur der Formel (LAr-2) höchstens zwei, besonders bevorzugt höchstens ein und speziell bevorzugt kein Stickstoffatom. Darüber hinaus sind Verbindungen bevorzugt, die dadurch gekennzeichnet sind, dass in Formel (LAr-2) pro Ring, mindestens vier, vorzugsweise alle X für $CR^1$ stehen, wobei vorzugsweise höchstens 4, besonders bevorzugt höchstens 3 und speziell bevorzugt höchstens 2 der Gruppen $CR^1$ für die X steht, ungleich der Gruppe CH ist. Besonders bevorzugt umfasst die verbindende Struktur der Formel (LAr-2) höchstens zwei, besonders bevorzugt höchstens ein und speziell bevorzugt keinen Rest $R^1$, der ungleich H ist.

Ferner kann vorgesehen sein, dass der Index p in Formel (H-1) bis (H-26) für 0, 1 oder 2 steht und der Index t in Formel (LAr-2) für 0, 1 oder 2 steht. Vorzugsweise kann der Index s in Formel (LAr-1) für 0, 1 oder 2 stehen und der Index t in Formel (LAr-2) für für 0, 1 oder 2 stehen. Weiterhin kann vorgesehen sein, dass die Differenz zwischen dem Index s in Formel (LAr-1) und dem Index t in Formel (LAr-2) höchstens 2, vorzugsweise höchstens 1 und speziell bevorzugt 0 ist.

Bevorzugte Verbindungen gemäß Formel (I) umfassen mindestens eine Donorgruppe gemäß Formeln (H-1) bis (H-26), wobei die Gruppe $Ar^2$ für eine verbindende Struktur der Formel (LAr-1) steht, und mindestens eine Akzeptorgruppe gemäß Formel (QL), wobei L' durch eine verbindende Struktur der Formel (LAr-2) darstellbar ist, worin der Index s in Formel (LAr-1) und der Index t in Formel (LAr-2) die folgenden Werte aufweisen:

| Verbindung | Index s in Formel (LAr-1) | Index t in Formel (LAr-2) |
|---|---|---|
| Formel (I-1) | 0 | 0 |
| Formel (I-2) | 1 | 1 |
| Formel (I-3) | 2 | 2 |
| Formel (I-4) | 0 | 1 |
| Formel (I-5) | 1 | 0 |
| Formel (I-6) | 1 | 2 |
| Formel (I-7) | 2 | 1 |
| Formel (I-8) | 3 | 3 |
| Formel (I-9) | 3 | 2 |
| Formel (I-10) | 2 | 3 |
| Formel (I-11) | 0 | 2 |
| Formel (I-12) | 2 | 0 |
| Formel (I-13) | 3 | 1 |

(fortgesetzt)

| Verbindung | Index s in Formel (LAr-1) | Index t in Formel (LAr-2) |
|---|---|---|
| Formel (I-14) | 1 | 3 |

In einer weiteren Ausgestaltung kann vorgesehen sein, dass die unter anderem in den Formel (I) dargelegte Gruppe Q oder die elektronenziehende Gruppe $Q^1$ ein heteroaromatisches Ringsystem darstellt, wobei die Ringatome 1 bis 4 Stickstoffatome umfassen und das Ringsystem durch einen oder mehrere Reste $R^1$ substituiert sein kann, vorzugsweise jedoch unsubstituiert ist, wobei $R^1$ die zuvor, insbesondere für Formel (I) dargelegte Bedeutung aufweist.

Darüber hinaus sind Verbindungen bevorzugt, die dadurch gekennzeichnet sind, dass die Akzeptorgruppe Q in Formel (I) oder die elektronenziehende Gruppe $Q^1$ ein heteroaromatisches Ringsystem mit mindestens zwei kondensierten Ringen ist, welches durch einen oder mehrere Reste $R^1$ substituiert sein kann, vorzugsweise jedoch unsubstituiert ist, wobei die Ringatome der mindestens zwei kondensierten Ringe mindestens ein, vorzugsweise mindestens zwei Stickstoffatome umfassen, wobei $R^1$ die zuvor, insbesondere für Formel (I) dargelegte Bedeutung aufweist.

Ferner kann vorgesehen sein, dass die unter anderem in den Formel dargelegte Gruppe Q oder die elektronenziehende Gruppe $Q^1$ ein heteroaromatisches Ringsystem mit 9 bis 14, vorzugsweise 10 Ringatomen darstellt, welches durch einen oder mehrere Reste $R^1$ substituiert sein kann, wobei $R^1$ die zuvor, insbesondere für Formel (I) dargelegte Bedeutung aufweist, vorzugsweise jedoch unsubstituiert ist.

Vorzugsweise kann die unter anderem in den Formel (I) dargelegte Gruppe Q oder die in Formel (QL) dargelegte Gruppe $Q^1$ ausgewählt sein aus Strukturen der Formeln (Q-1), (Q-2), (Q-4), (Q-4), (Q-5), (Q-6), (Q-7), (Q-8), (Q-9) und/oder (Q-10)

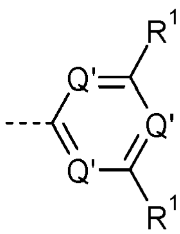
Formel (Q-1)

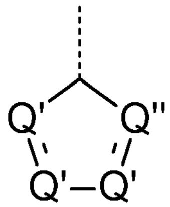
Formel (Q-2)

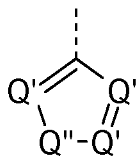
Formel (Q-3)

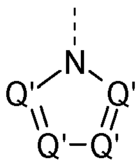
Formel (Q-4)

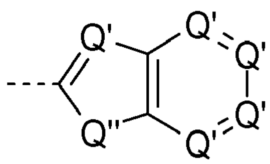
Formel (Q-5)

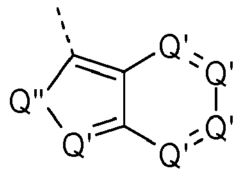
Formel (Q-6)

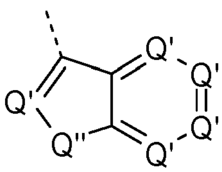
Formel (Q-7)

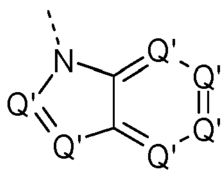
Formel (Q-8)

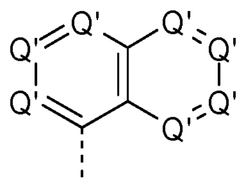
Formel (Q-9)

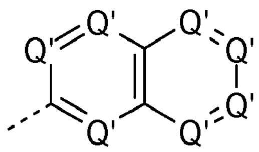

Formel (Q-10),

aufweist, wobei die gestrichelte Bindung die Anbindungsposition markiert,

Q‘ bei jedem Auftreten gleich oder verschieden $CR^1$ oder N darstellt, und
Q" $NR^1$, O oder S darstellt;

wobei wenigstens ein Q’ gleich N und/oder wenigstens ein Q" gleich $NR^1$ ist und

$R^1$ wie in Anspruch 1 definiert ist.

Vorzugsweisae wird Q in Formel (I) oder die in Formel (QL) dargelegte Gruppe $Q^1$ ausgewählt aus sStrukturen der Formeln (Q-11), (Q-12), (Q-13), (Q-14) und/oder (Q-15)

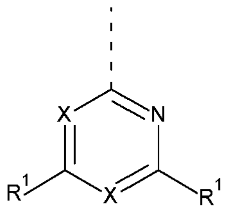

Formel (Q-11)

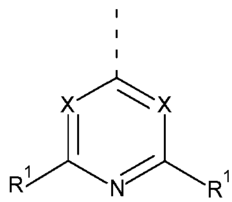

Formel (Q-12)

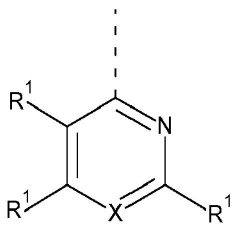

Formel (Q-13)

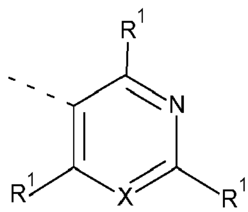

Formel (Q-14)

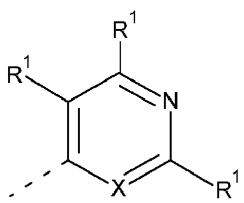

Formel (Q-15)

wobei das Symbol $R^1$ die zuvor unter anderem für Formel (I) genannte Bedeutung aufweist, X N oder $CR^1$ ist und die gestrichelte Bindung die Anbindungsposition markiert, wobei X vorzugsweise ein Stickstoffatom darstellt.

In einer weiteren Ausführungsform kann die unter anderem in Formel (I) dargelegte Gruppe Q oder die unter anderem in Formel (QL) dargelegte Gruppe $Q^1$ ausgewählt sein aus Strukturen der Formeln (Q-16), (Q-17), (Q-18), (Q-19), (Q-20), (Q-21) und/oder (Q-22)

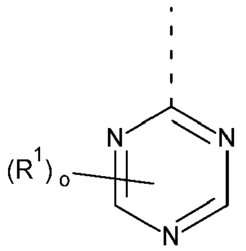

Formel (Q-16)

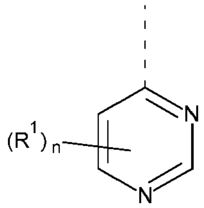

Formel (Q-17)

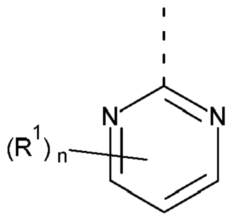

Formel (Q-18)

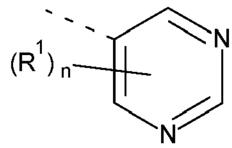

Formel (Q-19)

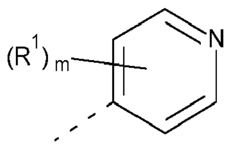

Formel (Q-20)

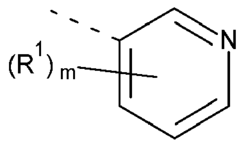

Formel (Q-21)

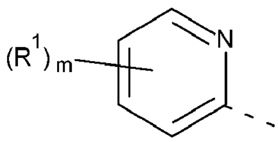

Formel (Q-22)

worin das Symbol $R^1$ die zuvor unter anderem für Formel (I) dargelegt Bedeutung aufweist, die gestrichelte Bindung die Anbindungsposition markiert und m 0, 1, 2, 3 oder 4, vorzugsweise 0, 1 oder 2, n 0, 1, 2 oder 3, vorzugsweise 0, 1 oder 2 und o 0,1 oder 2, vorzugsweise 1 oder 2 ist. Hierbei sind die Strukturen der Formeln (Q-16), (Q-17), (Q-18) und (Q-19) bevorzugt.

In einer weiteren Ausführungsform kann die unter anderem in den Formel (I), dargelegte Gruppe Q oder die unter anderem in Formel (QL) dargelegte Gruppe $Q^1$ ausgewählt sein aus Strukturen der Formeln (Q-23), (Q-24) und/oder (Q-25),

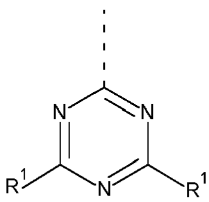

Formel (Q-23)

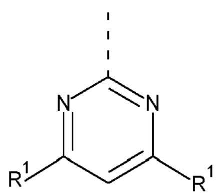

Formel (Q-24)

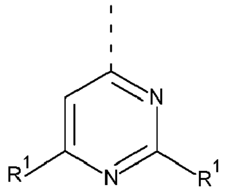

Formel (Q-25)

worin das Symbol $R^1$ die zuvor unter anderem für Formel (I) dargelegt Bedeutung aufweist und die gestrichelte Bindung die Anbindungsposition markiert.

In einer weiteren Ausführungsform kann die unter anderem in den Formel (I) dargelegte Gruppe Q oder die unter anderem in Formel (QL) dargelegte Gruppe $Q^1$ ausgewählt sein aus Strukturen der Formeln (Q-26), (Q-27), (Q-28), (Q-29) und/oder (Q-30),

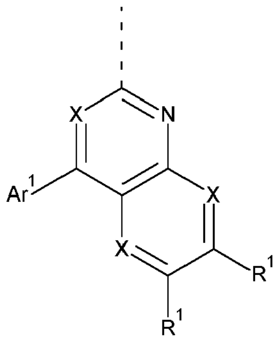

Formel (Q-26)

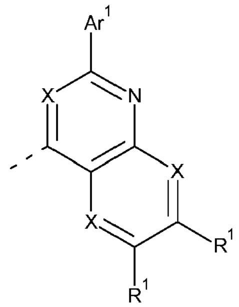

Formel (Q-27)

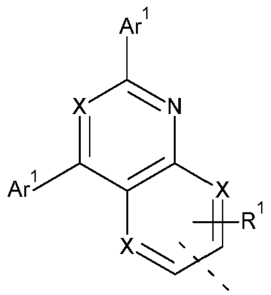

Formel (Q-28)

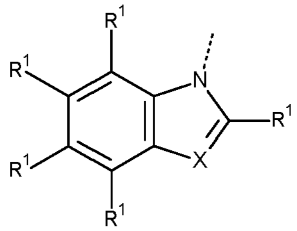

Formel (Q-29)

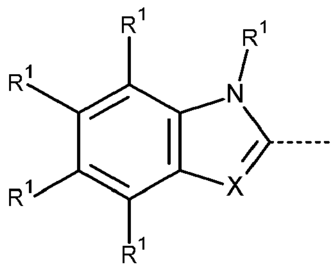

Formel (Q-30)

wobei X N oder $CR^1$ ist, das Symbol $R^1$, die zuvor unter anderem für Formel (I) genannte Bedeutung aufweisen, die gestrichelte Bindung die Anbindungsposition markiert, wobei X vorzugsweise ein Stickstoffatom darstellt und $Ar^1$ ein aromatisches oder heteroaromatisches Ringsystem mit 5 bis 60 aromatischen, vorzugsweise 5 bis 40 aromatischen

Ringatomen, das jeweils mit einem oder mehreren Resten $R^1$ substituiert sein kann, eine Aryloxygruppe mit 5 bis 60 aromatischen, vorzugsweise 5 bis 40 aromatischen Ringatomen, die mit einem oder mehreren Resten $R^1$ substituiert sein kann, oder einer Aralkylgruppe mit 5 bis 60 aromatischen, vorzugsweise 5 bis 40 aromatischen Ringatomen, die jeweils mit einem oder mehreren Resten $R^1$ substituiert sein kann, darstellt, wobei optional zwei oder mehr, vorzugsweise benachbarte Substituenten $R^1$ ein mono-oder polycyclisches, aliphatisches, heteroaliphatisches, aromatisches oder heteroaromatisches Ringsystem, vorzugsweise ein mono- oder polycyclisches, aliphatisches Ringsystem bilden können, das mit einem oder mehreren Resten $R^2$ substituiert sein kann.

Vorzugsweise kann die unter anderem in den Formel (I), dargelegte Gruppe Q oder die unter anderem in Formel (QL) dargelegte Gruppe $Q^1$ ausgewählt sein aus Strukturen der Formeln (Q-31), (Q-32), (Q-33), (Q-34), (Q-35), (Q-36), (Q-37), (Q-38), (Q-39), (Q-40), (Q-41), (Q-42), (Q-43) und/oder (Q-44)

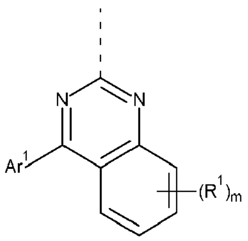

Formel (Q-31)

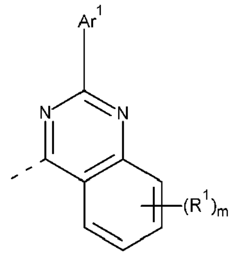

Formel (Q-32)

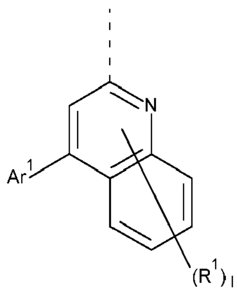

Formel (Q-33)

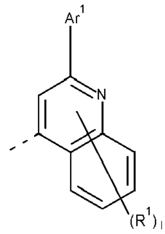

Formel (Q-34)

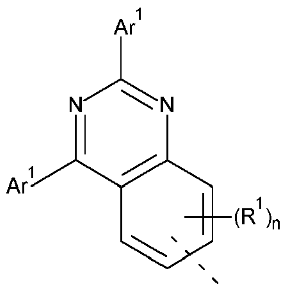

Formel (Q-35)

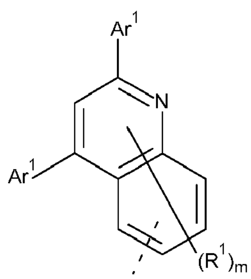

Formel (Q-36)

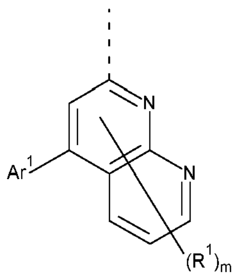

Formel (Q-37)

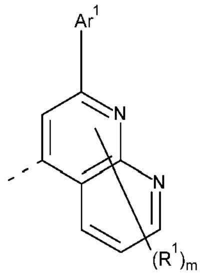

Formel (Q-38)

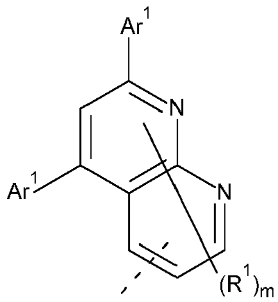

Formel (Q-39)

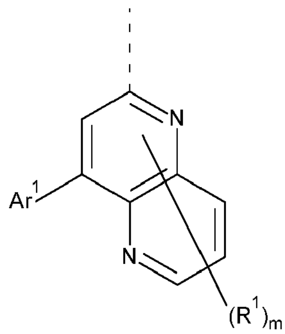

Formel (Q-40)

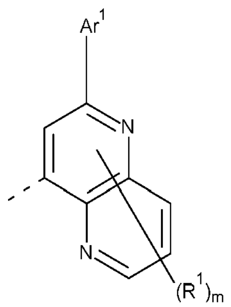

Formel (Q-41)

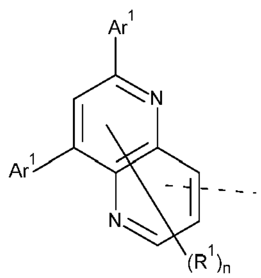

Formel (Q-42)

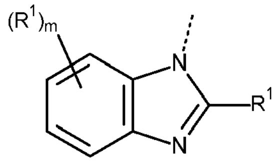

Formel (Q-43)

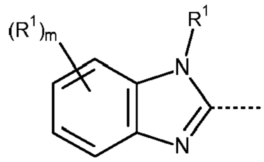

Formel (Q-44)

worin die Symbole $Ar^1$ die zuvor unter anderem für Formel Q-26), (Q-27), (Q-28), (Q-29) oder (Q-30) und $R^1$ die zuvor unter anderem für Formel (I) dargelegt Bedeutung aufweisen, die gestrichelte Bindung die Anbindungsposition markiert und m 0, 1, 2, 3 oder 4, vorzugsweise 0, 1 oder 2, n 0, 1, 2 oder 3, vorzugsweise 0 oder 1 und l 0, 1, 2, 3, 4 oder 5, vorzugsweise 0, 1 oder 2 ist.

Vorzugsweise steht das Symbol $Ar^1$ für einen Aryl- oder Heteroarylrest, so dass eine aromatische oder heteroaromatische Gruppe eines aromatischen oder heteroaromatischen Ringsystems direkt, d.h. über ein Atom der aromatischen oder heteroaromatischen Gruppe, an das jeweilige Atom der weiteren Gruppe gebunden ist, beispielweise das C- oder N-Atom der zuvor dargestellten Gruppen (Q-26) bis (Q-42).

In einer weiteren bevorzugten Ausführungsform der Erfindung steht $Ar^1$ gleich oder verschieden bei jedem Auftreten für ein aromatisches oder heteroaromatisches Ringsystem mit 6 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste $R^1$ substituiert sein kann, bevorzugt aber unsubstituiert ist, wobei $R^1$ die zuvor, insbesondere in Formel (I) dargestellte Bedeutung aufweisen kann.

Mit Vorteil stellt $Ar^1$ in den Formeln (Q-26) bis (Q-42) ein aromatisches Ringsystem mit 6 bis 12 aromatischen Ringatomen dar, welches mit einem oder mehreren Resten $R^1$ substituiert sein kann, vorzugsweise aber unsubstituiert ist, wobei $R^1$ die zuvor, insbesondere für Formel (I) dargestellte Bedeutung aufweisen kann.

Bevorzugt bilden die Reste $R^1$ in den Formeln (Q-1) bis (Q-44) mit den Ringatomen der Heteroarylgruppe, an die die Reste $R^1$ gebunden sind, kein kondensiertes Ringsystem. Dies schließt die Bildung eines kondensierten Ringsystems mit möglichen Substituenten $R^2$, $R^3$ ein, die an die Reste $R^1$ gebunden sein können.

Bevorzugt bilden die Reste $R^2$ mit den Ringatomen der Arylgruppe oder Heteroarylgruppe $Ar^1$, an die die Reste $R^2$ in den Formeln (Q-26) bis (Q-42) gebunden sein können, kein kondensiertes Ringsystem. Dies schließt die Bildung eines kondensierten Ringsystems mit möglichen Substituenten $R^3$ ein, die an die Reste $R^2$ gebunden sein können.

In einer bevorzugten Ausführungsform kann vorgesehen sein, dass die elektronenziehende Gruppe $Q^1$ ein aromatisches oder heteroaromatisches Ringsystem mit 5 bis 60, vorzugsweise mit 5 bis 40, bevorzugt mit 6 bis 24 aromatischen Ringatomen, besonders bevorzugt mit 6 bis 18 aromatischen aromatischen Ringatomen ist, welches einen oder mehrere elektronenziehende Substituenten aufweist. Vorzugsweise weist der elektronenziehende Substituent eine Hammett-Konstante, $\sigma$, von größer oder gleich Null auf. Ganz bevorzugt ist die Hammett-Konstante des elektronenziehenden Substituenten größer oder gleich 0,2, besonders bevorzugt größer oder gleich 0,4 und ganz besonder bevorzugt größer oder gleiche 0,55. Weitere Einzelheiten zur Bedeutung und Bestimmung der Hammett-Konstante sind dem Fachmann aus den Grundlagen der organischen Chemie gut bekannt und in gängigen Lehrbüchern offenbart. Die Hammett-Konstante und deren Bestimmung im Sinne der vorliegenden Erfindung ist wie in Kapitel 9 von Jerry March, Advanced Organic Chemistry, John Wiley & Sons, Fourth Edition, 1992 angegeben definiert.

Beispiele für geeignete aromatische oder heteroaromatische Ringsysteme, die einen oder mehrere elektronenziehende Substituenten aufweisen, sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3-oder 4-Dibenzofuranyl, 1-, 2-, 3- oder 4-Dibenzothienyl, Pyrenyl, Triazinyl, Imimdazolyl, Benzimidazolyl, Benzoxazolyl, Benzthiazolyl, 1- oder 2-Napthyl, Anthracenyl, die jeweils durch einen oder mehrere Reste $R^2$ substituiert sein können, wobei Phenyl, Spirobifluoren-, Fluoren-, Dibenzofuran-, Dibenzothiophen-, Anthracen-, Phenanthren-, Triphenylen-Gruppen besonders bevorzugt sind. Von den genannten Gruppen sind Phenylreste, die einen oder mehrere elektronenziehende Substituenten aufweisen, speziell bevorzugt.

Zu den bevorzugten elektronenziehende Substituent gehören unter anderem F, fluorierte Alkylgruppen, $CF_3$, $C_nF_{2n+1}$, $C(=O)OR^1$, $C(=O)N(R^1)_2$, $B(OR^1)_2$, $NO_2$, CHO, $C(=O)R^1$, $P(=O)(R^1)_2$, $S(=O)R^1$, $S(=O)_2R^1$ und/oder CN, wobei $CF_3$, $C_nF_{2n+1}$, $C(=O)OR^1$, $C(=O)N(R^1)_2$, $NO_2$, CHO, $C(=O)R^1$, $S(=O)R^1$, $S(=O)_2R^1$ und/oder CN bevorzugt und CN, F, fluorierte Alkylgruppen, $CF_3$, $C_nF_{2n,1}$ besonders bevorzugt sind. Die elektronenziehende Gruppe $Q^1$ kann vorzugsweise zwei oder mehr elektronenziehende Substituenten aufweisen, wobei diese gleich oder verschieden sein können.

Vorzugsweise steht das Symbol $Ar^a$ und/oder $Ar^b$ für einen Aryl- oder Heteroarylrest, so dass eine aromatische oder heteroaromatische Gruppe eines aromatischen oder heteroaromatischen Ringsystems direkt, d.h. über ein Atom der aromatischen oder heteroaromatischen Gruppe, an das jeweilige Atom der weiteren Gruppe gebunden ist.

In einer weiteren bevorzugten Ausführungsform der Erfindung steht das Symbol $Ar^a$ und/oder $Ar^b$ gleich oder verschieden für ein aromatisches oder heteroaromatisches Ringsystem mit 6 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste $R^1$ substituiert sein kann, bevorzugt aber unsubstituiert ist, wobei $R^1$ die zuvor, insbesondere in Formel (I) dargestellte Bedeutung aufweisen kann.

Mit Vorteil stellt das Symbol $Ar^a$ und/oder $Ar^b$ in Formel (I) jeweils unabhängig ein aromatisches Ringsystem mit 6 bis 12 aromatischen Ringatomen dar, welches mit einem oder mehreren Resten $R^1$ substituiert sein kann, vorzugsweise aber unsubstituiert ist, wobei $R^1$ die zuvor, insbesondere für Formel (I) dargestellte Bedeutung aufweisen kann. Speziell bevorzugt stellen $Ar^a$ und/oder $Ar^b$ in Formel (I) jeweils einen Phenylring dar, der mit einem oder mehreren Resten $R^1$ substituiert sein kann, vorzugsweise aber unsubstituiert ist.

Speziell bevorzugt unterscheidet sich die Gruppe $Ar^a$ und/oder $Ar^b$ in Formel (I) von einer Donorgruppe gemäß Symbol HL. Weiterhin kann vorgesehen sein, dass sich die Gruppe $Ar^a$ in Formel (I) von einer Donorgruppe gemäß Symbol HL unterscheidet. Ferner kann vorgesehen sein, dass sich die Gruppe $Ar^b$ in Formel (I) von einer Donorgruppe gemäß Symbol HL unterscheidet. Insbesondere bevorzugt unterscheiden sich beide Gruppen $Ar^a$ und $Ar^b$ in Formel (I) von einer Donorgruppe gemäß Symbol HL.

Speziell bevorzugt unterscheidet sich die Gruppe $Ar^a$ und/oder $Ar^b$ in Formel (I) von einer Akzeptorgruppe gemäß Symbol Q. Weiterhin kann vorgesehen sein, dass sich die Gruppe $Ar^a$ in Formel (I) von einer Akzeptorgruppe gemäß Symbol Q unterscheidet. Ferner kann vorgesehen sein, dass sich die Gruppe $Ar^b$ in Formel (I) von einer Akzeptorgruppe gemäß Symbol Q unterscheidet. Insbesondere bevorzugt unterscheiden sich beide Gruppen $Ar^a$ und $Ar^b$ in Formel (I) von einer Akzeptorgruppe gemäß Symbol Q.

Bevorzugt sind die Substituenten $R^a$, $R^b$ in Formel (I) gewählt aus der Gruppe bestehend aus F, CN, $N(Ar^1)_2$, $C(=O)Ar^1$, $P(=O)(Ar^1)_2$, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten $R^2$ substituiert sein kann, wobei eine oder mehrere nicht-benachbarte $CH_2$-Gruppen durch O ersetzt sein können und wobei ein oder mehrere H-Atome durch D oder F ersetzt sein können, einem aromatischen oder heteroaromatischen Ringsystem mit 5 bis 40, bevozugt mit aromatischen Ringatomen, das jeweils mit einem oder mehreren Resten $R^2$ substituiert sein kann, bevorzugt aber unsubstituiert ist, oder einer Aralkyl- oder Heteroaralkylgruppe mit 5 bis 25 aromatischen Ringatomen, die mit einem oder mehreren Resten $R^2$ substituiert sein kann; dabei können optional zwei Substituenten $R^1$, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten $R^1$ substituiert sein kann; wobei $Ar^1$ die für Formel (H-1) bis (H-26) oder/und Formel Q-26), (Q-27), (Q-28), (Q-29) oder (Q-30) dargelegte Bedeutung aufweisen kann, wobei die hierbei dargelegten Bevorzugungen auch in dieser Bedeutung gelten. Vorzugsweise stellt $Ar^1$ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten $R^2$ substituiert sein kann, vorzugsweise jedoch unsubstituiert ist.

Vorzugsweise steht das Symbol $R^a$ und/oder $R^b$ jeweils unabhängig für einen Aryl- oder Heteroarylrest, so dass eine aromatische oder heteroaromatische Gruppe eines aromatischen oder heteroaromatischen Ringsystems direkt, d.h. über ein Atom der aromatischen oder heteroaromatischen Gruppe, an das jeweilige Atom der weiteren Gruppe gebunden ist.

In einer weiteren bevorzugten Ausführungsform der Erfindung steht das Symbol $R^a$ und/oder $R^b$ jeweils unabhängig für ein aromatisches oder heteroaromatisches Ringsystem mit 5 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 5 bis 13 aromatischen Ringatomen, das jeweils durch einen oder mehrere Reste $R^1$ substituiert sein kann, bevorzugt aber unsubstituiert ist, wobei $R^1$ die zuvor, insbesondere in Formel (I) dargestellte Bedeutung aufweisen kann.

Mit Vorteil stellt das Symbol $R^a$ und/oder $R^b$ in Formel (I) jeweils unabhängig ein aromatisches Ringsystem mit 6 bis 12 aromatischen Ringatomen dar, welches mit einem oder mehreren Resten $R^1$ substituiert sein kann, vorzugsweise aber unsubstituiert ist, wobei $R^1$ die zuvor, insbesondere für Formel (I) dargestellte Bedeutung aufweisen kann. Speziell bevorzugt stellen $Ar^a$ und/oder $Ar^b$ in Formel (I) jeweils einen Phenylring dar, der mit einem oder mehreren Resten $R^1$ substituiert sein kann, vorzugsweise aber unsubstituiert ist.

Speziell bevorzugt unterscheidet sich die Gruppe $R^a$ und/oder $R^b$ in Formel (I) von einer Donorgruppe gemäß Symbol HL. Weiterhin kann vorgesehen sein, dass sich die Gruppe $R^a$ in Formel (I) von einer Donorgruppe gemäß Symbol HL unterscheidet. Ferner kann vorgesehen sein, dass sich die Gruppe $R^b$ in Formel (I) von einer Donorgruppe gemäß Symbol HL unterscheidet. Insbesondere bevorzugt unterscheiden sich beide Gruppen $R^a$ und $R^b$ in Formel (I) von einer Donorgruppe gemäß Symbol HL.

Speziell bevorzugt unterscheidet sich die Gruppe $R^a$ und/oder $R^b$ in Formel (I) von einer Akzeptorgruppe gemäß Symbol Q. Weiterhin kann vorgesehen sein, dass sich die Gruppe $R^a$ in Formel (I) von einer Akzeptorgruppe gemäß Symbol Q unterscheidet. Ferner kann vorgesehen sein, dass sich die Gruppe $R^b$ in Formel (I) von einer Akzeptorgruppe gemäß Symbol Q unterscheidet. Insbesondere bevorzugt unterscheiden sich beide Gruppen $R^a$ und $R^b$ in Formel (I) von einer Akzeptorgruppe gemäß Symbol Q.

Besonders bevorzugt stehen die Symbole $Ar^a$, $Ar^b$, $R^a$ und $R^b$ in Formel (I) jeweils unabhängig für ein aromatisches oder heteroaromatisches Ringsystem, welches bevorzugt 5 bis 24 aromatische Ringatome, besonders bevorzugt mit 6 bis 18 aromatische Ringatome aufweist, das jeweils durch einen oder mehrere Reste $R^1$ substituiert sein kann, bevorzugt aber unsubstituiert ist. Besonders bevorzugt stehen die Symbole $Ar^a$, $Ar^b$, $R^a$ und $R^b$ in Formel (I) jeweils unabhängig für ein aromatisches Ringsystem mit 6 bis 12 aromatischen Ringatomen bzw. ein heteroaromatisches Ringsystem mit 5 bis 13 aromatischen Ringatomen, das jeweils durch einen oder mehrere Reste $R^1$ substituiert sein kann, bevorzugt aber unsubstituiert ist, wobei $R^1$ die zuvor, insbesondere in Formel (I) dargestellte Bedeutung aufweisen kann. Speziell bevorzugt stellen Symbole $Ar^a$, $Ar^b$, $R^a$ und $R^b$ in Formel (I) jeweils einen Phenylring dar, der mit einem oder mehreren Resten $R^1$ substituiert sein kann, vorzugsweise aber unsubstituiert ist.

Hierbei können zwei oder mehr der Symbole $Ar^a$, $Ar^b$, $R^a$ und/oder $R^b$ in Formel (I) durch einen Ringschluss verknüpft sein, wobei die Verknüpfung besonders bevorzugt durch eine Bindung zwischen den jeweiligen Gruppen $Ar^a$, $Ar^b$, $R^a$ und/oder $R^b$ in Formel (I) erfolgen kann.

Wenn X für $CR^1$ steht bzw. wenn die aromatische und/oder heteroaromatische Gruppen durch Substituenten

$R^1$ substituiert sind, dann sind diese Substituenten $R^1$ bevorzugt gewählt aus der Gruppe bestehend aus H, D, F, CN, $N(Ar^1)_2$, $C(=O)Ar^1$, $P(=O)(Ar^1)_2$, einer geradkettigen Alkyl-oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten $R^2$ substituiert sein kann, wobei eine oder mehrere nicht-benachbarte $CH_2$-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten $R^2$ substituiert sein kann, bevorzugt aber unsubstituiert ist, oder einer Aralkyl- oder Heteroaralkylgruppe mit 5 bis 25 aromatischen Ringatomen, die mit einem oder mehreren Resten $R^2$ substituiert sein kann; dabei können optional zwei Substituenten $R^1$, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten $R^1$ substituiert sein kann; wobei $Ar^1$ die für Formel (H-1) bis (H-26) oder/und Formel Q-26), (Q-27), (Q-28), (Q-29) oder (Q-30) dargelegte Bedeutung aufweisen kann, wobei die hierbei dargelegten Bevorzugungen auch in dieser Bedeutung gelten.

Besonders bevorzugt sind diese Substituenten $R^1$ ausgewählt aus der Gruppe bestehend aus H, D, F, CN, $N(Ar^1)_2$, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten $R^2$ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten $R^1$ substituiert sein kann, bevorzugt aber unsubstituiert ist; dabei können optional zwei Substituenten $R^1$, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten $R^2$ substituiert sein kann, bevorzugt aber unsubstituiert ist, wobei $Ar^1$ die zuvor dargelegte Bedeutung aufweisen kann.

Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist.

Bevorzugt bilden die Reste $R^1$ mit den Ringatomen der Arylgruppe oder der Heteroarylgruppe, an die die Reste $R^1$ gebunden sind, kein kondensiertes Ringsystem. Dies schließt die Bildung eines kondensierten Ringsystems mit möglichen Substituenten $R^2$, $R^3$ ein, die an die Reste $R^1$ gebunden sein können.

Bevorzugt kann vorgesehen sein, dass die Gruppe $Ar^a$, $Ar^b$, $Ar^1$, $Ar^2$, $Ar^3$, $Ar^4$, $R^a$, $R^b$ und/oder $R^1$ in den Strukturen der gemäß Formel (I), (H-1) bis (H-26), (LAr-1), (LAr-2) und/oder (Q-1) bis (Q-44) ausgewählt ist aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl, Pyrenyl, Triazinyl, Imimdazolyl, Benzimidazolyl, Benzoxazolyl, Benzthiazolyl, 1-, 2-, 3- oder 4-Carbazolyl, 1- oder 2-Napthyl, Anthracenyl, vorzugsweise 9-Anthracenyl, Phenanthrenyl und/oder Triphenylenlyl, die jeweils durch einen oder mehrere Reste $R^2$ substituiert sein können, bevorzugt aber unsubstituiert sind, wobei Phenyl, Spirobifluoren-, Fluoren-, Dibenzofuran-, Dibenzothiophen-, Anthracen-, Phenanthren-, Triphenylen- Gruppen besonders bevorzugt sind.

Weiterhin kann vorgesehen sein, dass in einer Struktur gemäß Formel (I), (H-1) bis (H-26), (LAr-1), (LAr-2) und/oder (Q-1) bis (Q-44), mindestens ein Rest $Ar^a$, $Ar^b$, $Ar^1$, $Ar^3$, $Ar^4$, $R^a$, $R^b$ und/oder $R^1$ eine Gruppe umfasst, bevorzugt für eine Gruppe steht, die ausgewählt ist aus den Formeln ($R^1$-1) bis ($R^1$- 86)

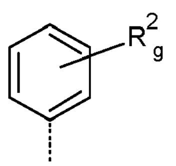

Formel ($R^1$-1)

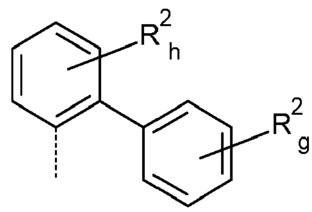

Formel ($R^1$-2)

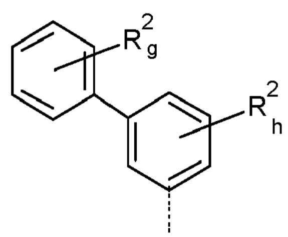

Formel ($R^1$-3)

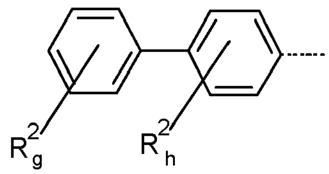
Formel ($R^1$-4)

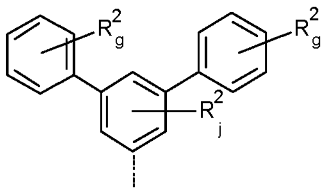
Formel ($R^1$-5)

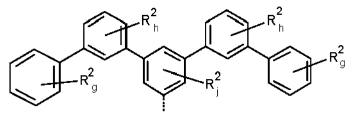
Formel ($R^1$-6)

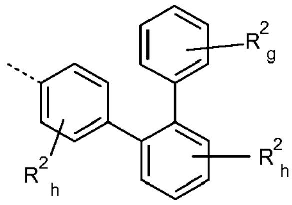
Formel ($R^1$-7)

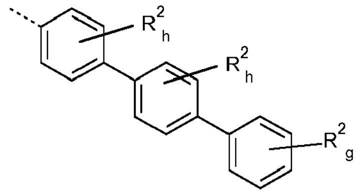
Formel ($R^1$-8)

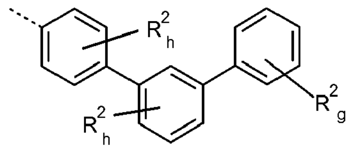
Formel ($R^1$-9)

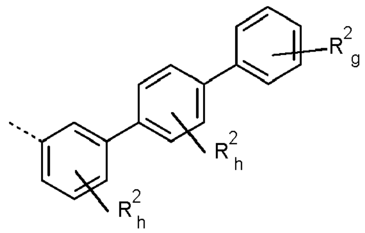
Formel ($R^1$-10)

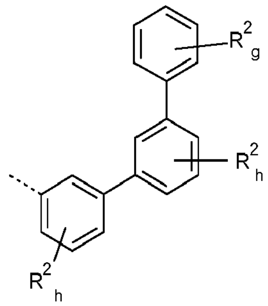
Formel ($R^1$-11)

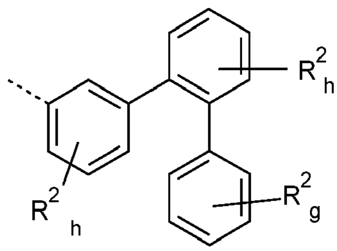
Formel ($R^1$-12)

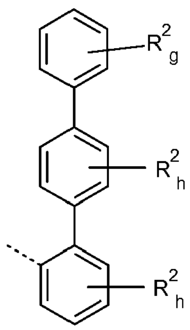
Formel ($R^1$-13)

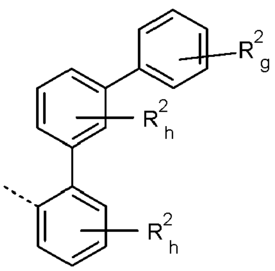
Formel ($R^1$-14)

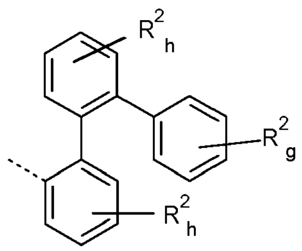
Formel ($R^1$-15)

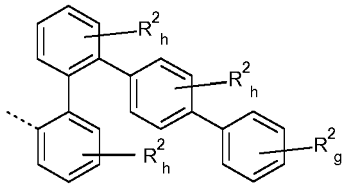

Formel ($R^1$-16)

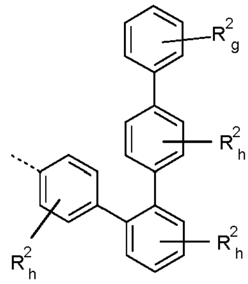

Formel ($R^1$-17)

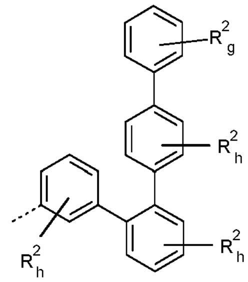

Formel ($R^1$-18)

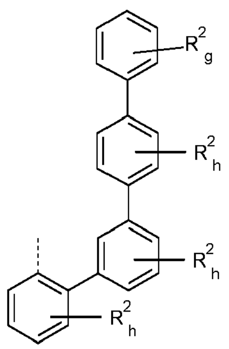

Formel ($R^1$-19)

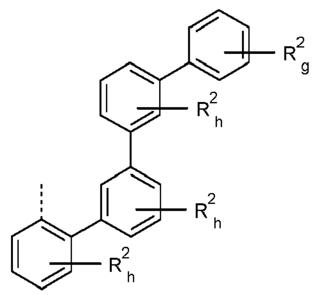

Formel ($R^1$-20)

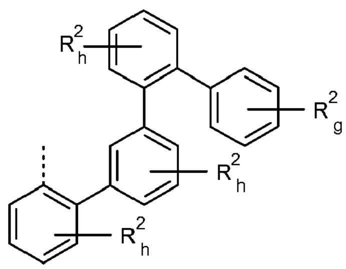

Formel ($R^1$-21)

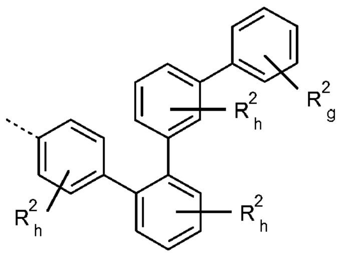

Formel ($R^1$-22)

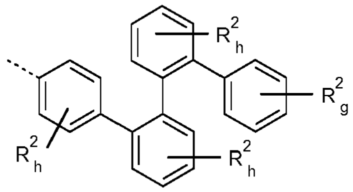

Formel ($R^1$-23)

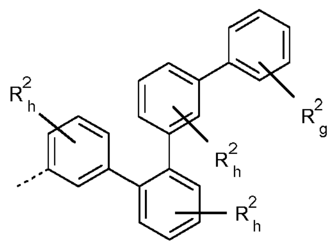

Formel ($R^1$-24)

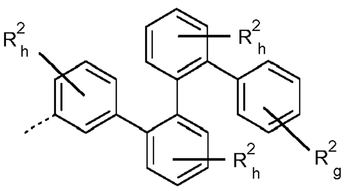

Formel ($R^1$-25)

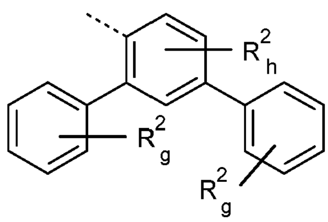

Formel ($R^1$-26)

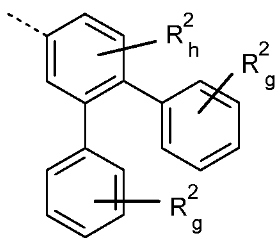

Formel ($R^1$-27)

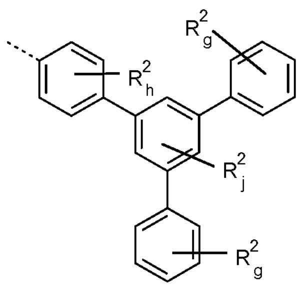

Formel ($R^1$-28)

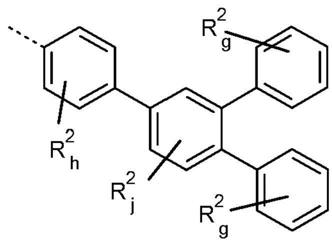

Formel ($R^1$-29)

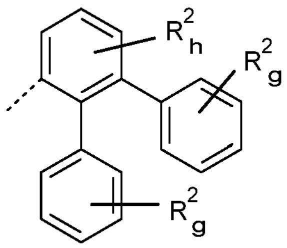

Formel ($R^1$-30)

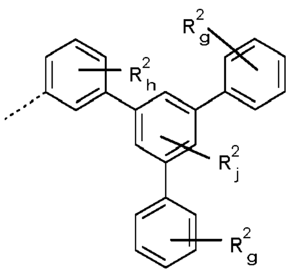

Formel ($R^1$-31)

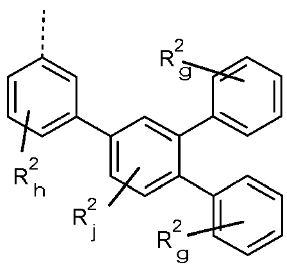

Formel ($R^1$-32)

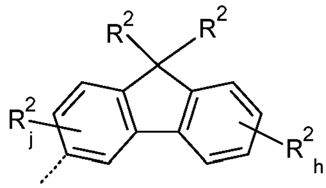

Formel ($R^1$-33)

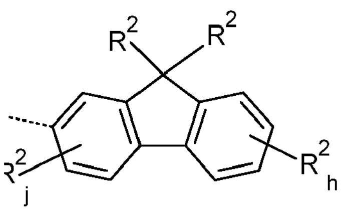

Formel ($R^1$-34)

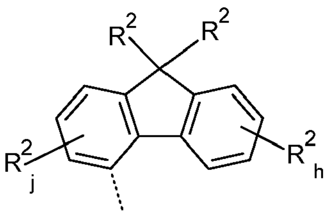

Formel ($R^1$-35)

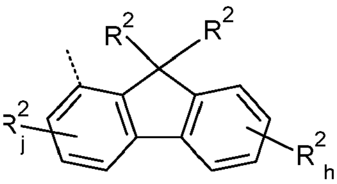

Formel ($R^1$-36)

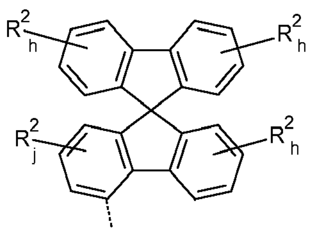

Formel ($R^1$-37)

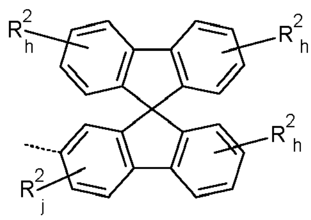

Formel ($R^1$-38)

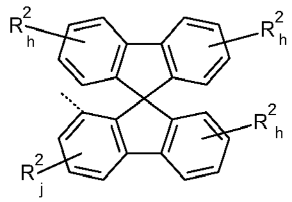

Formel ($R^1$-39)

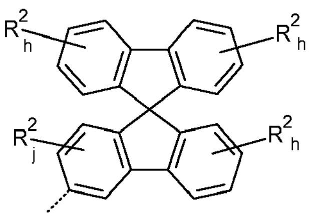

Formel ($R^1$-40)

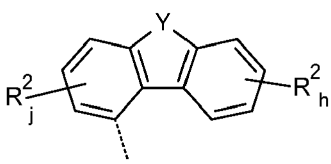

Formel ($R^1$-41)

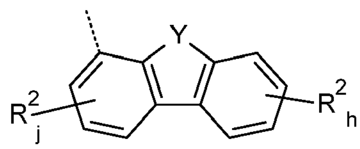

Formel ($R^1$-42)

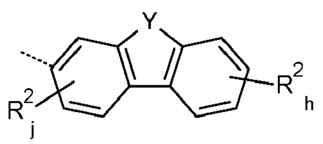

Formel ($R^1$-43)

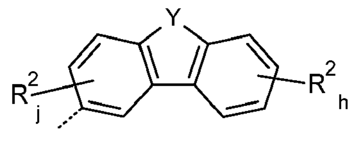

Formel ($R^1$-44)

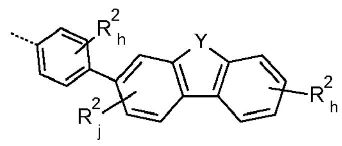

Formel ($R^1$-45)

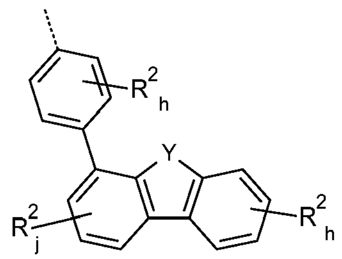

Formel ($R^1$-46)

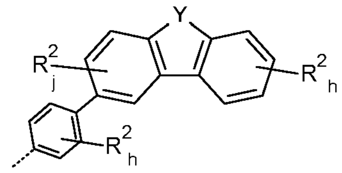

Formel ($R^1$-47)

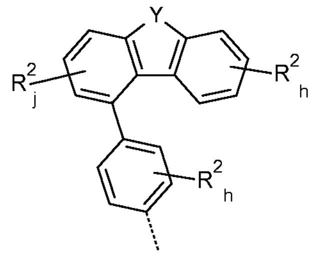

Formel ($R^1$-48)

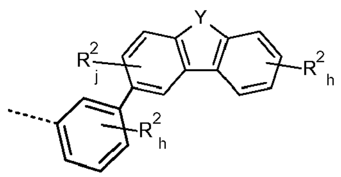

Formel ($R^1$-49)

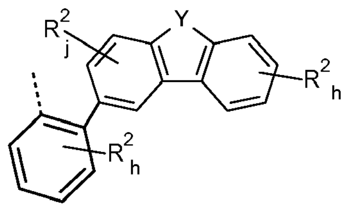

Formel ($R^1$-50)

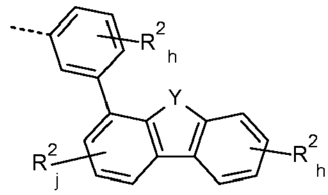

Formel ($R^1$-51)

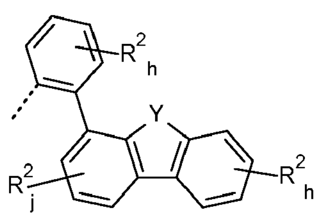

Formel ($R^1$-52)

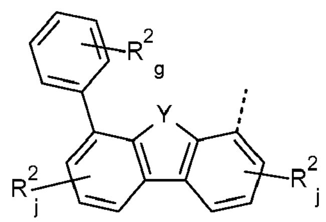

Formel ($R^1$-53)

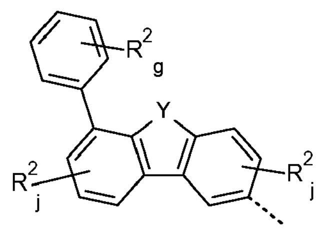

Formel ($R^1$-54)

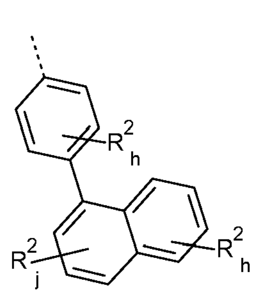

Formel ($R^1$-55)

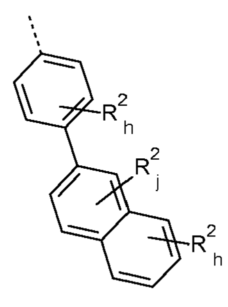

Formel ($R^1$-56)

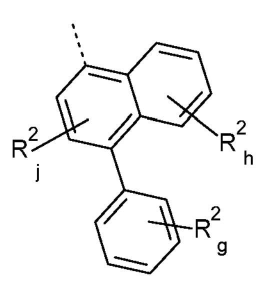

Formel ($R^1$-57)

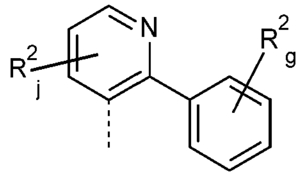

Formel ($R^1$-58)

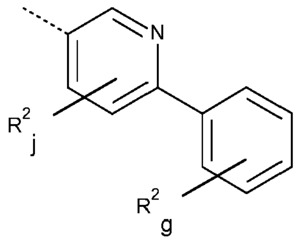

Formel ($R^1$-59)

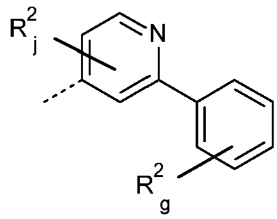

Formel ($R^1$-60)

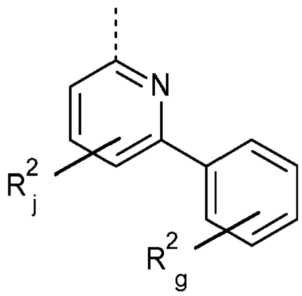

Formel ($R^1$-61)

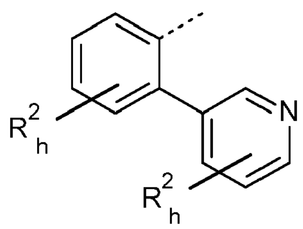

Formel ($R^1$-62)

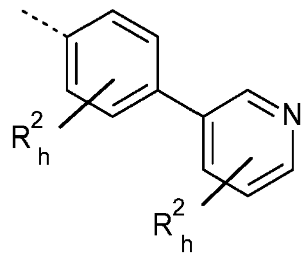

Formel ($R^1$-63)

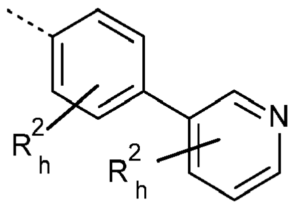

Formel ($R^1$-64)

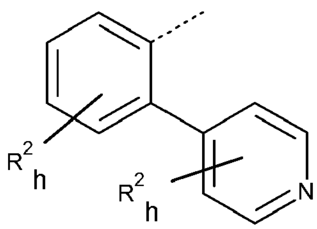

Formel ($R^1$-65)

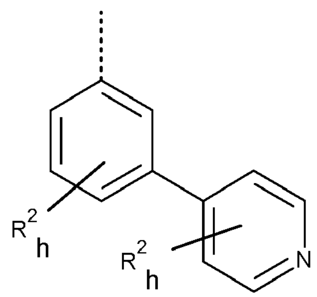

Formel ($R^1$-66)

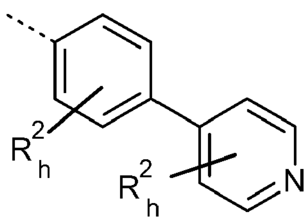

Formel ($R^1$-67)

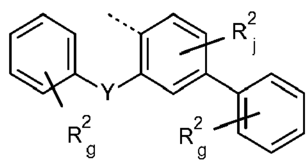

Formel ($R^1$-68)

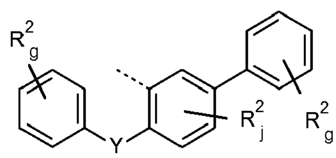

Formel ($R^1$-69)

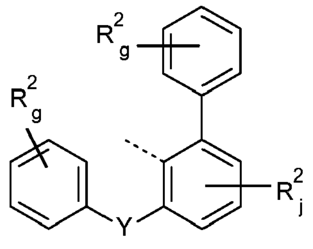

Formel ($R^1$-70)

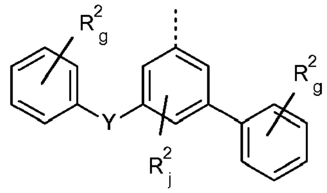

Formel ($R^1$-71)

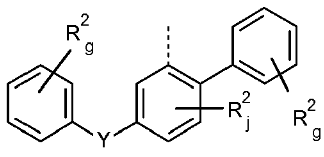

Formel ($R^1$-72)

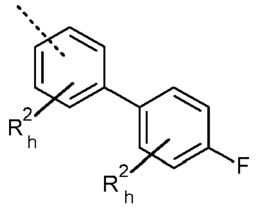

Formel ($R^1$-73)

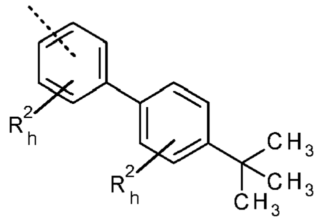

Formel ($R^1$-74)

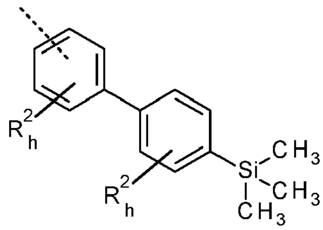

Formel ($R^1$-75)

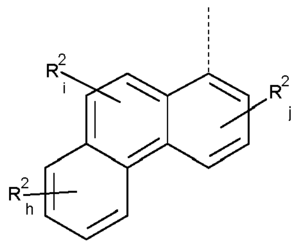

Formel ($R^1$-76)

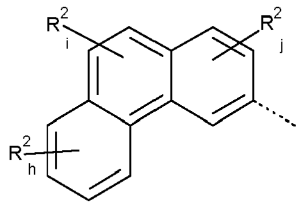

Formel ($R^1$-77)

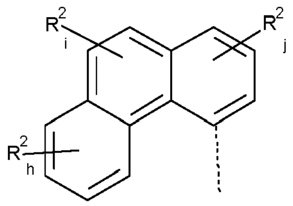

Formel ($R^1$-78)

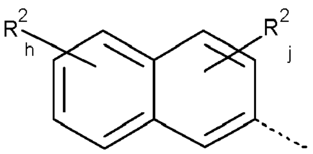

Formel ($R^1$-79)

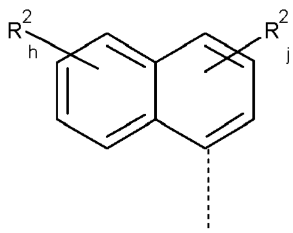

Formel ($R^1$-80)

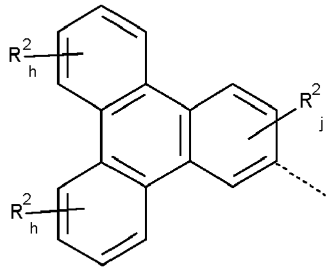

Formel ($R^1$-81)

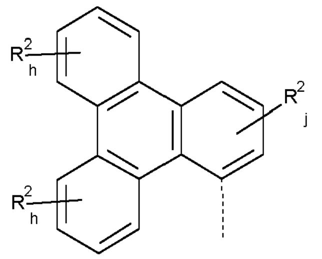

Formel ($R^1$-82)

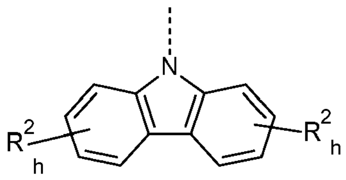

Formel ($R^1$-83)

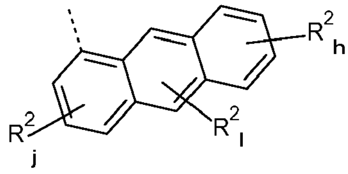

Formel ($R^1$-84)

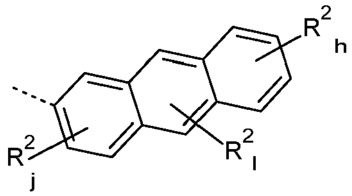

Formel ($R^1$-85)

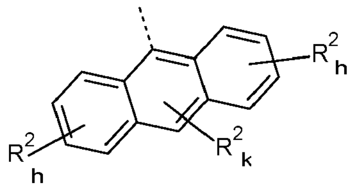

Formel ($R^1$-86)

wobei für die verwendeten Symbole gilt:

Y ist O, S oder $NR^2$, vorzugsweise O oder S;
i ist bei jedem Auftreten unabhängig 0, 1 oder 2;
j ist bei jedem Auftreten unabhängig 0, 1, 2 oder 3;
h ist bei jedem Auftreten unabhängig 0, 1, 2, 3 oder 4;
g ist bei jedem Auftreten unabhängig 0, 1, 2, 3, 4 oder 5;
$R^2$ kann die zuvor genannte, insbesondere für Formel (I) genannte Bedeutung aufweisen und

die gestrichelte Bindung markiert die Anbindungsposition. Hierbei sind die Gruppen der Formeln R1-1 bis R1-54 bevorzugt und Gruppen der Formeln R1-1, R1-3, R1-5, R1-6, R1-15, R1-29, R1-34, R1-35, R1-45, R1-46, R1-47 und/oder R1-48 besonders bevorzugt.

Vorzugsweise kann vorgesehen sein, dass die Summe der Indices i, j, h und g in den Strukturen der Formel ($R^1$-1) bis ($R^1$-86) jeweils höchstens 3, vorzugsweise höchstens 2 und besonders bevorzugt höchstens 1 beträgt.

Bevorzugt bilden die Reste $R^2$ in den Formeln ($R^1$-1) bis ($R^1$-86) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

Bevorzugt kann die Gruppe $L^1$ mit der Gruppe $Q^1$ und dem aromatischen Rest, an den die Gruppe L' gemäß Formel (I) beziehungsweise (QL) gebunden ist, eine durchgängige Konjugation ausbilden. Eine durchgängie Konjugation der aromatischen beziehungsweise heteroaromatischen Systeme wird ausgebildet sobald direkte Bindungen zwischen benachbarten aromatischen oder heteroaromatischen Ringen gebildet werden. Eine weitere Verknüpfung zwischen den zuvor genannten konjugierten Gruppen, die beispielsweise über ein S-, N- oder O-Atom oder eine Carbonylgruppe erfolgt, schadet einer Konjugation nicht. Bei einem Fluorensystem sind die beiden aromatischen Ringe unmittelbar gebunden, wobei das $sp^3$ hybridisierte Kohlenstoffatom in Position 9 zwar eine Kondensation dieser Ringe unterbindet, jedoch eine Konjugation erfolgen kann, da dieses $sp^3$ hybridisierte Kohlenstoffatom in Position 9 nicht zwingend zwischen der Gruppe $Q^1$ und dem aromatischen Rest liegt, an den die Gruppe L' gemäß Formel (I) beziehungsweise (QL) gebunden ist. Im Gegensatz hierzu kann bei einer zweiten Spirobifluorenstruktur eine durchgängie Konjugation ausgebildet werden, falls die Verbindung zwischen der Gruppe $Q^1$ und dem aromatischen Rest der Formel (I) über die gleiche Phenylgruppe der Spirobifluorenstruktur oder über Phenylgruppen der Spirobifluorenstruktur, die unmittelbar aneinander gebunden sind und in einer Ebene liegen, erfolgt. Falls die Verbindung zwischen der Gruppe $Q^1$ und dem aromatischen Rest der Formel (I) über verschiedene Phenylgruppen der zweiten Spirobifluorenstruktur erfolgt, die über das $sp^3$ hybridisierte Kohlenstoffatom in Position 9 verbunden sind, ist die Konjugation unterbrochen. Bevorzugt bilden auch die Reste $Ar^2$ beispielsweise in Formeln (H-1) bis H-26) mit den Gruppen, an die die Reste $Ar^2$ gebunden sind, eine durchgängige Konjugation aus. Ferner bilden die verbindenden Strukturen der Formel (LAr-1) und/oder (LAr-2) mit den Gruppen, an die die verbindenden Strukturen der Formel (LAr-1) und/oder (LAr-2) gebunden sind, eine durchgängige Konjugation aus.

In einer weiteren bevorzugten Ausführungsform der Erfindung steht L' oder die Gruppe $Ar^2$ für eine Bindung

oder für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste $R^1$ substituiert sein kann, bevorzugt aber unsubstituiert ist, wobei $R^1$ die zuvor, insbesondere für Formel (I) genannte Bedeutung aufweisen kann. Besonders bevorzugt steht L' oder die Gruppe $Ar^2$ für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste $R^2$ substituiert sein kann, bevorzugt aber unsubstituiert ist, wobei $R^2$ die zuvor, insbesondere für Formel (I) genannte Bedeutung aufweisen kann.

Weiterhin bevorzugt steht das unter anderem in den Strukturen gemäß Formel (QL) dargelegte Symbol L' oder die in Formeln (H-1) bis (H-26) dargelegte Gruppe $Ar^2$ gleich oder verschieden bei jedem Auftreten für eine Bindung oder einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die unter anderem in den Strukturen gemäß Formel (QL) dargelegte Symbol L' oder die in Formeln (H-1) bis (H-26) dargelegte Gruppe $Ar^2$ ein aromatisches Ringsystem mit höchstens zwei kondensierten aromatischen und/oder heteroaromatischen Ringen, vorzugsweise kein kondensiertes aromatisches oder heteroaromatisches Ringsystem umfasst. Demgemäß sind Naphtylstrukturen gegenüber Antracenstrukturen bevorzugt. Weiterhin sind Fluorenyl-, Spirobifluorenyl-, Dibenzofuranyl- und/oder Dibenzothienyl-Strukturen gegenüber Naphtylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme L' oder $Ar^2$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl, Pyrenyl, Triazinyl, Imimdazolyl, Benzimidazolyl, Benzoxazolyl, Benzthiazolyl, 1-, 2-, 3- oder 4-Carbazolyl, 1- oder 2-Napthyl, Anthracenyl, vorzugsweise 9-Anthracenyl, Phenanthrenyl und/oder Triphenylenyl, die jeweils durch einen oder mehrere Reste $R^2$ substituiert sein können, bevorzugt aber unsubstituiert sind, wobei Phenyl, Spirobifluoren-, Fluoren-, Dibenzofuran-, Dibenzothiophen-, Anthracen-, Phenanthren-, Triphenylen- Gruppen besonders bevorzugt sind-

Ferner kann vorgesehen sein, dass in der Struktur gemäß Formeln (H-1) bis (H-26) die Gruppe $Ar^2$ und/oder in Formel (QL) die Gruppe L' für eine Bindung oder eine Gruppe steht, die ausgewählt ist aus den Formeln ($L^1$-1) bis ($L^1$- 108) oder die Struktur der Formel (LAr-1) oder (LAr-2) eine Bindung darstellt oder eine Gruppe bildet die ausgewählt ist aus den Formeln ($L^1$-1) bis ($L^1$-108)

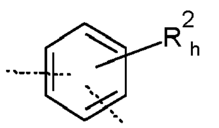

Formel ($L^1$-1)

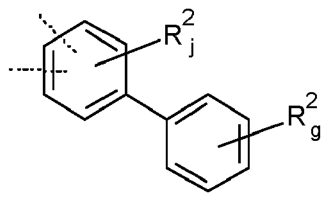

Formel ($L^1$-2)

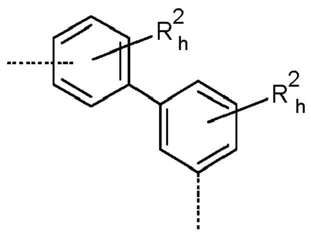

Formel ($L^1$-3)

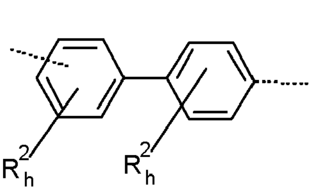

Formel ($L^1$-4)

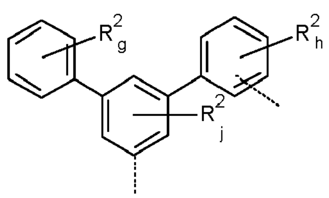

Formel ($L^1$-5)

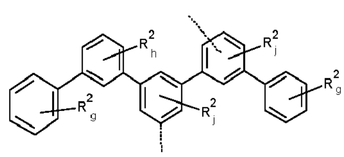

Formel ($L^1$-6)

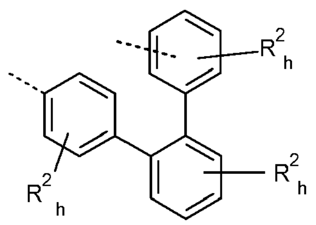

Formel ($L^1$-7)

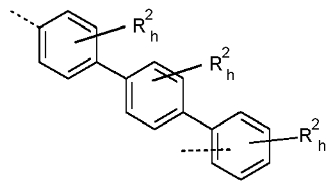

Formel ($L^1$-8)

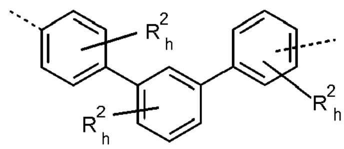

Formel ($L^1$-9)

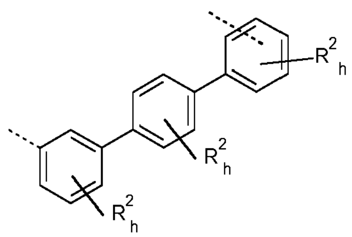

Formel ($L^1$-10)

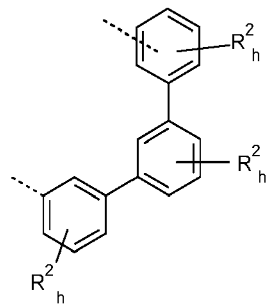

Formel ($L^1$-11)

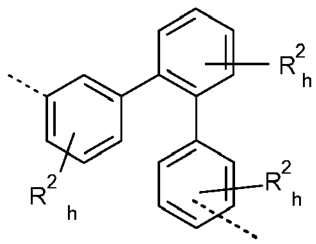

Formel ($L^1$-12)

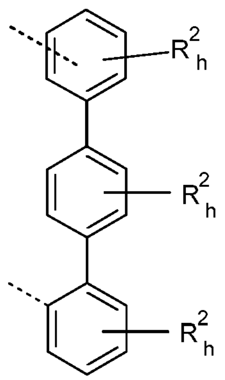

Formel ($L^1$-13)

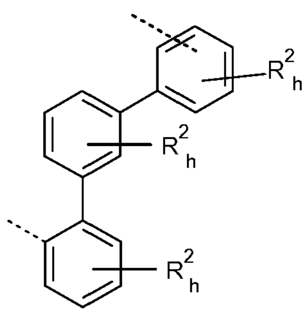

Formel ($L^1$-14)

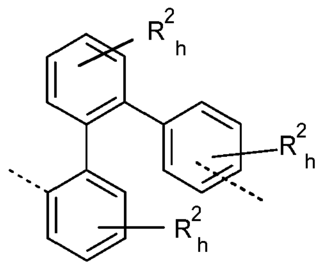

Formel ($L^1$-15)

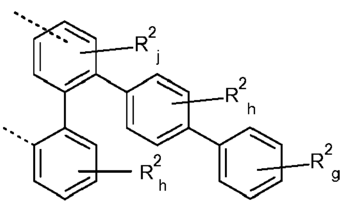

Formel ($L^1$-16)

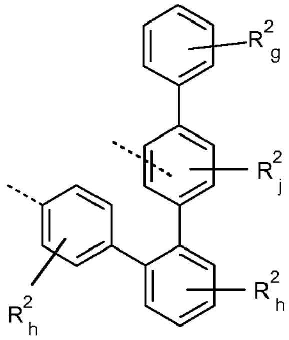

Formel ($L^1$-17)

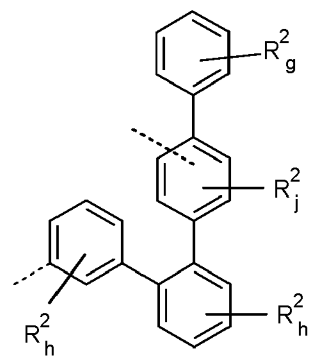

Formel ($L^1$-18)

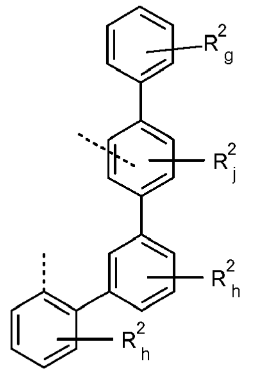

Formel ($L^1$-19)

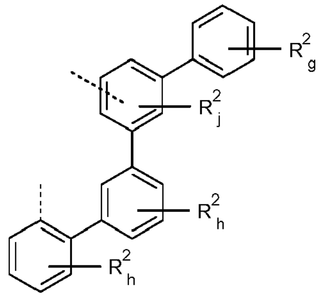

Formel ($L^1$-20)

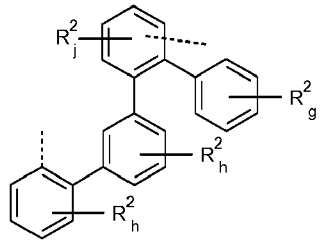

Formel ($L^1$-21)

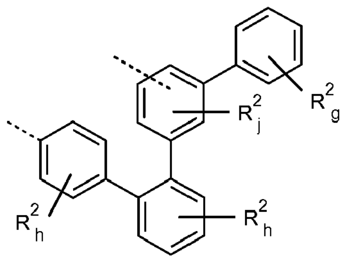

Formel ($L^1$-22)

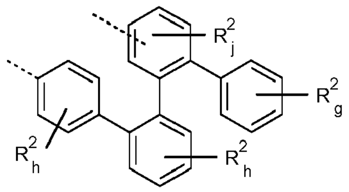

Formel ($L^1$-23)

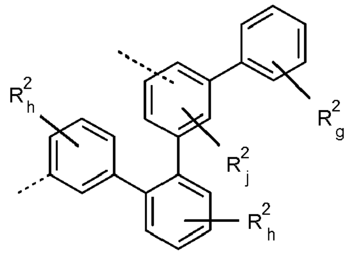

Formel ($L^1$-24)

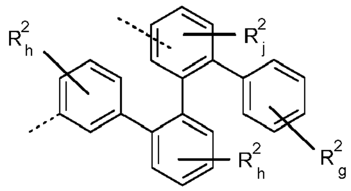

Formel ($L^1$-25)

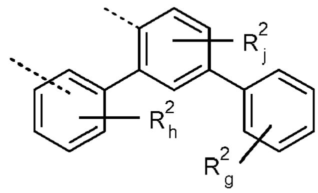

Formel ($L^1$-26)

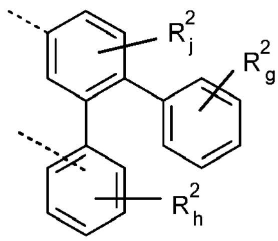

Formel ($L^1$-27)

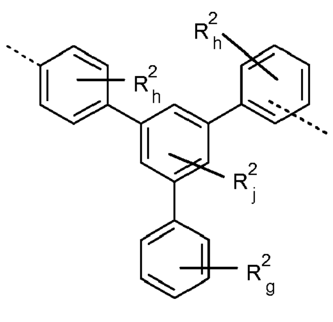

Formel ($L^1$-28)

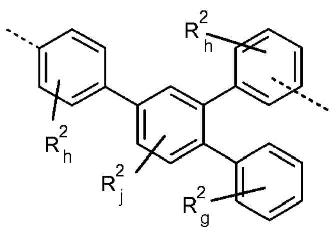

Formel ($L^1$-29)

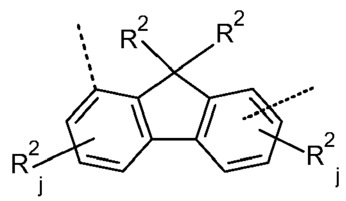

Formel ($L^1$-30)

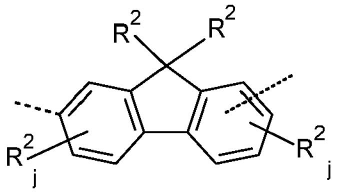
Formel ($L^1$-31)

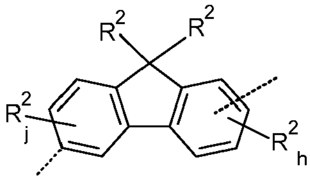
Formel ($L^1$-32)

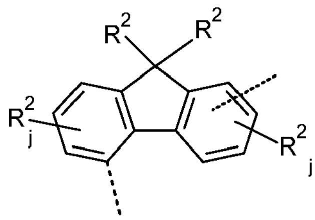
Formel ($L^1$-33)

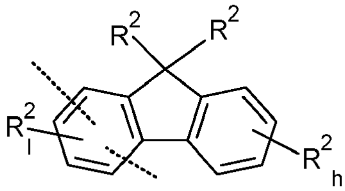
Formel ($L^1$-34)

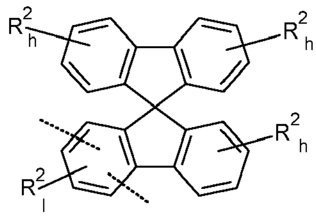
Formel ($L^1$-35)

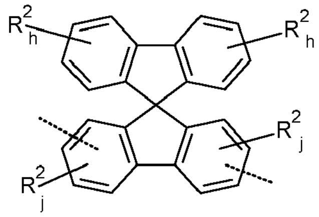
Formel ($L^1$-36)

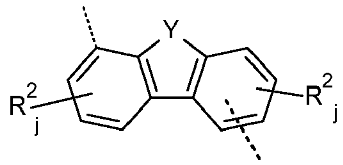
Formel ($L^1$-37)

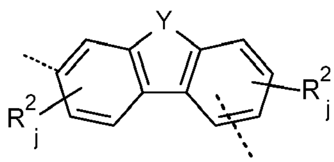
Formel ($L^1$-38)

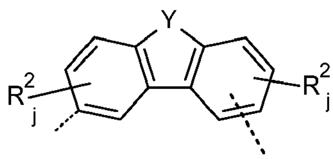
Formel ($L^1$-39)

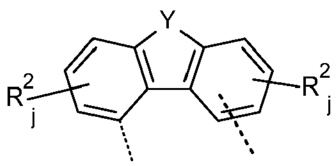
Formel ($L^1$-40)

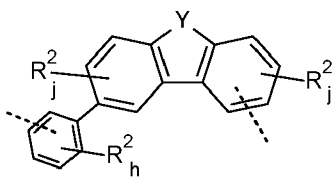
Formel ($L^1$-41)

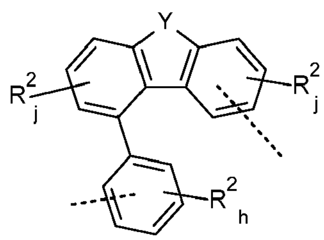
Formel ($L^1$-42)

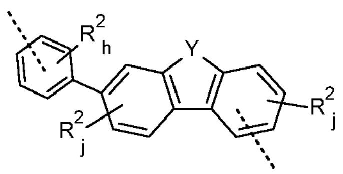
Formel ($L^1$-43)

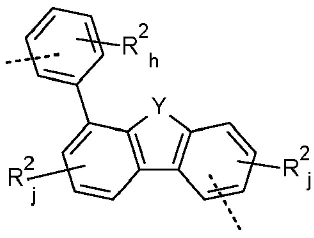
Formel ($L^1$-44)

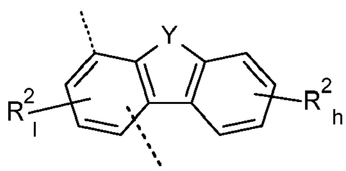
Formel ($L^1$-45)

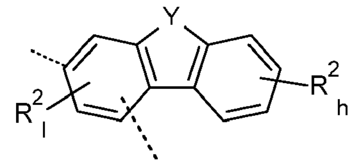

Formel ($L^1$-46)

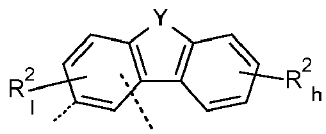

Formel ($L^1$-47)

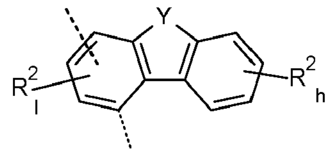

Formel ($L^1$-48)

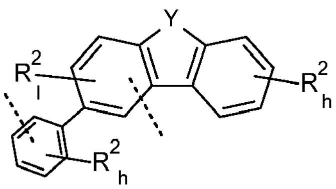

Formel ($L^1$-49)

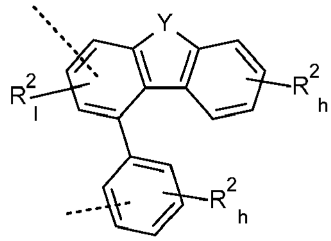

Formel ($L^1$-50)

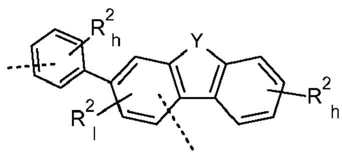

Formel ($L^1$-51)

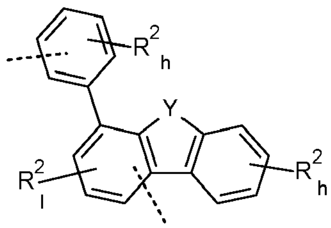

Formel ($L^1$-52)

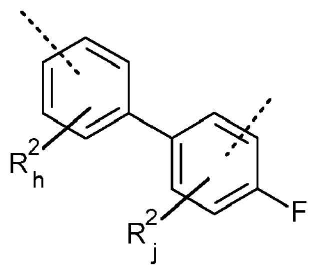

Formel ($L^1$-53)

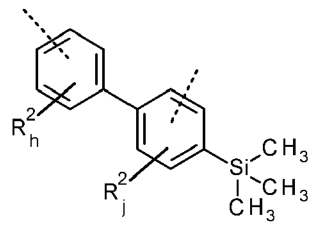

Formel ($L^1$-54)

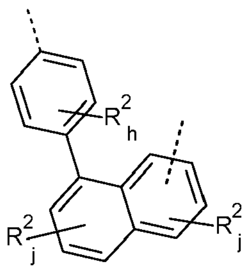

Formel ($L^1$-55)

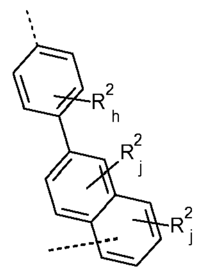

Formel ($L^1$-56)

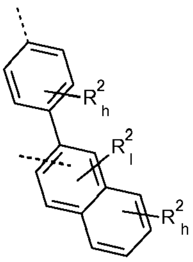

Formel ($L^1$-57)

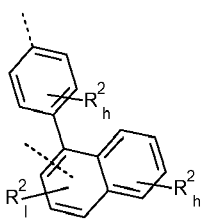

Formel ($L^1$-58)

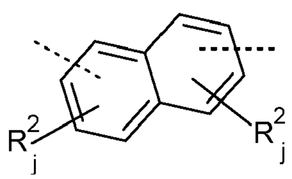

Formel ($L^1$-59)

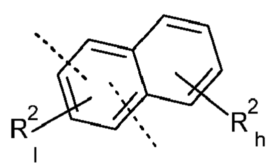

Formel ($L^1$-60)

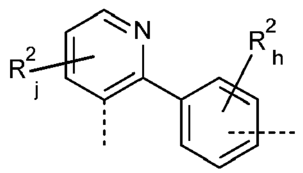

Formel ($L^1$-61)

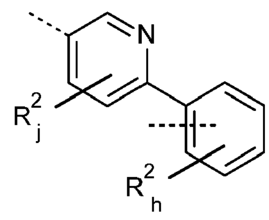

Formel ($L^1$-62)

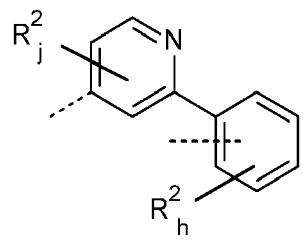

Formel ($L^1$-63)

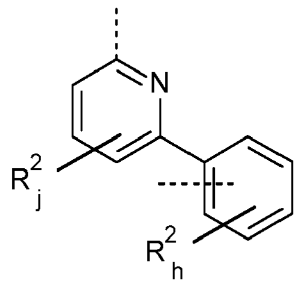

Formel ($L^1$-64)

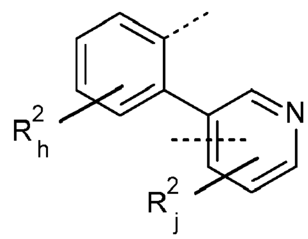

Formel ($L^1$-65)

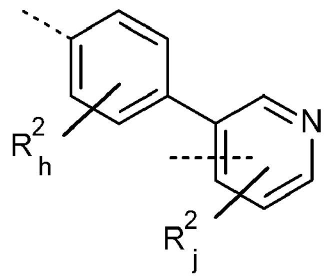

Formel ($L^1$-66)

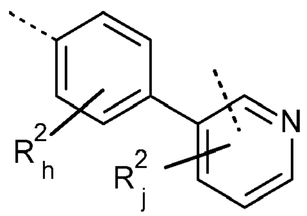

Formel ($L^1$-67)

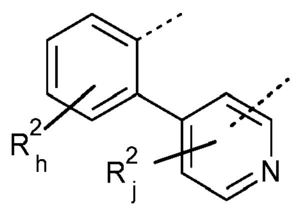

Formel ($L^1$-68)

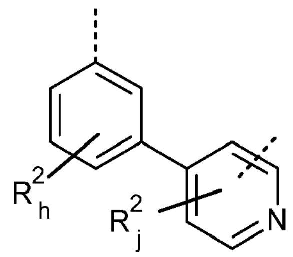

Formel ($L^1$-69)

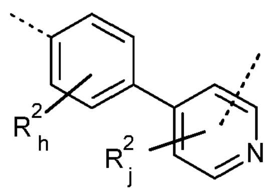

Formel ($L^1$-70)

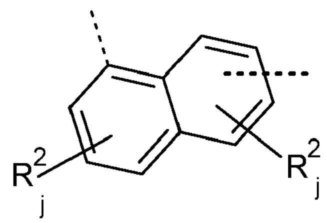

Formel ($L^1$-71)

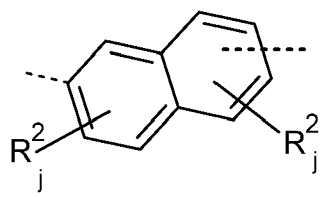

Formel ($L^1$-72)

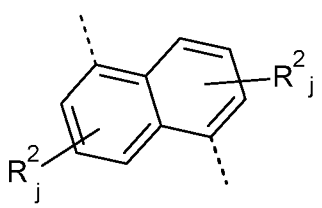

Formel ($L^1$-73)

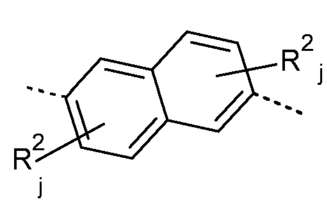

Formel ($L^1$-74)

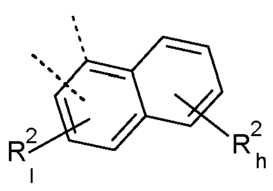

Formel ($L^1$-75)

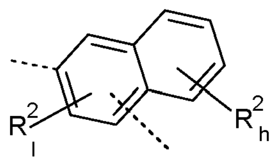

Formel ($L^1$-76)

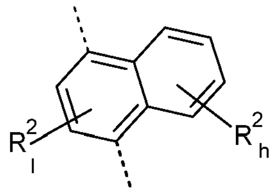

Formel ($L^1$-77)

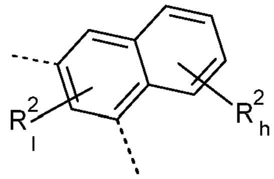

Formel ($L^1$-78)

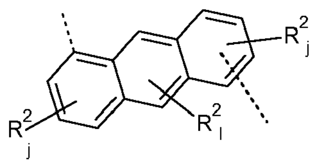

Formel ($L^1$-79)

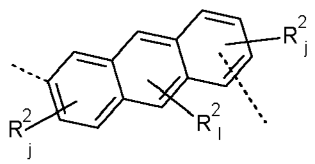

Formel ($L^1$-80)

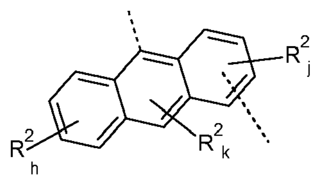

Formel ($L^1$-81)

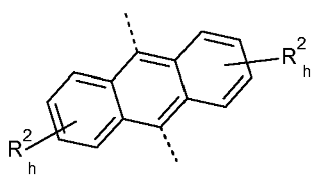

Formel ($L^1$-82)

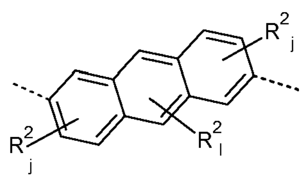

Formel ($L^1$-83)

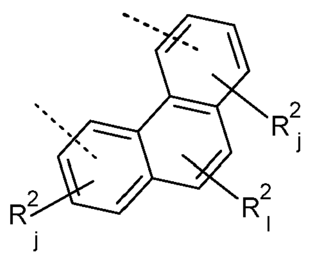

Formel ($L^1$-84)

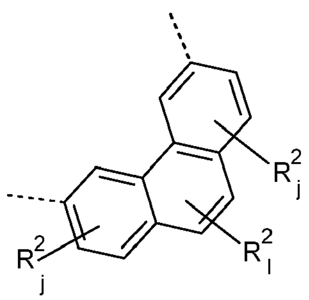

Formel ($L^1$-85)

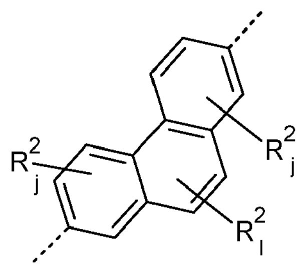

Formel ($L^1$-86)

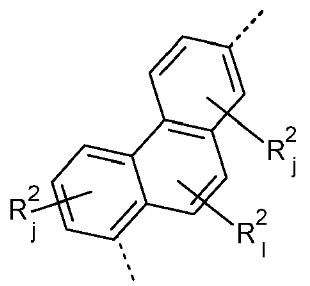

Formel ($L^1$-87)

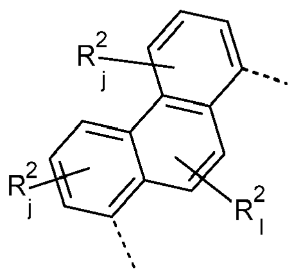

Formel ($L^1$-88)

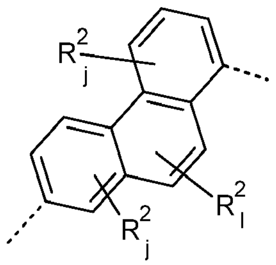

Formel ($L^1$-89)

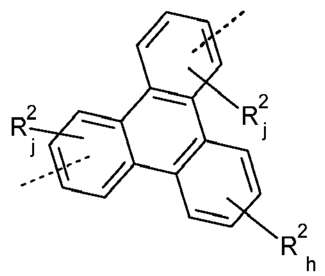

Formel ($L^1$-90)

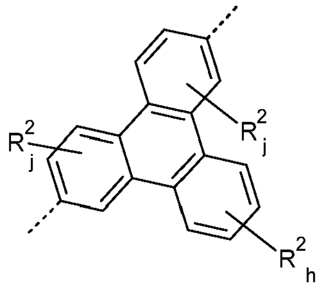

Formel ($L^1$-91)

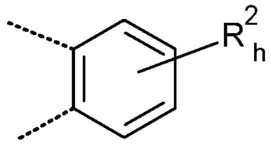

Formel ($L^1$-92)

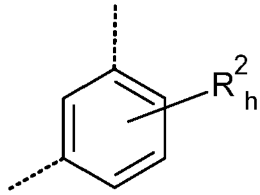

Formel ($L^1$-93)

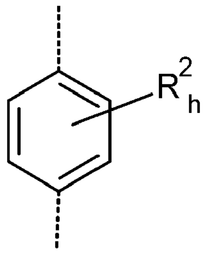

Formel ($L^1$-94)

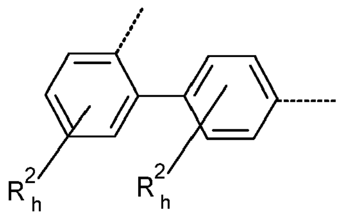

Formel ($L^1$-95)

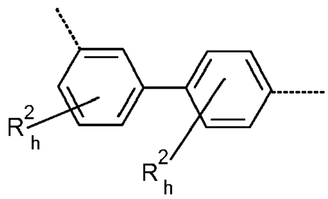

Formel ($L^1$-96)

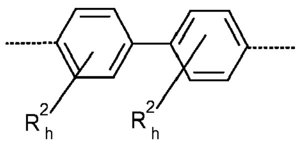

Formel ($L^1$-97)

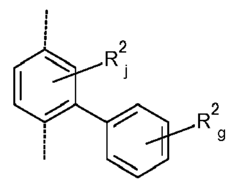

Formel ($L^1$-98)

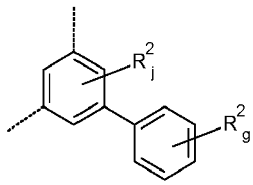

Formel ($L^1$-99)

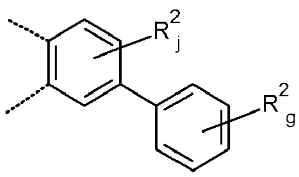

Formel ($L^1$-100)

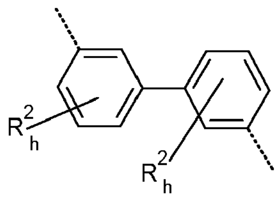

Formel ($L^1$-101)

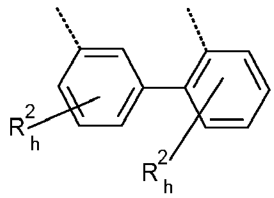

Formel ($L^1$-102)

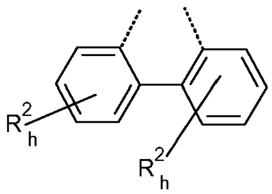

Formel ($L^1$-103)

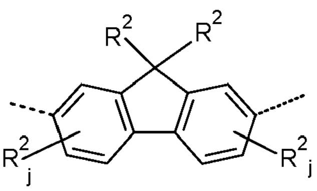

Formel ($L^1$-104)

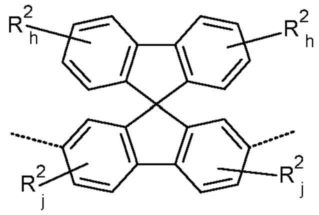

Formel ($L^1$-105)

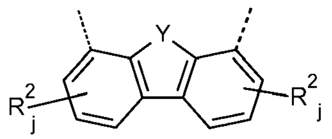

Formel ($L^1$-106)

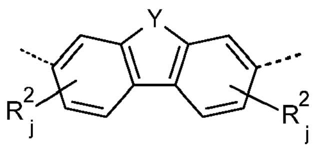

Formel ($L^1$-107)

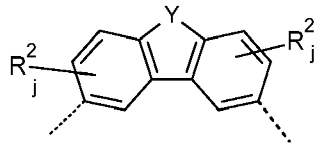

Formel ($L^1$-108)

wobei die gestrichelten Bindungen jeweils die Anbindungspositionen markieren, der Index k bei jedem Auftreten unabhängig 0 oder 1 ist, der Index l bei jedem Auftreten unabhängig 0, 1 oder 2 ist, der Index j bei jedem Auftreten unabhängig 0, 1, 2 oder 3 ist; der Index h bei jedem Auftreten unabhängig 0, 1, 2, 3 oder 4 ist, der Index g bei jedem Auftreten unabhängig 0, 1, 2, 3, 4 oder 5 ist; das Symbol Y O, S oder $NR^2$, vorzugsweise O oder S ist; und das Symbol $R^2$ die zuvor, insbesondere für Formel (I) genannte Bedeutung aufweist.

Vorzugsweise kann vorgesehen sein, dass die Summe der Indices k, l, g, h und j in den Strukturen der Formel ($L^1$-1) bis ($L^1$-108) jeweils höchstens 3, vorzugsweise höchstens 2 und besonders bevorzugt höchstens 1 beträgt.

Bevorzugte erfindungsgemäße Verbindungen umfassen eine Gruppe L' oder $Ar^2$, die eine Bindung darstellt oder die ausgewählt ist aus einer der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103). Mit Vorteil kann die Summe der Indices k, l, g, h und j in den Strukturen der Formeln ($L^1$-1) bis ($L^1$-78) und/oder ($L^1$-92) bis ($L^1$-108), bevorzugt der Formel ($L^1$-1) bis ($L^1$-54) und/oder ($L^1$ -92) bis ($L^1$-108), speziell bevorzugt der Formel ($L^1$-1) bis ($L^1$-29) und/oder ($L^1$-92) bis ($L^1$-103) jeweils höchstens 3, vorzugsweise höchstens 2 und besonders bevorzugt höchstens 1 betragen.

Bevorzugt bilden die Reste $R^2$ in den Formeln ($L^1$-1) bis ($L^1$-108) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

In einer weiteren bevorzugten Ausführungsform der Erfindung ist $R^2$, beispielsweise bei einer Struktur gemäß Formel (I) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

In einer weiteren bevorzugten Ausführungsform der Erfindung ist $R^3$, beispielsweise bei einer Struktur gemäß Formel (I) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, F, CN, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Wenn die erfindungsgemäße Verbindung mit aromatischen oder heteroaromatischen Gruppen $R^1$ bzw. $R^2$ substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

Besonders bevorzugt sind erfindungsgemäße Verbindungen, welche die folgenden Eigenschaften aufweisen:

| $Ar^a$ und $Ar^b$ | $R^a$ und $R^b$ | Akzeptorgruppe (QL) | | Donorgruppe |
|---|---|---|---|---|
| | | Q1 | $L^1$ | |
| $R^1$-1 bis $R^1$-86 | $R^1$-1 bis $R^1$-86 | Q-1 bis Q-44 | Bindung | H-1 bis H-44 |
| $R^1$-1 bis $R^1$-82 | $R^1$-1 bis $R^1$-82 | Q-1 bis Q-44 | Bindung | H-1 bis H-44 |
| $R^1$-1 bis $R^1$-54 | $R^1$-1 bis $R^1$-54 | Q-1 bis Q-44 | Bindung | H-1 bis H-44 |
| $R^1$-1 | $R^1$-1 | Q-1 bis Q-44 | Bindung | H-1 bis H-44 |

(fortgesetzt)

| $Ar^a$ und $Ar^b$ | $R^a$ und $R^b$ | Akzeptorgruppe (QL) | | Donorgruppe |
|---|---|---|---|---|
| | | Q1 | $L^1$ | |
| $R^1$-1 bis $R^1$-86 | $R^1$-1 bis $R^1$-86 | Q-1 bis Q-44 | L-1 | H-1 bis H-44 |
| $R^1$-1 bis $R^1$-82 | $R^1$-1 bis $R^1$-82 | Q-1 bis Q-44 | L-1 | H-1 bis H-44 |
| $R^1$-1 bis $R^1$-54 | $R^1$-1 bis $R^1$-54 | Q-1 bis Q-44 | L-1 | H-1 bis H-44 |
| $R^1$-1 | $R^1$-1 | Q-1 bis Q-44 | L-1 | H-1 bis H-44 |

Besonders bevorzugt sind erfindungsgemäße Verbindungen, die mindestens eine Donorgruppe gemäß Formeln (H-1) bis (H-26), wobei die Gruppe $Ar^2$ für eine verbindende Struktur der Formel (LAr-1) steht, und mindestens eine Akzeptorgruppe gemäß Formel (QL) umfassen, wobei $L^1$ durch eine verbindende Struktur der Formel (LAr-2) darstellbar ist, wobei folgende zusätzliche Eigenschaften erfüllt sind:

| $Ar^a$ und $Ar^b$ | $R^a$ und $R^b$ | Index s in Formel (LAr-1) | Index t in Formel (LAr-2) |
|---|---|---|---|
| $R^1$-1 bis $R^1$-86 | $R^1$-1 bis $R^1$-86 | 0, 1 oder 2 | 0 |
| $R^1$-1 bis $R^1$-86 | $R^1$-1 bis $R^1$-86 | 0, 1 oder 2 | 1 |
| $R^1$-1 bis $R^1$-86 | $R^1$-1 bis $R^1$-86 | 0, 1 oder 2 | 2 |
| $R^1$-1 bis $R^1$-86 | $R^1$-1 bis $R^1$-86 | 0 | 0, 1 oder 2 |
| $R^1$-1 bis $R^1$-86 | $R^1$-1 bis $R^1$-86 | 1 | 0, 1 oder 2 |
| $R^1$-1 bis $R^1$-86 | $R^1$-1 bis $R^1$-86 | 2 | 0, 1 oder 2 |
| $R^1$-1 bis $R^1$-82 | $R^1$-1 bis $R^1$-82 | 0 | 0 |
| $R^1$-1 bis $R^1$-82 | $R^1$-1 bis $R^1$-82 | 1 | 1 |
| $R^1$-1 bis $R^1$-82 | $R^1$-1 bis $R^1$-82 | 2 | 2 |
| $R^1$-1 bis $R^1$-82 | $R^1$-1 bis $R^1$-82 | 0 | 1 |
| $R^1$-1 bis $R^1$-82 | $R^1$-1 bis $R^1$-82 | 1 | 2 |
| $R^1$-1 bis $R^1$-82 | $R^1$-1 bis $R^1$-82 | 2 | 3 |
| $R^1$-1 bis $R^1$-82 | $R^1$-1 bis $R^1$-82 | 1 | 0 |
| $R^1$-1 bis $R^1$-82 | $R^1$-1 bis $R^1$-82 | 2 | 1 |
| $R^1$-1 bis $R^1$-82 | $R^1$-1 bis $R^1$-82 | 3 | 2 |
| $R^1$-1 | $R^1$-1 | 0, 1 oder 2 | 0 |
| $R^1$-1 | $R^1$-1 | 0, 1 oder 2 | 1 |
| $R^1$-1 | $R^1$-1 | 0, 1 oder 2 | 2 |
| $R^1$-1 | $R^1$-1 | 0 | 0, 1 oder 2 |
| $R^1$-1 | $R^1$-1 | 1 | 0, 1 oder 2 |
| $R^1$-1 | $R^1$-1 | 2 | 0, 1 oder 2 |
| $R^1$-1 | $R^1$-1 | 0 | 0 |
| $R^1$-1 | $R^1$-1 | 1 | 1 |
| $R^1$-1 | $R^1$-1 | 2 | 2 |
| $R^1$-1 | $R^1$-1 | 0 | 1 |
| $R^1$-1 | $R^1$-1 | 1 | 2 |
| $R^1$-1 | $R^1$-1 | 2 | 3 |

(fortgesetzt)

| $Ar^a$ und $Ar^b$ | $R^a$ und $R^b$ | Index s in Formel (LAr-1) | Index t in Formel (LAr-2) |
|---|---|---|---|
| $R^1$-1 | $R^1$-1 | 1 | 0 |
| $R^1$-1 | $R^1$-1 | 2 | 1 |
| $R^1$-1 | $R^1$-1 | 3 | 2 |

In den zuvor dargelegten Tabellen bedeutet die Zuordnung $Ar^a$ und $Ar^b$ ist $R^1$-1 bis $R^1$-86, dass sowohl die Gruppe $Ar^a$ als auch die Gruppe $Ar^b$ ausgewählt ist aus Resten der zuvor dargelegten Formeln $R^1$-1 bis $R^1$-86, vorzugsweise $R^1$-1. Die Zuordnung $R^a$ und $R^b$ bedeutet, dass sowohl die Gruppe $R^a$ als auch die Gruppe $R^b$ ausgewählt ist aus Resten der zuvor dargelegten Formeln $R^1$-1 bis $R^1$-86, vorzugsweise $R^1$-1. Die weiteren Zuordnungen gelten entsprechend.

Beispiele für geeignete erfindungsgemäße Verbindungen sind die nachstehend gezeigten Strukturen gemäß den folgenden Formeln 1 bis 51:

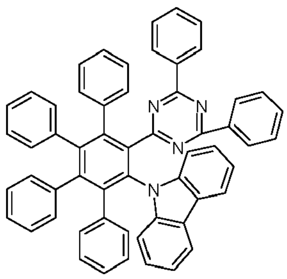

Formel 1

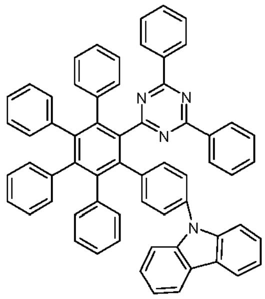

Formel 2

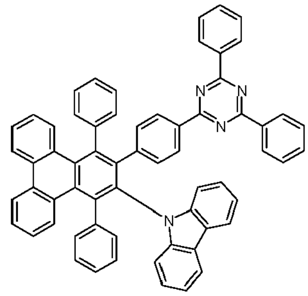

Formel 3

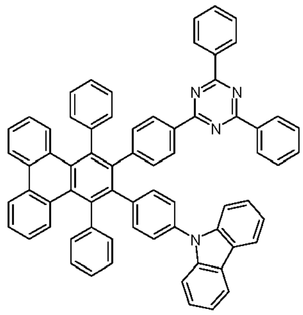

Formel 4

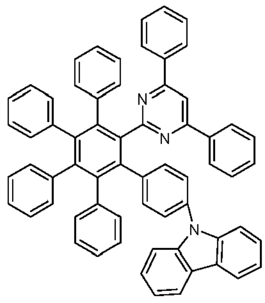

Formel 5

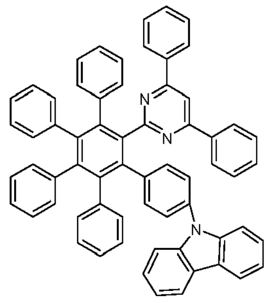

Formel 6

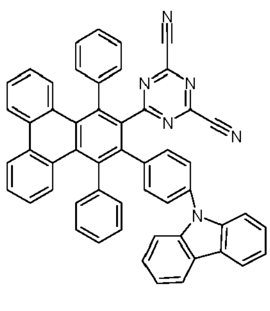

Formel 7

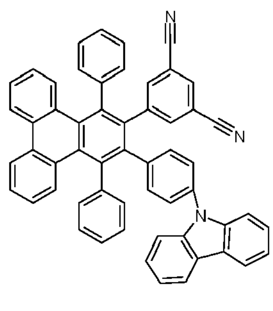

Formel 8

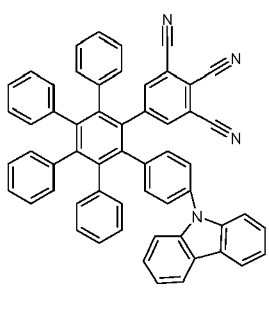

Formel 9

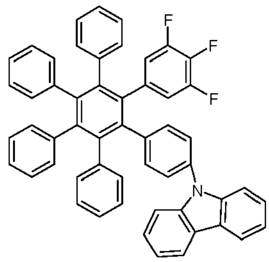

Formel 10

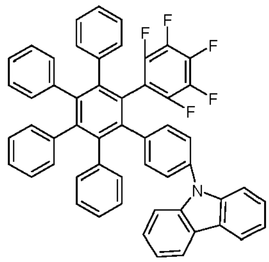

Formel 11

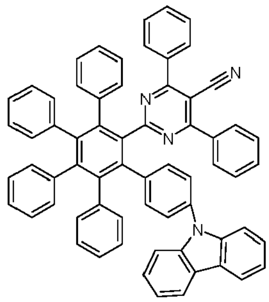

Formel 12

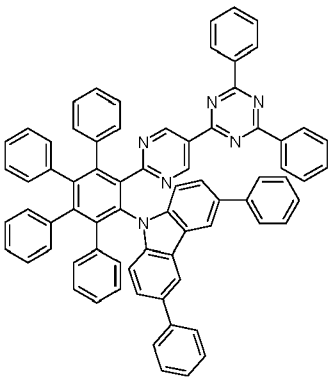

Formel 13

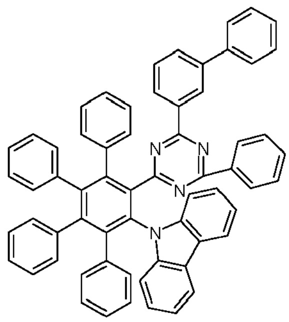

Formel 14

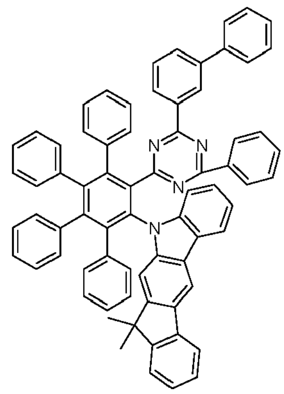

Formel 15

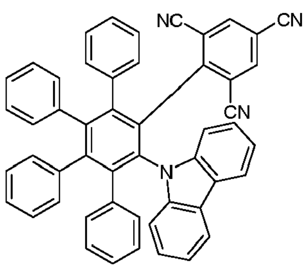

Formel 16

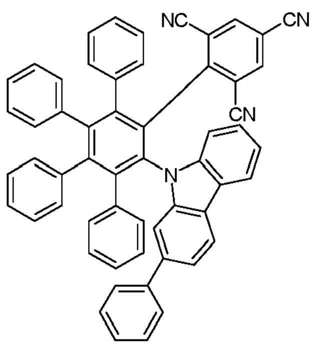

Formel 17

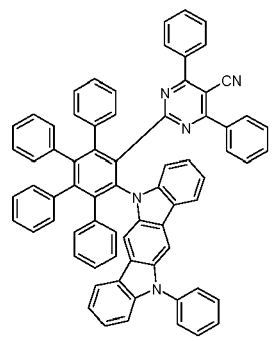

Formel 18

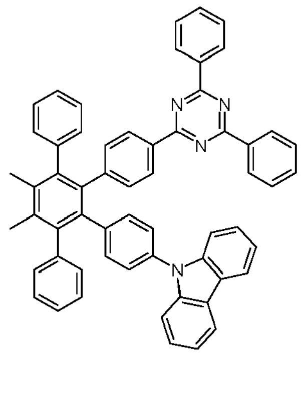

Formel 19

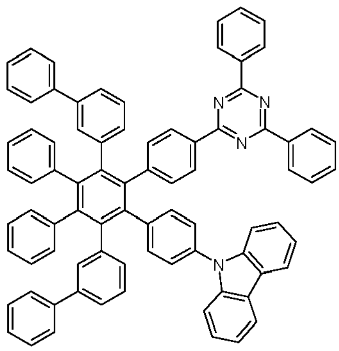

Formel 20

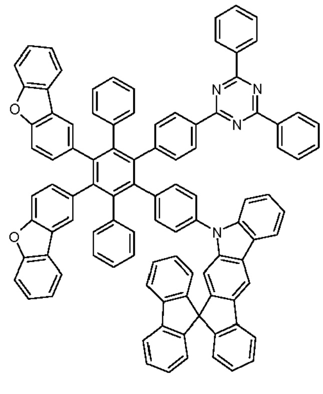

Formel 21

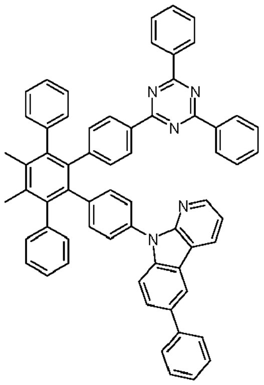

Formel 22

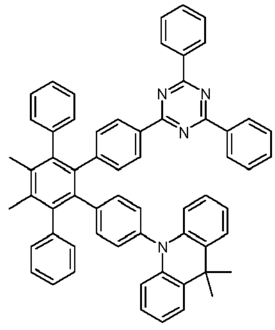

Formel 23

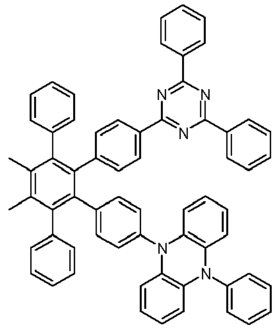

Formel 24

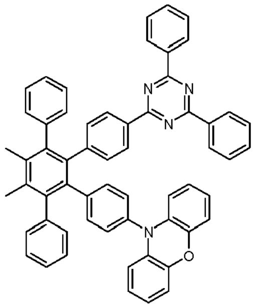

Formel 25

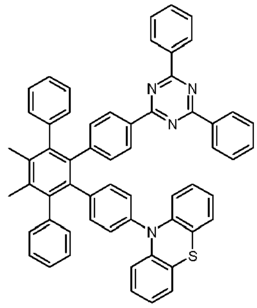

Formel 26

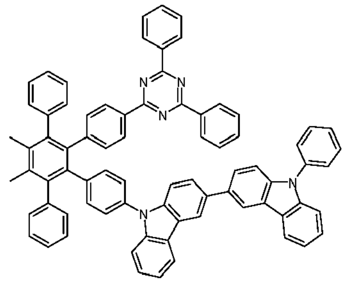

Formel 27

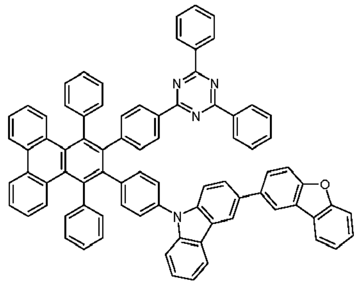

Formel 28

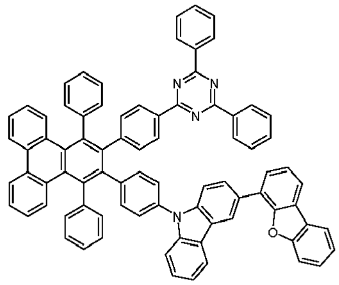

Formel 29

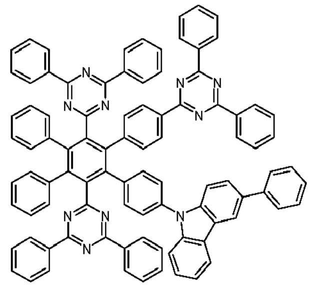

Formel 30

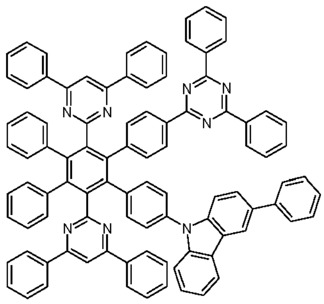

Formel 31

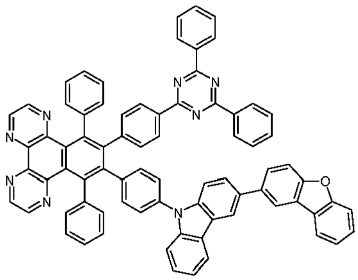

Formel 32

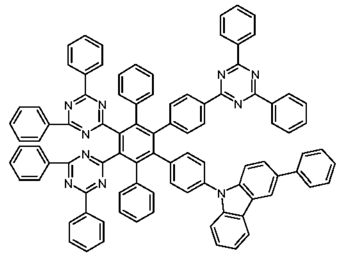

Formel 33

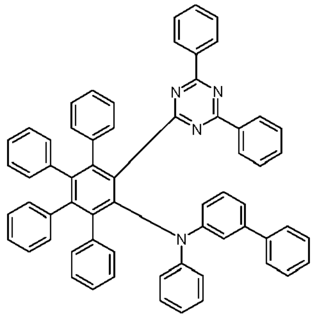

Formel 34

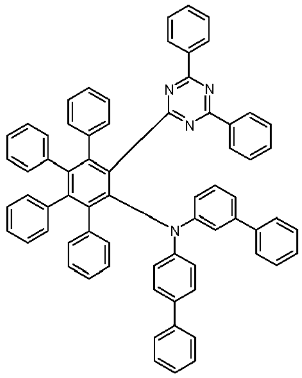

Formel 35

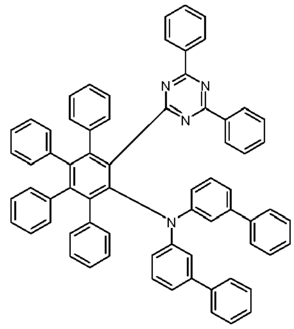

Formel 36

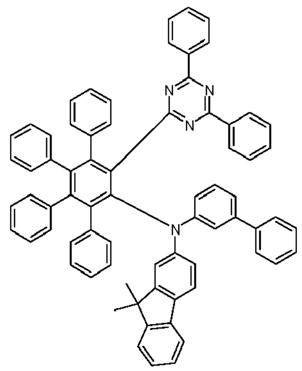

Formel 37

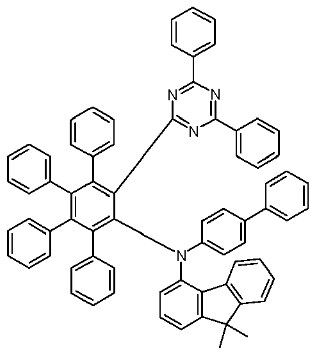

Formel 38

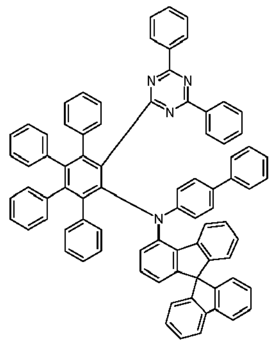

Formel 39

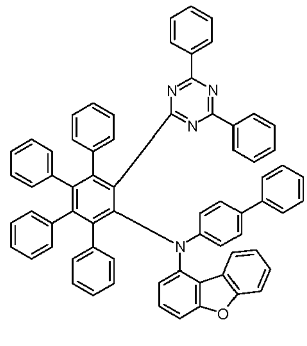

Formel 40

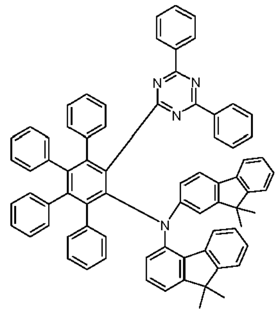

Formel 41

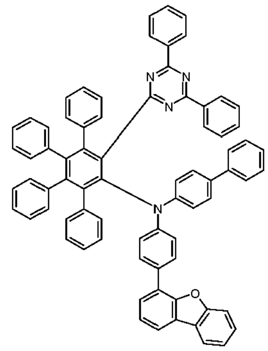

Formel 42

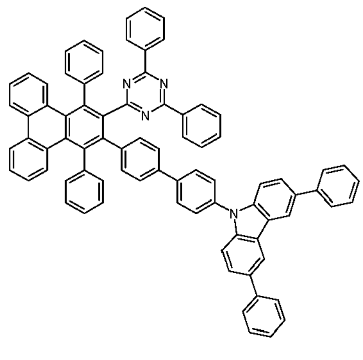

Formel 43

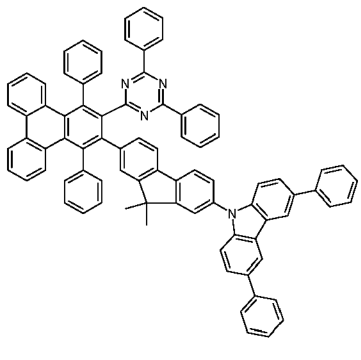

Formel 44

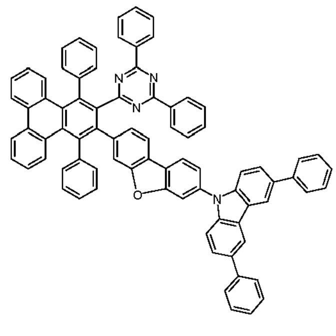

Formel 45

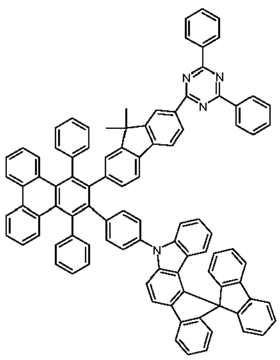

Formel 46

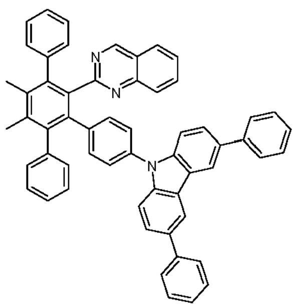

Formel 47

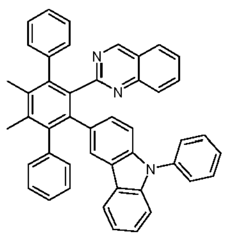

Formel 48

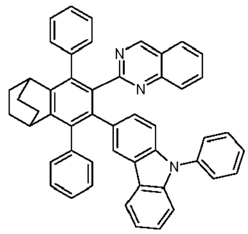

Formel 49

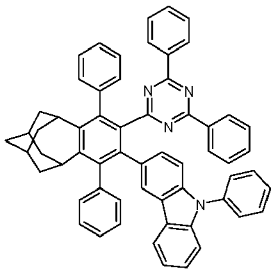

Formel 50

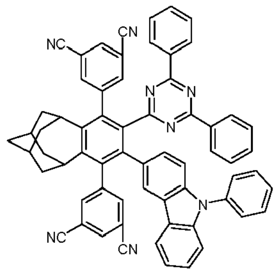

Formel 51

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der Verbindungen, umfassend mindestens eine Struktur gemäß Formel (I), bei dem in einer Kupplungsreaktion eine Verbindung, umfassend mindestens eine Donorgruppe, mit einer Akzeptorgruppe verbunden wird.

Geeignete Verbindungen mit einer Donorgruppe können vielfach kommerziell erhalten werden, wobei die in den Beispielen dargelegten Ausgangsverbindungen durch bekannte Verfahren erhältlich sind, so dass hierauf verwiesen wird.

Vorzugsweise kann ein Cyclopentadienon-Derivat mit einem Alkin-Derivat umgesetzt werden, wobei dies in einer Diels-Alder-Reaktion erfolgen kann. Das Diels-Alder-Produkt reagiert anschließend unter Abspaltung von CO zu einer erfindungsgemäßen Verbindung. Diese Reaktion ist insbesondere für den Fall von Vorteil, dass die Gruppen $Ar^a$, $Ar^b$, $R^a$ und $R^b$ Aryl- oder Heteroarylgruppen darstellen.

Hierbei können entsprechende Alkinderivate von Verbindungen, die vorzugsweise eine Donor- und/oder eine Akzeptorgruppe umfassen, durch Umsetzung von Verbindungen, die entsprechende reaktive Gruppen enthalten, mit aromatischen oder heteroaromatischen Alkinverbindungen erhalten werden. Hierfür geeignete Kupplungsreaktionen, z. B. Suzuki-Kupplung, sind allgemein bekannt, wobei sich die sogenannte Sonogashira-Reaktion hierfür als besonders zweckmäßig erwiesen hat. Die Umsetzungsbedinungen für eine Suzuki-Kupplung oder eine Sonogashira-Reaktion sind in der Fachwelt weithin bekannt, wobei in diesem Zusammenhang die Beispiele wertvolle Hinweise liefern.

Die als Edukt für eine Suzuki-Kupplung oder eine Sonogashira-Reaktion einzusetzenden reaktiven Eduktverbindungen können beispielsweise durch bekannte Halogenierungen, vorzugsweise Bromierungen aus bekannten Aryl- oder Heteroarylverbindungen erhalten werden.

Diese Verbindungen können durch bekannte Kupplungsreaktionen mit weiteren Arylverbindungen umgesetzt werden, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Durch diese Verfahren, gegebenenfalls gefolgt von Aufreinigung, wie z. B. Umkristallisation oder Sublimation, lassen sich die erfindungsgemäßen Verbindungen, umfassend Strukturen gemäß Formel (I) in hoher Reinheit, bevorzugt mehr als 99 % (bestimmt mittels $^1$H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl-oder verzweigte Terphenyl- oder Quaterphenylgruppen, die eine Löslichkeit in gängigen organischen Lösemitteln bewirken, wie beispielsweise Toluol oder Xylol bei Raumtemperatur in ausreichender Konzentration löslich, um die Verbindungen aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen, umfassend mindestens eine Struktur der Formel (I) bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formel (I) oder erfindungsgemäße Verbindungen, wobei ein oder mehrere Bindungen der erfindungsgemäßen Verbindungen oder der Strukturen der Formel (I) zum Polymer, Oligomer oder Dendrimer vorhanden sind. Je nach Verknüpfung der Strukturen der Formel (I) bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (I) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen umfassend Strukturen der allgemeinen Formel (I) bzw. die zuvor und nachfolgend ausgeführten bevorzugten Ausführungsformen bevorzugt, die eine Glasübergangstemperatur von mindestens 70°C, besonders bevorzugt von mindestens 110°C, ganz besonders bevorzugt von mindestens 125°C und insbesondere bevorzugt von mindestens 150°C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, insbesondere ein phosphoreszierender Dotand, und/oder ein weiteres Matrixmaterial. Diese weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, HostMaterialien, Elektronentransportmaterialien, Elektroneninjektionsmaterialien, Lochleitermaterialien, Lochinjektionsmaterialien, Elektronenblockiermaterialien, Lochblockiermaterialien, Wide-Band-Gap-Materialien und n-Dotanden.

Die vorliegenden Erfindung betrifft daher auch eine Zusammensetzung enthaltend wenigstens eine Verbindung umfassend Strukuren gemäß Formel (I) bzw. die zuvor und nachfolgend ausgeführten bevorzugten Ausführungsformen sowie wenigstens ein Matrixmaterial. Das Matrixmaterial wird auch als Hostmaterial bezeichnet. Hierbei kann die erfindungsgemäße Verbindung Eigenschaften eines Matrixmaterials oder eines Emitters aufweisen. Falls die Verbindung umfassend Strukuren gemäß Formel (I) bzw. die zuvor und nachfolgend ausgeführten bevorzugten Ausführungsformen als Matrixmaterial eingesetzt wird, wird hierin ein sich von den erfindungsgemäßen Verbindungen unterschiedliches Hostmaterial insbesondere auch als weiteres Matrixmaterial bezeichnet. Gemäß einem besonderen Aspekt der vorliegenden Erfindung weist das weitere Matrixmaterial lochtransportierende Eigenschaften auf.

Das Matrixmaterial kann insbesondere gemäß der Art der elektronischen Vorrichtung, insbesondere der Verwendung der erfindungsgemäßen Verbindung ausgewählt werden. Falls die erfindungsgemäße Verbindung beispielsweise als Emitter, beispielsweise in organischen Elektrolumineszenzvorrichtungen oder organischen lichtemittierenden elektrochemische Zellen eingesetzt wird, weist das Hostmaterial bevorzugt eine größere Energielücke auf als die Verbindung umfassend Strukturen gemäß Formel (I), die erfindungsgemäß als fluoreszierendes TADF-Material eingesetzt wird.

Vorzugsweise wirkt das Hostmaterial als Elektronen- bzw. Lochleiter bzw. als kombinierter Elektronen-/Lochleiter.

Gemäß einer Ausführungsform überlappt das Absorptionsspektrum der als als fluoreszierendes TADF-Material eingesetzten erfindungsgemäßen Verbindung mit dem Photolumineszenzspektrum des zumindest einen Hostmaterials, sodass ein Energieübergang zwischen der erfindungsgemäßen Verbindung und dem zumindest einen Hostmaterial erfolgen kann. An sich können Hostmaterialien verwendet werden, wie sie bei der Anwendung in OLECs oder OLEDs bekannt bzw. üblich sind.

Gemäß einer Ausführungsform ist das Hostmaterial ein loch- oder elektronen-transportierendes Material.

Gemäß einer Ausführungsform weist das Hostmaterial einen niedrigsten Triplett-Zustand $T_1^H$ auf, der eine Energie aufweist, welche höher liegt als die Energie des niedrigsten Triplett-Zustands $T_1$ der neutralen organischen lumineszierenden Verbindung.

Geeignete Hostmaterial sind beispielsweise ausgewählt aus Anthracenen, Benzanthracenen, Indenofluorenen, Fluorenen, spiro-Bifluorenen, Phenanthrenen, Dehydrophenanthrenen, Dehydrofluorenen, Thiophenen, Triazinen, Carbazolen, Indenocarbazolen, Indolocarbazolen, Pyrimidinen, Lactamen, Benzophenonen, Triarlyaminen, Chinazolinen und Imidazolen.

Ganz bevorzugte Hostmaterialien sind ausgewählt aus aus Indenofluorenen, Fluorenen, spiro-Bifluorenen, Phenanthrenen, Dehydrophenanthrenen, Dehydrofluorenen, Thiophenen, Triazinen, Carbazolen, Indenocarbazolen, Indolocarbazolen, Pyrimidinen, Lactamen, Benzophenonen, Triarlyaminen, Chinazolinen und Imidazolen.

Gemäß einer Ausführungsform umfasst die Zusammensetzung vier oder weniger Hostmaterialien, gemäß einer weiteren Ausführungsform drei oder weniger Hostmaterialein, und gemäß einer weiteren Ausführungsform ein oder zwei Hostmaterialien.

Besonders bevorzugte Hostmaterialien sind ausgewählt aus der Gruppe der Oligoarylene, wie beispielsweise 2,2',7,7'-tetraphenylspirobifluoren, welches in der EP 676461 beschrieben ist, oder Dinaphthylanthracen. Bevorzugt sind Oligoarylene, welche kondensierte aromatische Gruppen umfassen, wie beispielsweise Phenanthren, Tetracen, Coronen, Chrysen, Fluoren, Spirofluoren, Perylen, Phthaloperylen, Naphthaloperylen, Decacyclen, Rubren, Oligoarylenvinylene, wie beispielsweise 4,4'-bis(2,2-diphenylethenyl)-1,1 '-biphenyl (DPVBi) oder 4,4-bis-2,2-diphenylvinyl-1,1 - spirobiphenyl (spiro-DPVBi), wie sie in der EP 676461 beschrieben sind.

Weitere geeignete Hostmaterialien sind polypodale Metallkomplexe, wie sie beispielsweise in der WO

2004/081017 beschrieben werden, beispielsweise Metallkomplexe mit 8-Hydroxychinolin, wie Aluminium(III)-tris-(8-hydroxychinolin) (Alqs), oder bis-(2-methyl-8-chinolinolato)-4-(phenylphenolinolato)aluminium, Imidazol-Chelat verbindungen, wie sie in der US 2007/0092753 A1 beschrieben sind, sowie Chinolin-Metallkomplexe, Aminochinoin-Metallkomplexe, Benzochinolin-Metallkomplexe, Loch-transportierende Materialien, wie sie beispielsweise in der WO 2004/058911 beschrieben sind, Elektronen-transportierende Materialien, insbesondre Ketone, Phosphinoxide, Sulfoxide, wie sie beispielsweise in der WO 2005/084081 und WO 2005/08408 beschrieben sind, Atropisomere, wie sie in der WO 2006/048268 beschrieben sind, Boronsäurederivate, wie sie beispielsweise in der WO 2006/117052 beschrieben sind, oder Benzanthrazene, wie sie in der DE 102007024850 beschrieben sind.

Weitere geeignete Hostmaterialien sind Oligoarylene, wie Naphthalin, Anthracen, Benzanthracen, und Pyren, sowie Atropisomere dieser Verbindungen, Ketone, Phosphinoxide, und Sulfoxide. Bevorzugt wird das Hostmaterial ausgewählt aus der Klasse der Oligoarylene, insbesondere Naphthalin, Anthracen, Benzanthracen, und Pyren. Unter einer Oligoarylverbindung wird im Sinne dieser Erfindung eine Verbindung verstanden, welche zumindest drei miteinander verbundene Aryl- oder Arylengruppen umfasst.

Weitere geeignete Hostmaterialien sind ausgewählt aus Verbindungen der Formel (HM-1):

$$Ar^5\text{-}(Ar^6)_p\text{-}Ar^7 \qquad \text{Formel (HM-1)}$$

wobei bedeutet:

$Ar^5$, $Ar^6$, $Ar^7$ bei jedem Auftreten gleich oder verschieden eine Aryl- oder Heteroarylgruppe mit 5 bis 30 aromatischen Ringen, die auch ein- oder mehrfach substituiert sein können,

$p = 1, 2$ oder 3;

wobei die Summe der $\pi$-Elektronen in den Gruppen $Ar^5$, $Ar^6$, $Ar^7$ zumindest 30 beträgt, wenn $p = 1$, und zumindest 36 beträgt, falls $p = 2$, und zumindest 42 beträgt, falls $p = 3$.

Innerhalb der Hostmaterialien der Formel (HM-1) bedeutet $Ar^6$ bevorzugt Anthracen, welches mit einem oder mehreren Resten $R^1$ substituiert sein kann, wobei die Gruppen $Ar^5$ und $Ar^7$ gemäß einer weiteren Ausführungsform an den Positionen 9 und 10 gebunden sind. Besonders bevorzugt ist zumindest eine der Gruppen $Ar^5$ und $Ar^7$ eine kondensierte Arylgruppe, welche bevorzugt ausgewählt ist aus der Gruppe von 1- oder 2-naphthyl, 2-, 3- oder 9-phenanthrenyl oder 2-, 3-, 4-, 5-, 6- oder 7-benzanthracenyl, die jeweils einfach oder mehrfach durch $R^1$ substituiert sein können. $R^1$ ist dabei wie oben definiert.

Geeignete Anthracenverbindungen sind beispielsweise beschrieben in US 2007/0092753 A1 und US 2007/0252517 A1, wie beispielsweise 2-(4-Methylphenyl)-9,10-di-(2-naphthyl)anthracen, 9-(2-Naphthyl)-10-(1,1'-biphenyl)anthracen und 9,10-bis[4-(2,2-Diphenylethenyl)phenyl]anthracen, 9,10-Diphenylanthracen, 9,10-bis(Phenylethynyl)anthracen und 1,4-bis(9'-Ethynylanthracenyl)benzol. Bevorzugt sind auch Hostmaterialien mit zumindest zwei Anthracengruppen (US 2008/0193796 A1), wie beispielsweise 10,10'-bis[1,1',4',1"]Terphenyl-2-yl-9,9'-bisanthracenyl.

Weitere geeignete Hostmaterialien sind Derivate von Arlyaminen, Styryolaminen, Fluorescein, Perynon, Phthaloperynon, Naphthaloperynon, Diphenylbutadien, Tetraphenylbutadien, Cyclopentadiene, Tetraphenylcyclopentadien, Pentaphenylcyclopentadien, Cumarin, Oxadiazol, Bisbenzoxazolin, Oxazon, Pyridin, Pyrazin, Imine, Benzothiazole, Benzoxazole, Benzimidazole (US 2007/0092753 A1), wie beispielsweise 2,2',2"-(1,3,5-phenylen)tris[1-phenyl-1H-benzimidazol], Aldazine, Stilben, Styrylarylen Derivative, wie beispielsweise 9,10-bis[4-(2,2-Diphenylethenyl)phenyl]anthracen, und Distyrylarylenderivative (US 5121029), Diphenylethylen, Vinylanthracen, Diaminocarbazol, Pyran, Thiopyran, Diketopyrrolopyrrol, Polymethin, Mellocyanin, Acridon, Chinacridon, Zimtsäureester sowie Fluoreszenzfarbstoffe.

Gemäß einer bevorzugten Ausführungsform werden Arylamin- sowie Styrylaminderivate als Hostmaterialien verwendet, wie beispielsweise 4,4'-bis[N-(1-naphthyl)-N-(2-naphthyl)amino]biphenyl (TNB).

Als Hostmaterial geeignete Oligoarylene sind beispielsweise beschrieben in US 2003/0027016 A1, US 7326371 B2, US 2006/043858 A, US 7326371 B2, US 2003/0027016 A1, WO 2007/114358, WO 2008/145239, JP 3148176 B2, EP 1009044, US 2004/018383, WO 2005/061656 A1, EP 0681019B1, WO 2004/013073A1, US 5077142, WO 2007/065678, sowie US 2007/0205412 A1.

Als Hostmaterial besonders bevorzugte Verbindungen sind in den folgenden Formeln (HM-2) bis (HM-8) wiedergegeben:

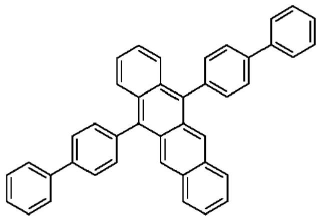
Formel (HM-2)

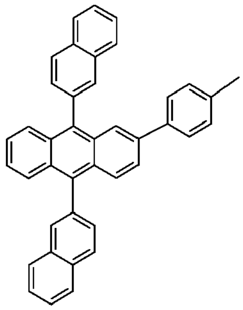
Formel (HM-3)

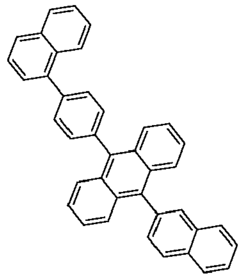
Formel (HM-4)

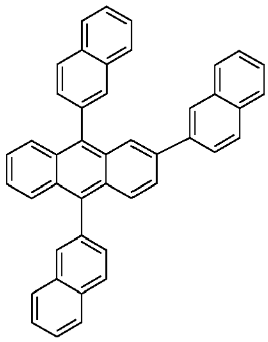
Formel (HM-5)

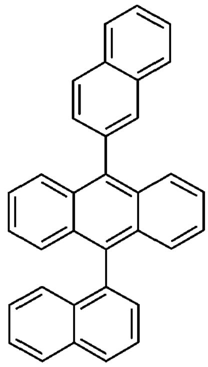
Formel (HM-6)

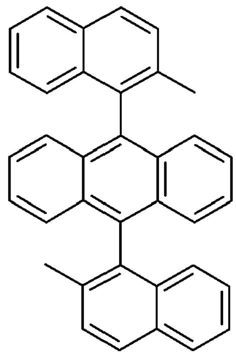
Formel (HM-7)

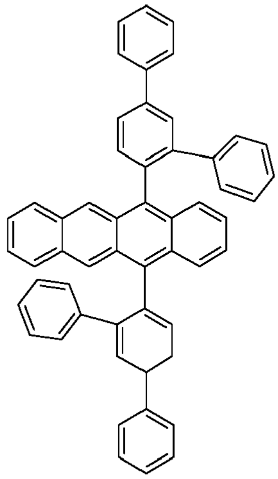
Formel (HM-8)

Weitere geeignete Hostmaterialien sind Spirobifluoren und Derivate hiervon, beispielsweise das in der EP 0676461 beschriebene Spiro-DPVBi oder Indenofluoren, welches in der US 6562485 beschrieben ist.

Die Kombination von erfindungsgemäßen Verbindungen, die als Emitter oder als TADF-Material eingesetzt werden, mit den zuvor genannten Hostmaterialien, kann zur Bildung einer emittierenden Schicht in einer OLEC entsprechend durch einen Elektrolyten, welcher bewegliche Ionen zur Verfügung stellt, ergänzt werden, wie diese aus dem Stand der Technik, der insbesondere im einleitenden Teil dieser Anmeldung dargestellt ist, bekannt sind.

Ein weiterer Gegenstand der vorliegenden stellt eine Zusammensetzung dar, enthaltend wenigstens eine Verbindung, umfassend mindestens eine Struktur gemäß Formel (I) bzw. die zuvor und nachfolgend ausgeführten bevorzugten Ausführungsformen sowie wenigstens ein Wide-Band-Gap-Material, wobei unter Wide-Band-Gap-Material ein Material im Sinne der Offenbarung von US 7,294,849 verstanden wird. Diese Systeme zeigen besondere vorteilhafte Leistungsdaten in elektrolumineszierenden Vorrichtungen.

Vorzugsweise kann die zusätzliche Verbindung eine Bandlücke (band gap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31 G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Ferner kann eine erfindungsgemäße Verbindung als Matrixmaterial in Kombination mit einem Emitter eingesetzt werden.

Daher betrifft die vorliegende Erfindung auch eine Zusammensetzung umfassend wenigstens eine Verbindung mit Strukuren gemäß Formel (I) bzw. die zuvor und nachfolgend ausgeführten bevorzugten Ausführungsformen sowie wenigstens einen fluoreszierenden Emitter, wobei unter dem Begriff fluoreszierende Emitter auch fluoreszierende Dotanden verstanden werden. Vorzugsweise kann vorgesehen sein, dass das Gewichtsverhältnis von fluoreszierendem Emitter zu Verbindung, Oligomer, Polymer oder Dendrimer gemäß der vorliegenden Erfindung im Bereich von 0,1 bis 50 Gew.-% liegt, 1 bis 20 Gew.-% und 3 bis 15 Gew.-% liegt.

Die vorliegende Erfindung betrifft auch eine Zusammensetzung umfassend wenigstens eine Verbindung umfassend Strukuren gemäß Formel (I) bzw. die zuvor und nachfolgend ausgeführten bevorzugten Ausführungsformen sowie wenigstens einen phosphoreszierende Emitter, wobei unter dem Begriff phosphoreszierende Emitter auch phosphoreszierende Dotanden verstanden werden.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Matrix-Systemen, vorzugsweise Mixed-Matrix-Systemen sind die im Folgenden angebenen bevorzugten phosphoreszierenden Dotanden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die

Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2016/124304, WO 2016/015815, WO 2016/000803, WO 2015/117718, WO 2015/104045 und WO 2015/036074 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Explizite Beispiele für phosphoreszierende Dotanden sind in der folgenden Tabelle aufgeführt.

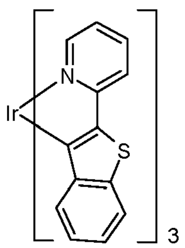
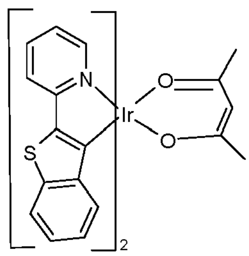
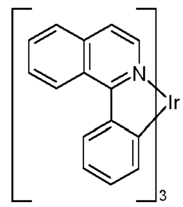

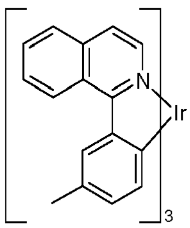
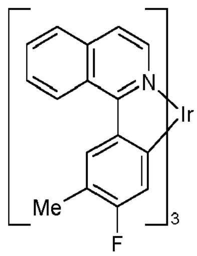
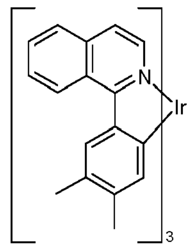

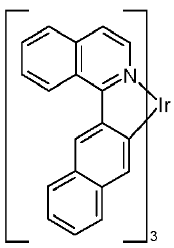
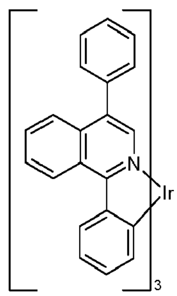
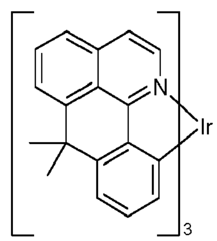

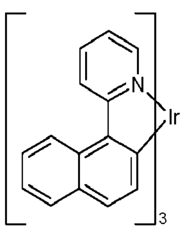
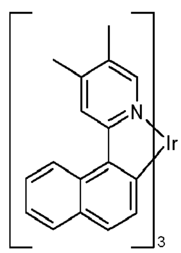
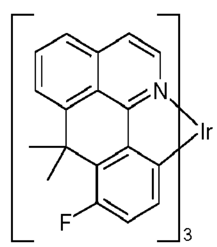

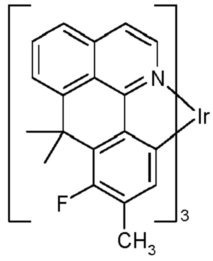
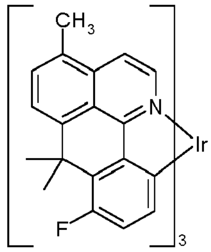
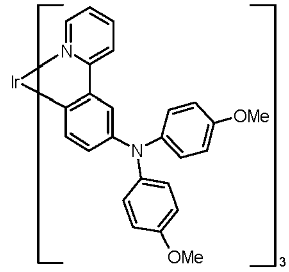

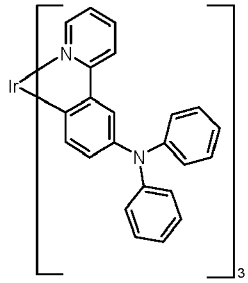
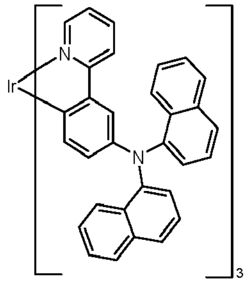
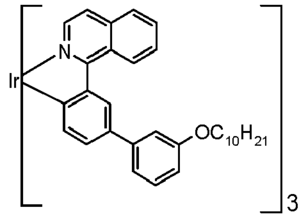

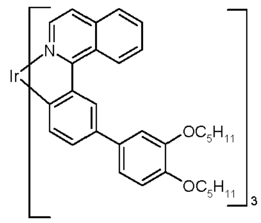
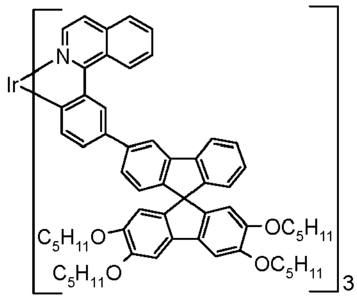
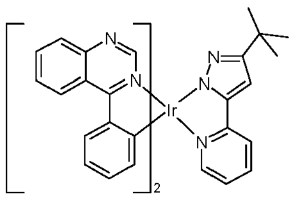

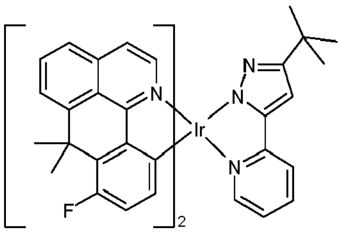
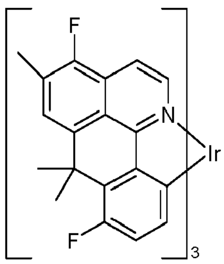
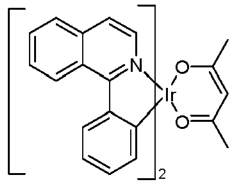

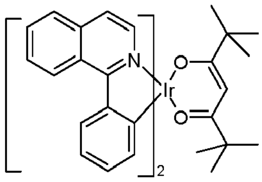
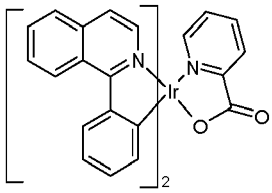
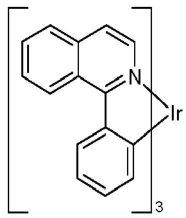

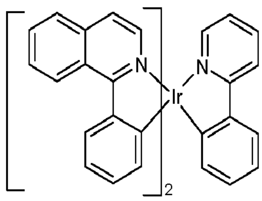
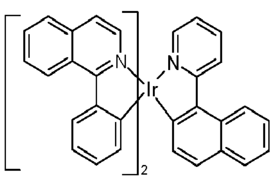
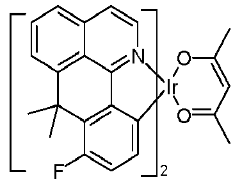

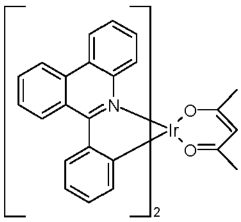
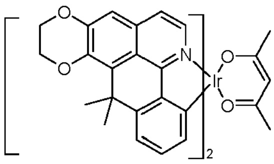
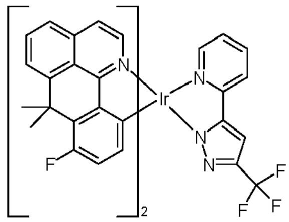

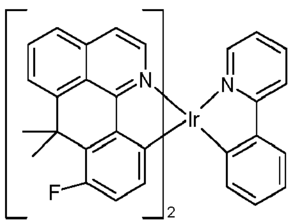
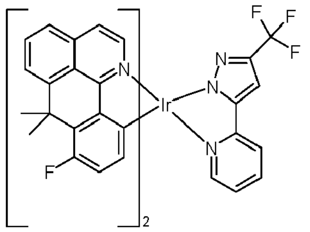
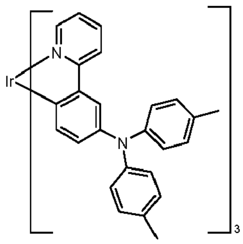

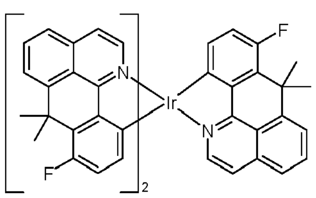
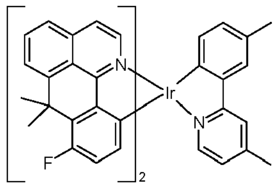
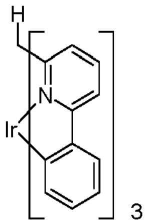

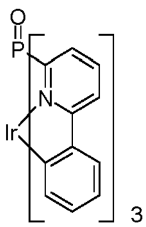
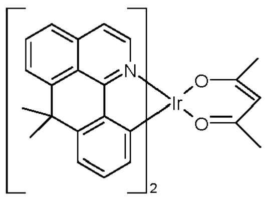
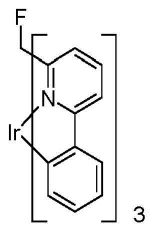

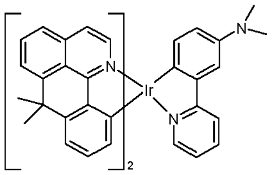
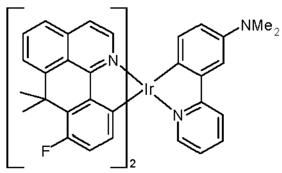
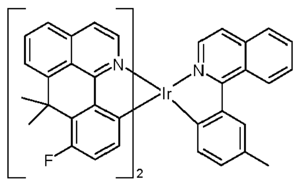

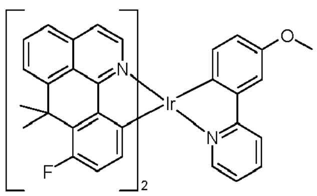
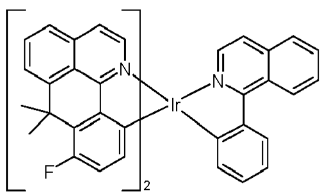
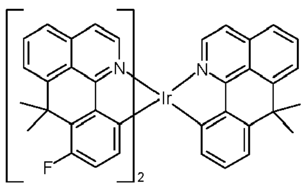

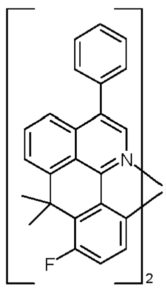
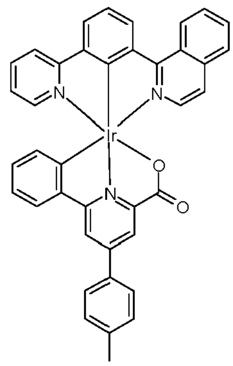
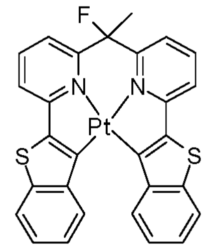
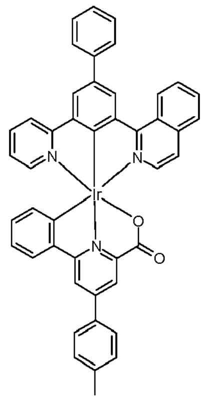
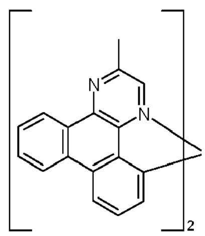
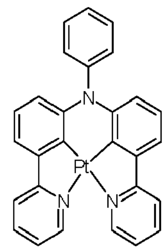
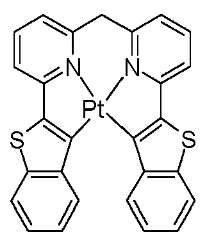
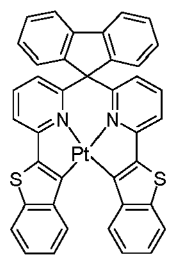
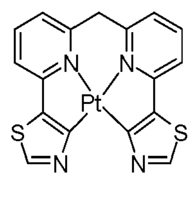
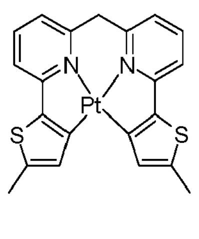
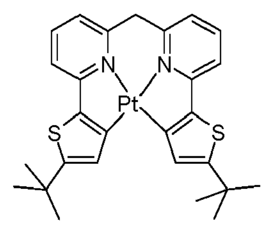
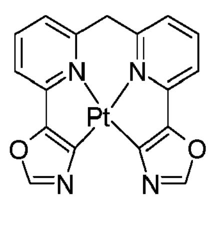
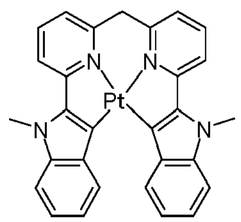
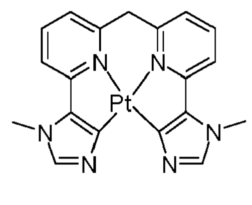
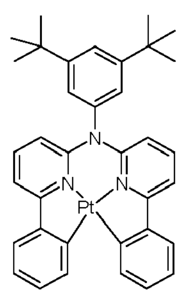

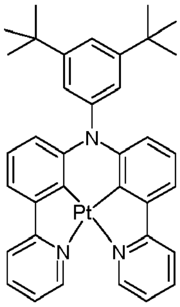
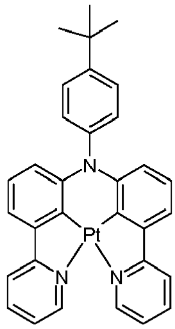
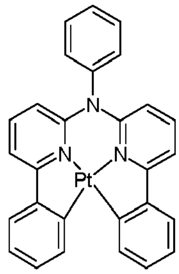
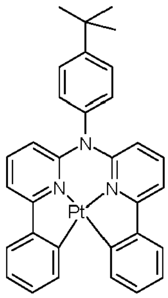
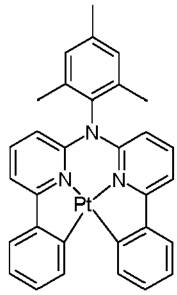
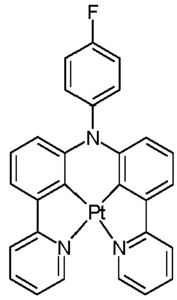
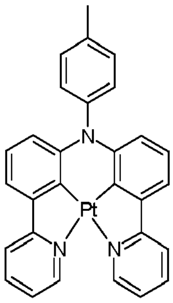
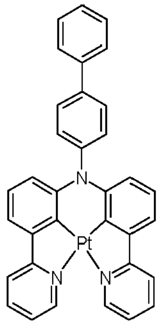
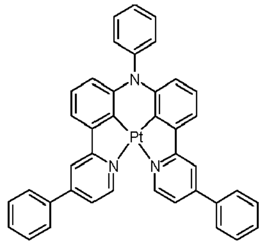
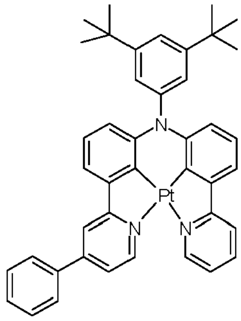
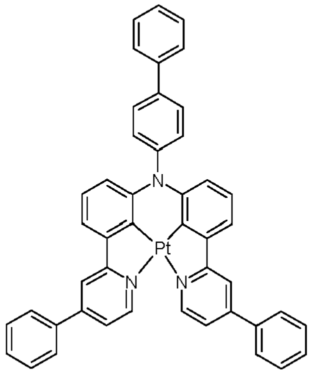
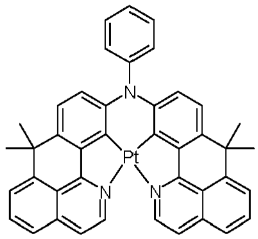

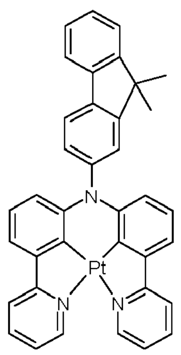
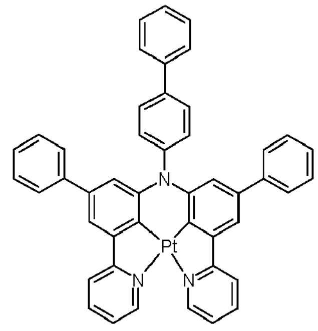
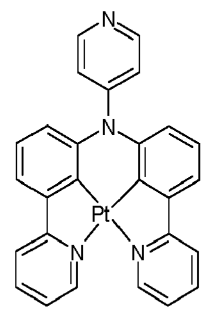

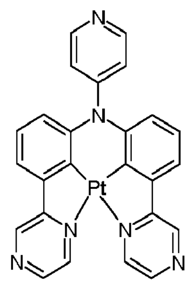
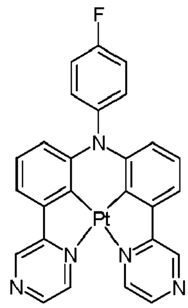
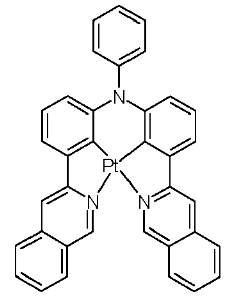

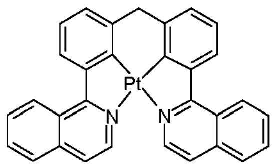
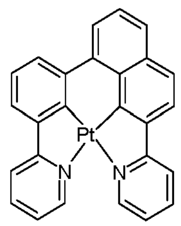
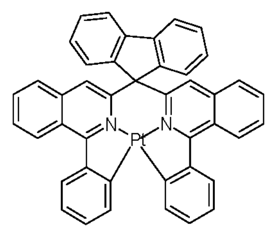

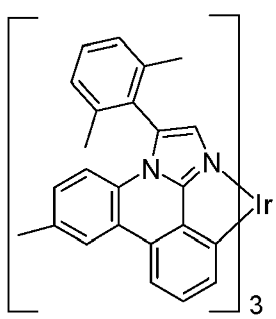
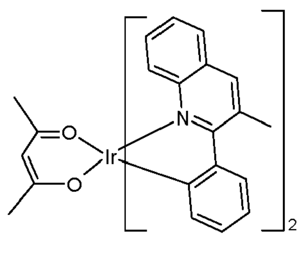
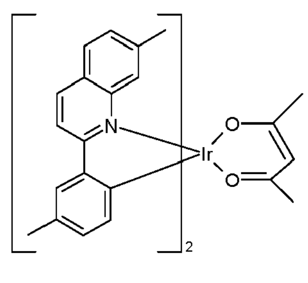

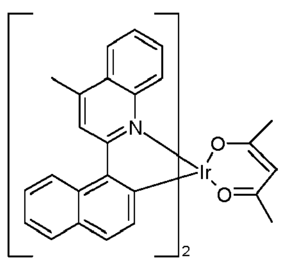
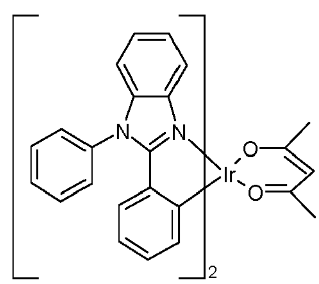
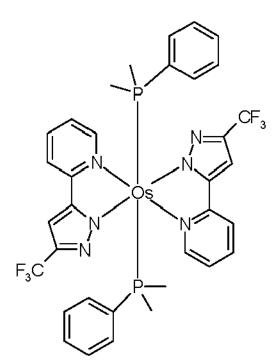

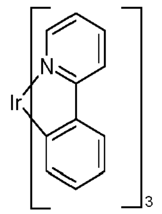

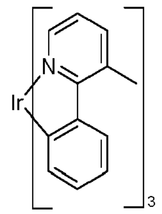

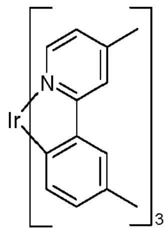

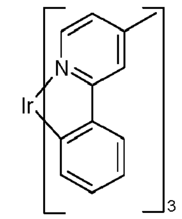

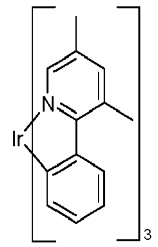

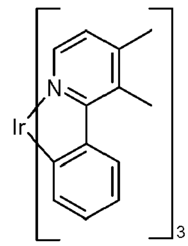

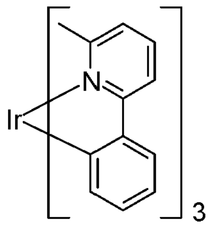

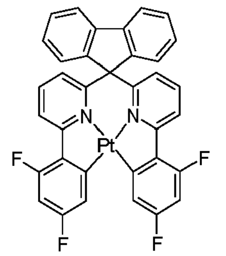

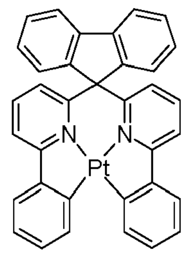

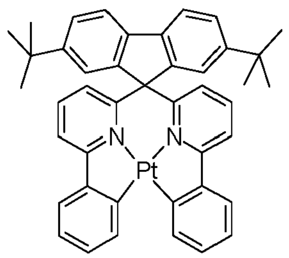

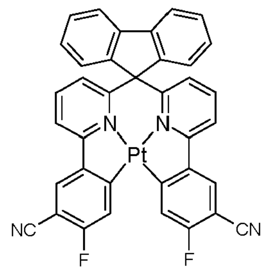

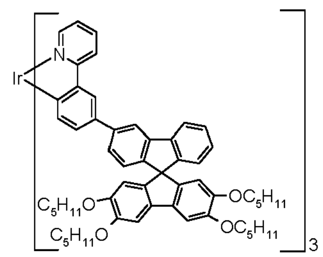

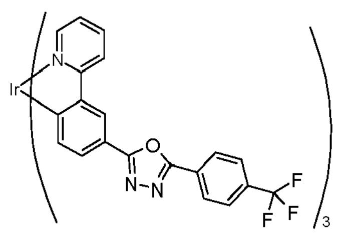

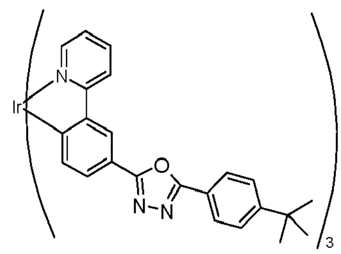

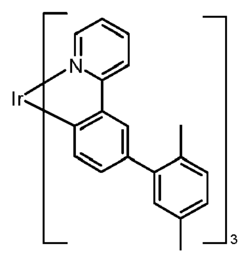

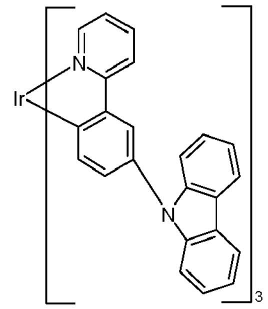
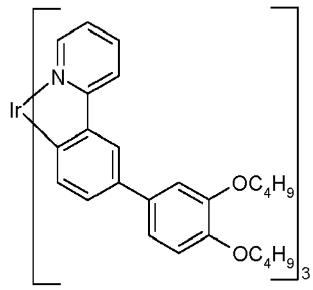
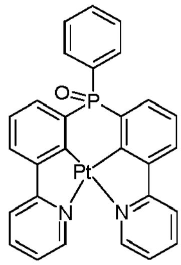

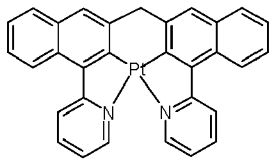
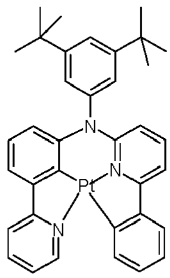
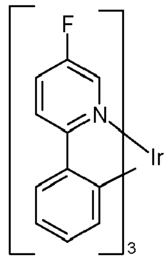

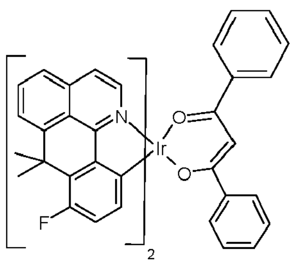
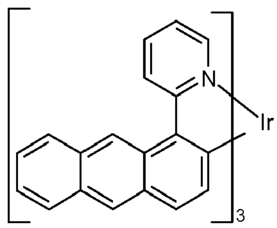
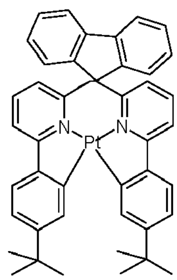

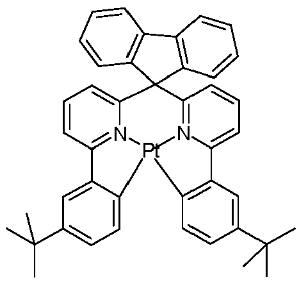
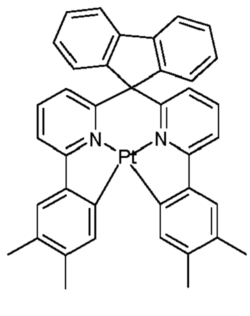
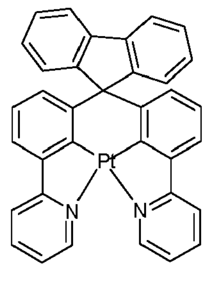

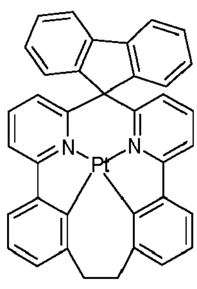
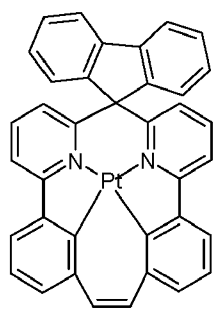
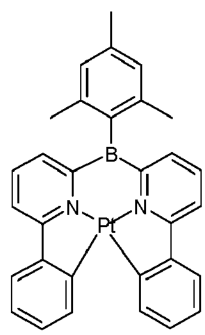

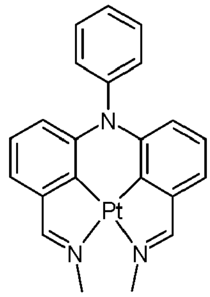
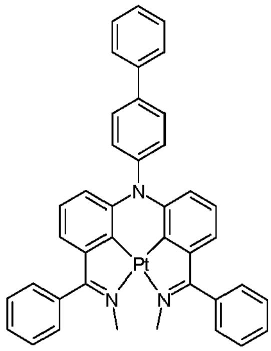
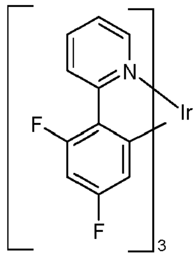

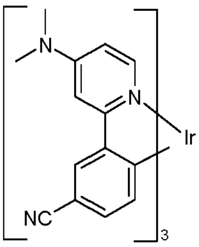
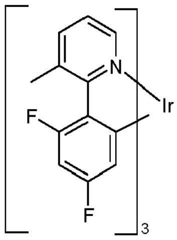
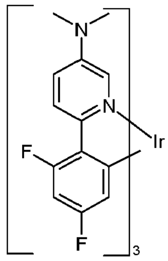

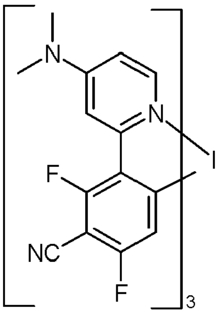
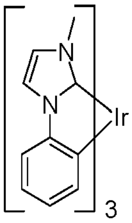
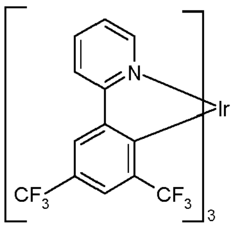

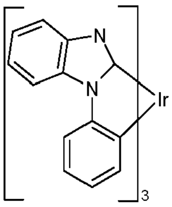
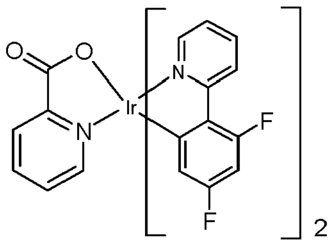
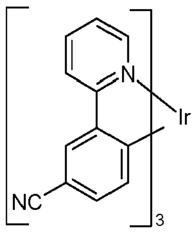

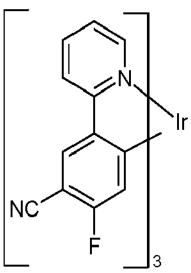
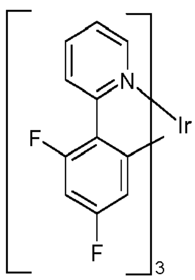
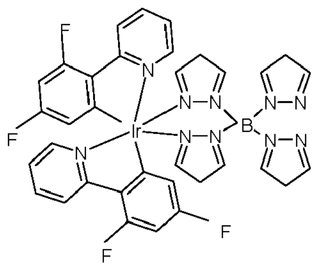

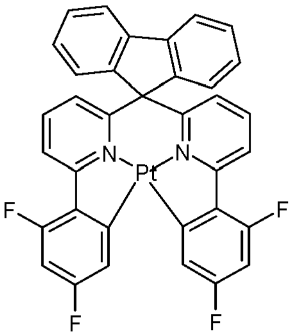

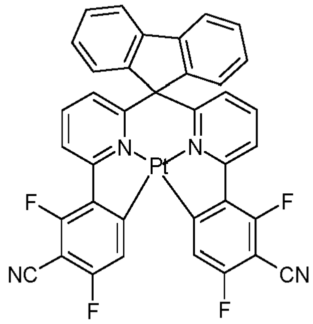

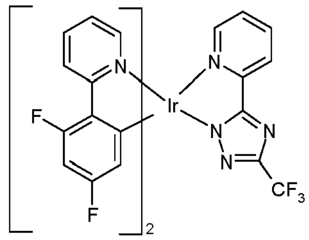

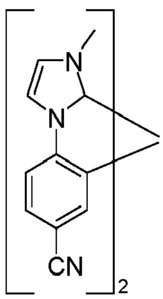

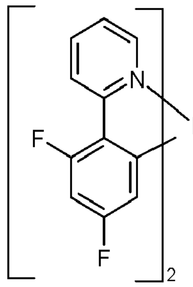

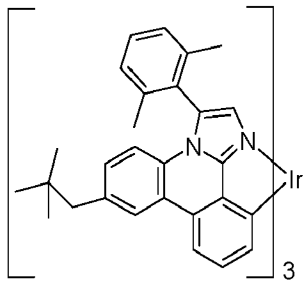

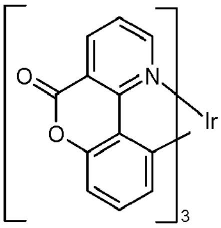

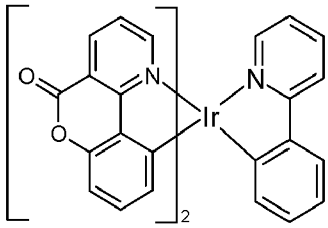

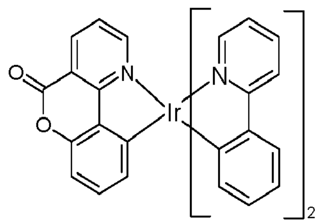

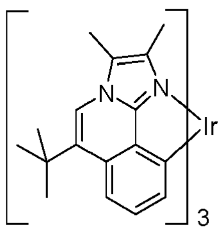

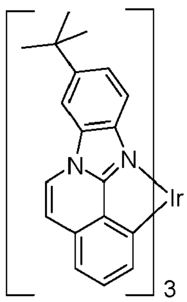

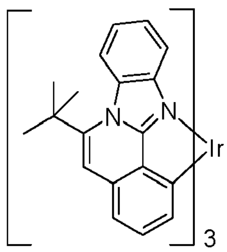

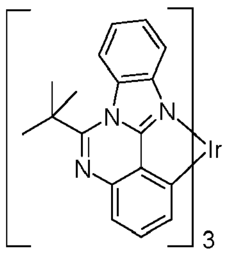

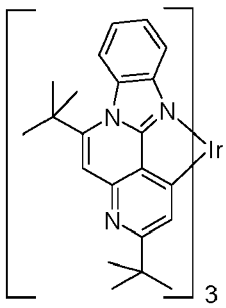

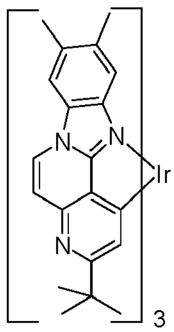

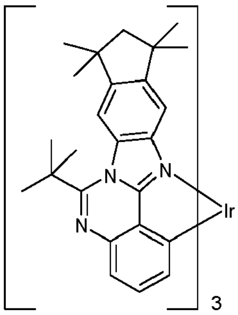
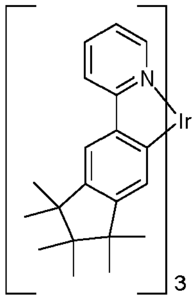
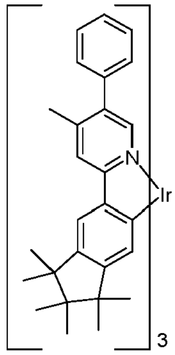
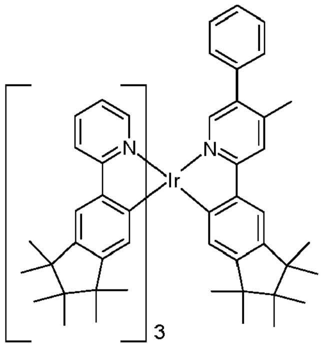
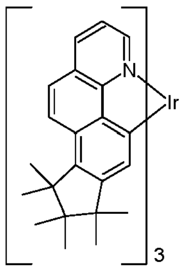
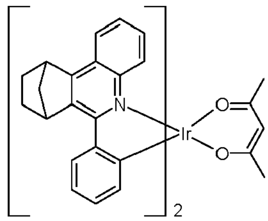
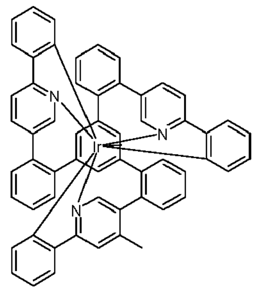
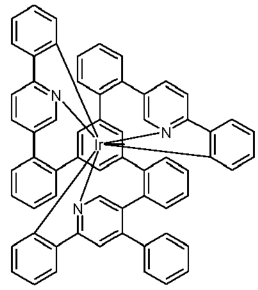
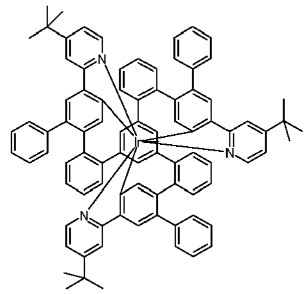
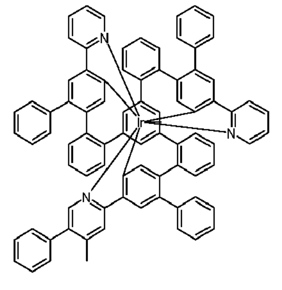
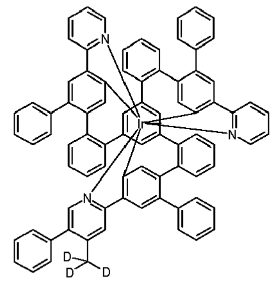
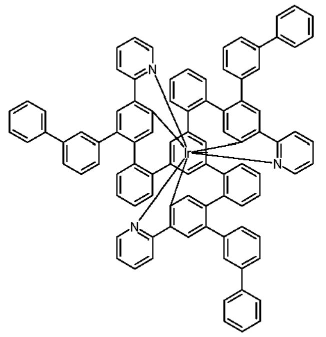

Die oben beschriebenen Verbindung, umfassend Strukturen der Formel (I) bzw. die oben aufgeführten bevorzugten Ausführungsformen, können in einer elektronischen Vorrichtung bevorzugt als aktive Komponente verwendet werden. Unter einer elektronischen Vorrichtung wird eine Vorrichtung verstanden, welche Anode, Kathode und mindestens eine zwischen Anode und Kathode liegende Schicht enthält, wobei diese Schicht mindestens eine organische bzw. metallorganische Verbindung enthält. Die erfindungsgemäße elektronische Vorrichtung enthält also Anode, Kathode und mindestens eine dazwischen liegende Schicht, welche mindestens eine Verbindung, umfassend Strukturen der Formel (I), enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), vorzugsweise organische lichtemittierende elektrochemische Zellen (OLECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs) oder organische lichtemittierende elektrochemische Zellen (OLECs), insbesondere phosphoreszierenden OLEDs oder organischen lichtemittierende elektrochemische Zellen OLECs,enthaltend in mindestens einer Schicht mindestens eine Verbindung, umfassend Strukturen der Formel (I). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie MoOs oder WOs oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die efindungsgemäße Verbindung umfassend Strukturen gemäß Formel (I) bzw. die oben aufgeführten bevorzugten Ausführungsformen als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist. Eine emittierende Schicht umfasst mindestens eine emittierende Verbindung.

Geeignete Matrixmaterialien, welche in Kombination mit den Verbindungen gemäß Formel (I) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, insbesondere Monoamine, z. B. gemäß WO 2014/015935, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinckomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivate, z. B. gemäß WO 2012/048781, Lactame, z. B. gemäß WO 2011/116865, WO 2011 /137951 oder WO 2013/064206, oder 4-Spirocarbazol-Derivate, z. B. gemäß WO 2014/094963 oder der noch nicht offen gelegten Anmeldung EP 14002104.9. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame und Carbazolderivate.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der TriplettEmitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung umfassend Strukuren gemäß Formel (I) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend Verbindung umfassend Strukuren gemäß Formel (I) bzw. die zuvor und nachfolgend ausgeführten bevorzugten Ausführungsformen in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindung umfassend Strukuren gemäß Formel (I) bzw. die zuvor und nachfolgend ausgeführten bevorzugten Ausführungsformen als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung oder eine organische elektrochemische Zelle Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren emittierenden Schichten umfasst, als Matrixmaterial.

Weiterhin ist eine elektronische Vorrichtung, vorzugsweise eine organische Elektrolumineszenzvorrichtung oder eine organische elektrochemische Zelle Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren emittierenden Schichten umfasst, als Emittermaterial.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B.

Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise MoOs oder WOs, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine efindungsgemäße Verbindung umfassend Strukturen gemäß Formel (I) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronischen Vorrichtungen, insbesondere organische Elektrolumineszenzvorrichtungen enthaltend efindungsgemäße Verbindungen umfassend Strukturen gemäß Formel (I) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronischen Vorrichtungen, insbesondere organische Elektrolumineszenzvorrichtungen und ganz besonders organische lichtemitierende Dioden oder organische lichtemittierende elektrochemische Zellen enthaltend Verbindungen, Oligomere, Polymere oder Dendrimere mit Strukturen gemäß Formel (I) bzw. die zuvor und nachfolgend ausgeführten bevorzugten Ausführungsformen, insbesondere als elektronenleitende Materialien, weisen eine sehr

gute Lebensdauer auf.

2. Elektronischen Vorrichtungen, insbesondere organische Elektrolumineszenzvorrichtungen und ganz besonders organische lichtemitierende Dioden oder organische lichtemittierende elektrochemische Zellen enthaltend Verbindungen, Oligomere, Polymere oder Dendrimere mit Strukturen gemäß Formel (I) bzw. die zuvor und nachfolgend ausgeführten bevorzugten Ausführungsformen als elektronenleitende Materialien, Elektroneninjektionsmaterialien und/oder Hostmaterialien weisen eine hervorragende Effizienz auf. Insbesondere ist die Effizienz deutlich höher gegenüber analogen Verbindungen, die keine Struktureinheit gemäß Formel (I) enthalten. Hierbei bewirken erfindungsgemäßen Verbindungen, Oligomere, Polymere oder Dendrimere mit Strukturen gemäß Formel (I) bzw. die zuvor und nachfolgend ausgeführten bevorzugten Ausführungsformen eine geringe Betriebsspannung bei Verwendung in elektronischen Vorrichtungen. Hierbei bewirken diese Verbindungen insbesondere einen geringen Roll-off, d.h. einen geringen Abfall der Leistungseffizienz der Vorrichtung bei hohen Leuchtdichten.

3. Die erfindungsgemäßen Verbindungen, Oligomere, Polymere oder Dendrimere mit Strukturen gemäß Formel (I) bzw. die zuvor und nachfolgend ausgeführten bevorzugten Ausführungsformen zeigen eine sehr hohe Stabilität (bspw. Redoxstabilität, thermische Stabilität oder Photostabilität) und führen zu Verbindungen mit einer sehr hohen Lebensdauer.

4. Mit Verbindungen, Oligomere, Polymere oder Dendrimere mit Strukturen gemäß Formel (I) bzw. die zuvor und nachfolgend ausgeführten bevorzugten Ausführungsformen kann in elektronischen Vorrichtungen, insbesondere organische Elektrolumineszenzvorrichtungen und ganz besonders organische lichtemitierende Dioden oder organische lichtemittierende elektrochemische Zellen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

5. Die Verwendung von Verbindungen, Oligomere, Polymere oder Dendrimere mit Strukturen gemäß Formel (I) bzw. die zuvor und nachfolgend ausgeführten bevorzugten Ausführungsformen in Schichten elektronischer Vorrichtungen, insbesondere organischer Elektrolumineszenzvorrichtungen und ganz besonders organische lichtemitierende Dioden oder organischer lichtemittierender elektrochemischen Zellen führt zu einer hohen Mobilität der Elektron-Leiterstrukturen.

6. Verbindungen, Oligomere, Polymere oder Dendrimere mit Strukturen gemäß Formel (I) bzw. die zuvor und nachfolgend ausgeführten bevozugten Ausführungsformen zeichnen sich durch eine ausgezeichnete thermische Stabilität aus, wobei Verbindungen mit einer Molmasse von weniger als ca. 1200 g/mol gut sublimierbar sind.

7. Verbindungen, Oligomere, Polymere oder Dendrimere mit Strukturen gemäß Formel (I) bzw. die zuvor und nachfolgend ausgeführten bevorzugten Ausführungsformen weisen eine ausgezeichnete Glasfilmbildung auf.

8. Verbindungen, Oligomere, Polymere oder Dendrimere mit Strukturen gemäß Formel (I) bzw. die zuvor und nachfolgend ausgeführten bevorzugten Ausführungsformen bilden aus Lösungen sehr gute Filme.

9. Die Verbindungen, Oligomere, Polymere oder Dendrimere umfassend Strukturen gemäß Formel (I) bzw. die zuvor und nachfolgend ausgeführten bevorzugten Ausführungsformen weisen ein überraschend hohes Triplett-Niveau $T_1$ auf, wobei dies insbesondere Verbindungen gilt, die als elektronenleitende Materialien eingesetzt werden.

Diese oben genannten Vorteile gehen nicht mit einer Verschlechterung der weiteren elektronischen Eigenschaften einher.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Ein nochmals weiterer Gegenstand der vorliegenden Erfindung ist die Verwendung einer erfindungsgemäßen Verbindung und/oder eines erfindungsgemäßen Oligomers, Polymers oder Dendrimers in einer elektronischen Vorrichtung als TADF-Material, Hostmaterial, Lochleitmaterial, Elektroneninjektionsmaterial und/oder Elektronentransportmaterial, vorzugsweise als TADF-Material, Hostmaterial, Lochleitmaterial und/oder Elektronentransportmaterial.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen. Besonders bevorzugt ist elektronische Vorrichtung ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), vorzugsweise organische lichtemittierende elektrochemische Zellen (OLECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs) oder organische lichtemittierende elektrochemische Zellen (OLECs), insbesondere phosphoreszierende oder fluoreszierende OLEDs oder fluoreszierende OLECs.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Elektronentransportschicht einzusetzen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (I) bzw. gemäß den bevorzugten Ausführungsformen einsetzen.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

Der Fachmann kann aus den Schilderungen ohne erfinderisches Zutun weitere erfindungsgemäße elektronische Vorrichtungen herstellen und somit die Erfindung im gesamten beanspruchten Bereich ausführen.

**Beispiele:**

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern

beziehen sich auf die CAS-Nummern der literaturbekannten Verbindungen.

**Synthese der Synthone**

**Beispiel S1:**

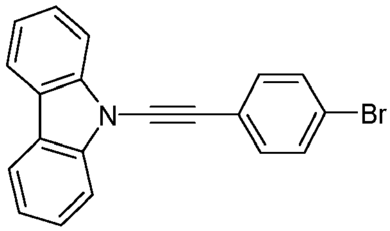

Durchführung analog A. Sagadevan, et al., Green Chemistry, 17(2), 1113-1119; 2015. Ein gut gerührtes Gemisch aus 16.7 g (100 mmol) Carbazol [86-74-8], 34.6 g (250 mmol) Kaliumcarbonat, 2.5 g (10 mmol) Kupfer(II)sulfat-Pentahydrat [7758-99-8], 3.6 g (20 mmol) Phenanthrolin, 300 ml Toluol und 50 g Glaskugeln wird mit 33.8 g (130 mmol) 1-Brom-4-(2-bromethynyl)benzene [934-94-1] versetzt und dann 16 h bei 80 °C gerührt. Nach Erkalten filtriert man über ein Celitet-Bett ab, wäscht dieses nach und engt das Filtrat zur Trockene ein. Der Rückstand wird Flash-chromatographisch (CombiFlash Torrent der Fa. Axel Semrau) gereinigt. Ausbeute: 23.2 g (67 mmol), 67 %; Reinheit: 95 % n. $^1$H-NMR.

**Beispiel S100:**

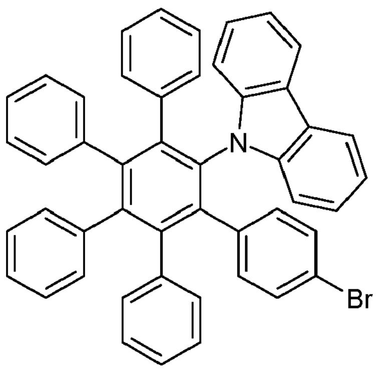

Eine Gemisch aus 34.6 g (100 mmol) S1, 38.5 g (100 mmol) 2,3,4.5-Tetraphenyl-2,4-cyclopentadien-1-on [479-33-4] und 130 ml Diphenylether wird 24 h auf 265 °C erhitzt. Nach Erkalten und Entfernen des Diphenylethers im Vakuum kocht man den Rückstand mit 300 ml Ethanol aus, saugt vom Feststoff ab, wäscht dreimal mit je 100 ml EtOH nach und trocknet im Vakuum. Der Rückstand wird Flash-chromatographisch (CombiFlash Torrent der Fa. Axel Semrau) gereinigt. Ausbeute: 51.4 g (73 mmol), 73 %; Reinheit: 98 % n. $^1$H-NMR.

Analog können folgende Verbindungen unter Einsatz von S1 bzw. 1-Brom-4-[2-(4-chlorphenyl)ethynyl]-benzol [832744-28-2] als Dienophiel darestellt werden:

| Bsp. | Dien | Produkt | Ausbeute |
|---|---|---|---|
| S101 | 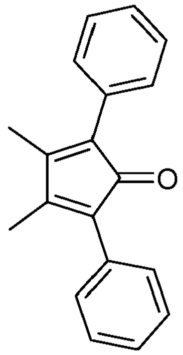 S1 | 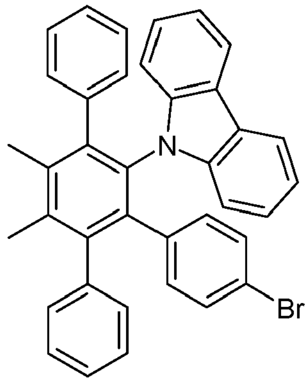 | 54 % |
| S102 | 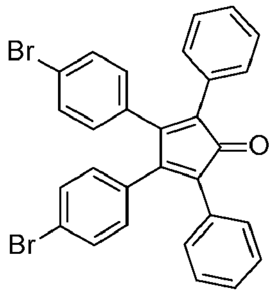 38268-11-0 S1 | 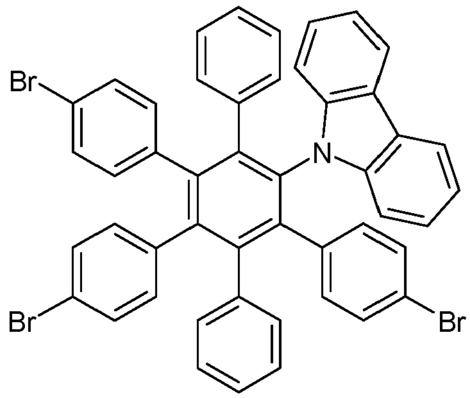 | 56 % |
| S103 | 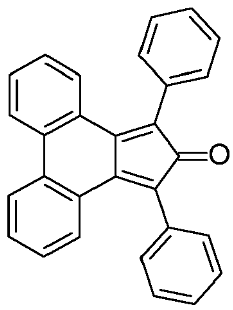 5660-91-3 S1 | 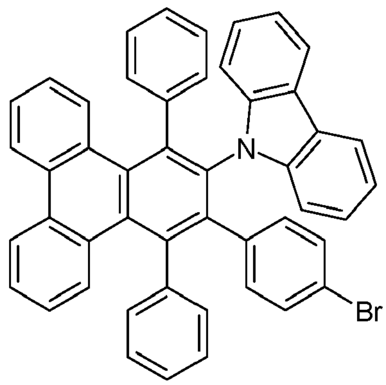 | 52 % |
| S104 | 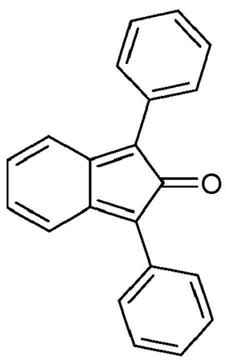 23414-46-2 | 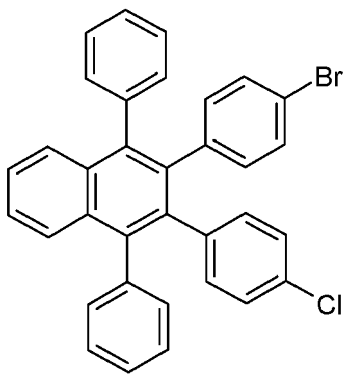 | 63 % |

(fortgesetzt)

| Bsp. | Dien | Produkt | Ausbeute |
|---|---|---|---|
| S105 | 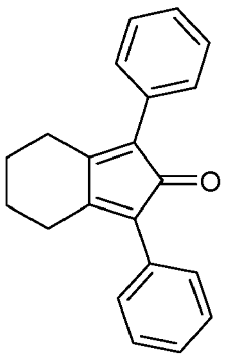 412268-65-6 | 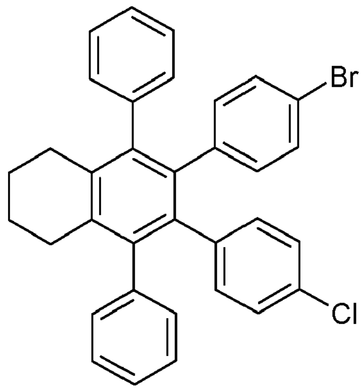 | 43 % |
| S106 | 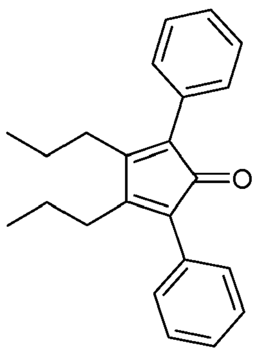 1607433-47-5 | 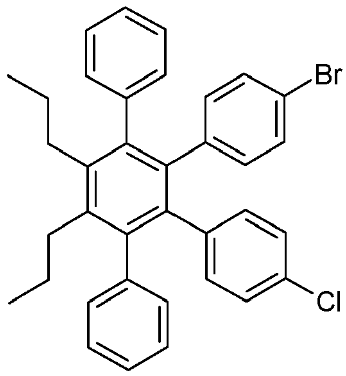 | 55 % |
| S107 | 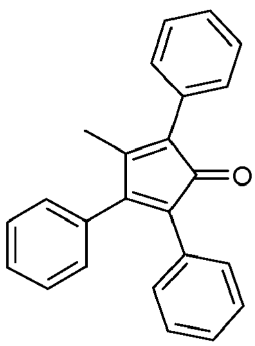 107319-64-2 | 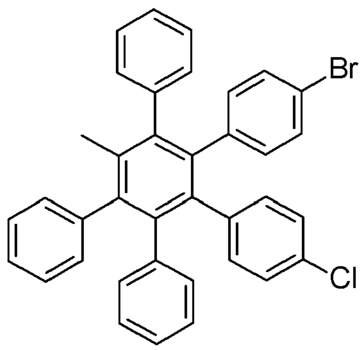 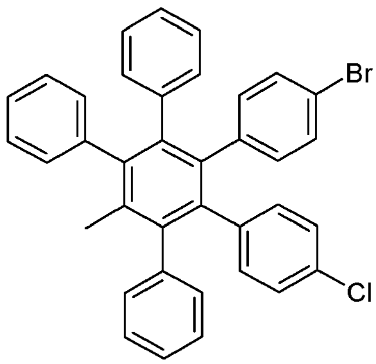 Isomerengemisch | 53 % |

(fortgesetzt)

| Bsp. | Dien | Produkt | Ausbeute |
|---|---|---|---|
| S108 | 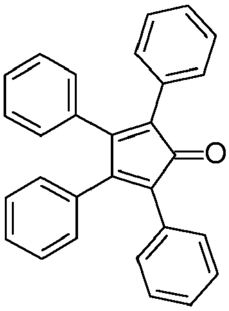 479-33-4 | 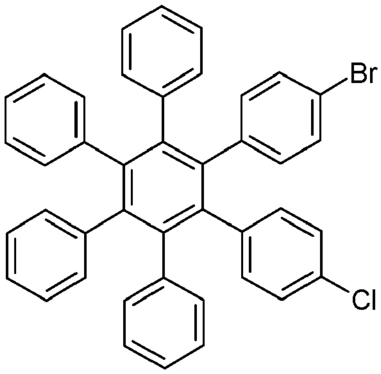 | 67 % |
| S109 | 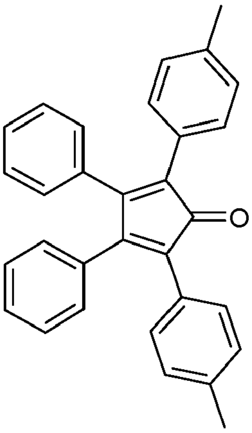 38268-18-7 | 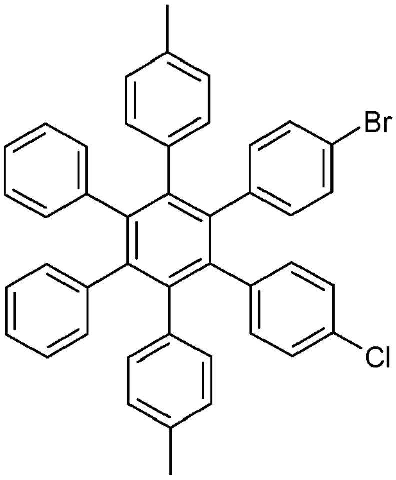 | 60 % |
| S110 | 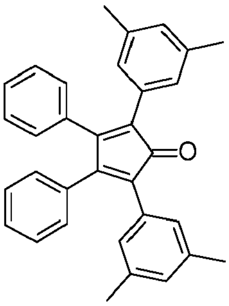 356074-64-1 | 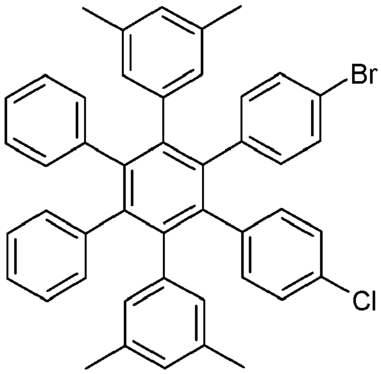 | 63 % |
| S111 | 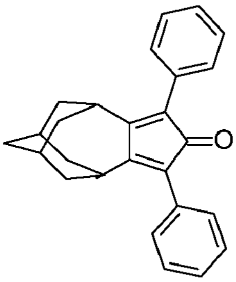 106821-16-3 | 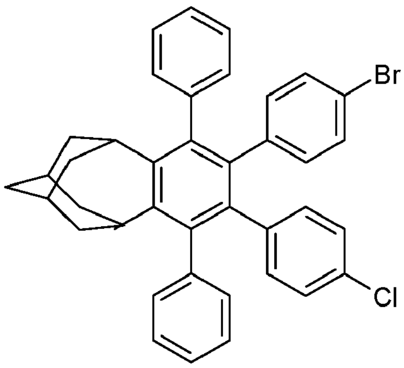 | 68 % |

(fortgesetzt)

| Bsp. | Dien | Produkt | Ausbeute |
|---|---|---|---|
| S112 | 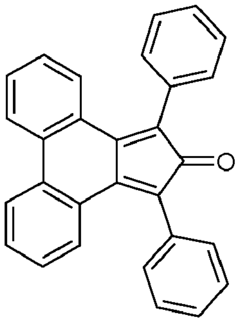 5660-91-3 S1 | 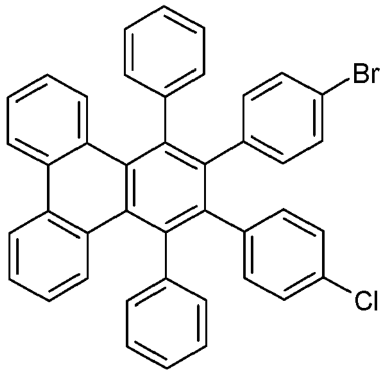 | 73 % |
| S113 | 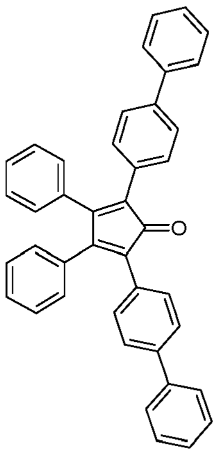 19059-92-8 | 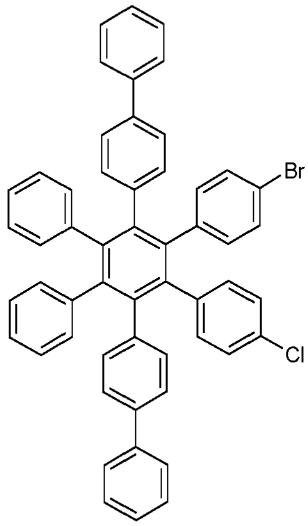 | 69 % |
| S114 | 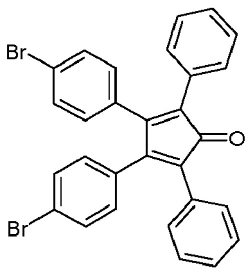 38268-11-0 | 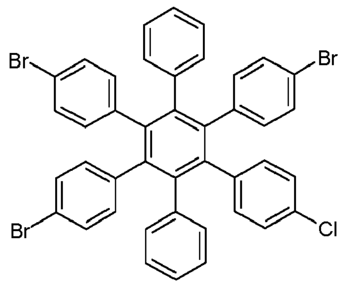 | 57 % |

(fortgesetzt)

| Bsp. | Dien | Produkt | Ausbeute |
|---|---|---|---|
| S115 | 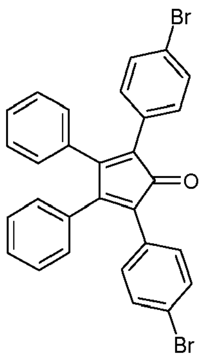 54523-24-9 | 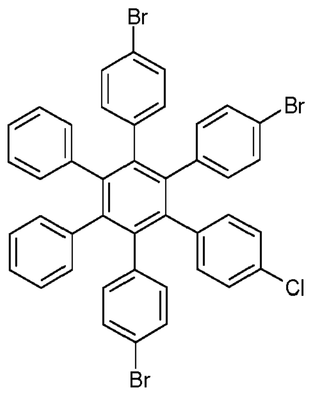 | 53 % |
| S116 | 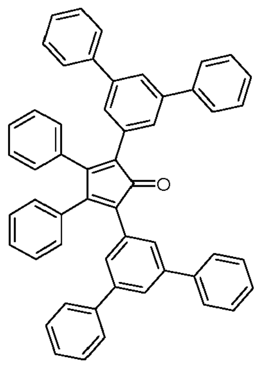 297154-31-2 | 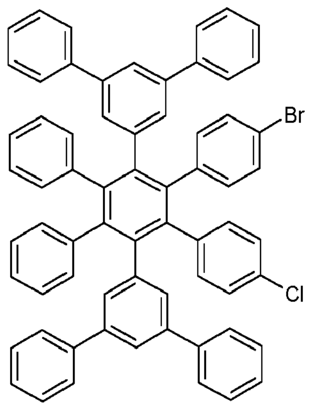 | 62 % |
| S117 | 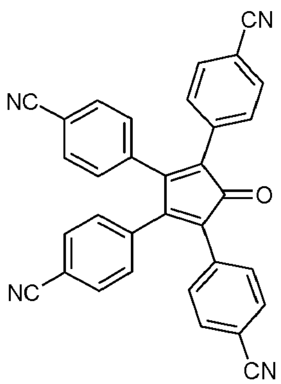 1465020-42-1 | 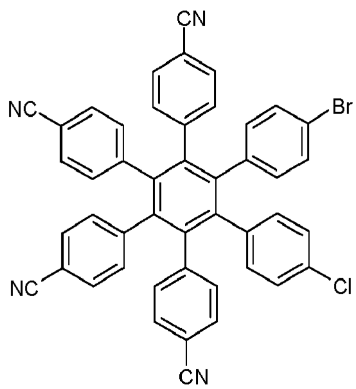 | 43 % |

(fortgesetzt)

| Bsp. | Dien | Produkt | Ausbeute |
|---|---|---|---|
| S118 | 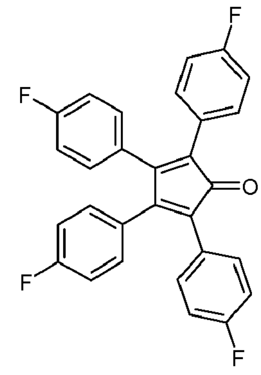 163132-55-6 | 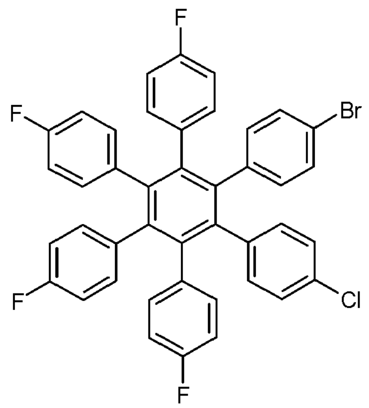 | 66 % |
| S119 | 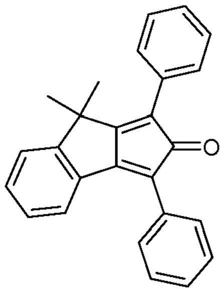 132665-86-2 | 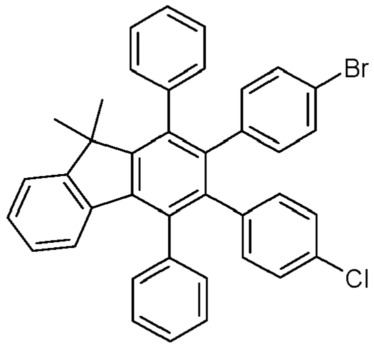 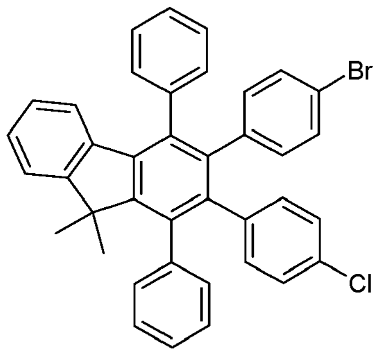 Isomerengemisch | 60 % |
| S120 | 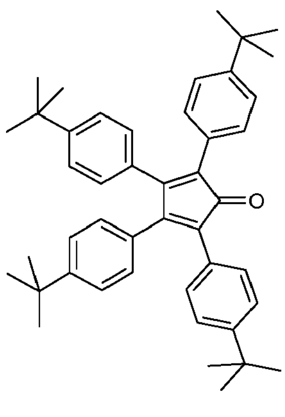 196505-83-6 | 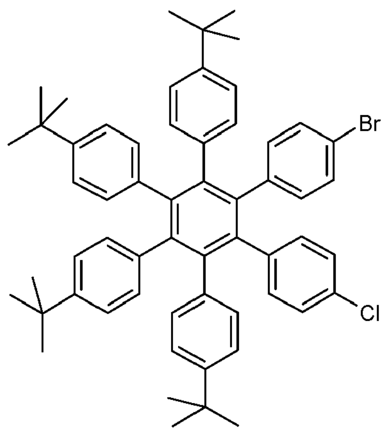 | 67 % |

(fortgesetzt)

| Bsp. | Dien | Produkt | Ausbeute |
|---|---|---|---|
| S121 | 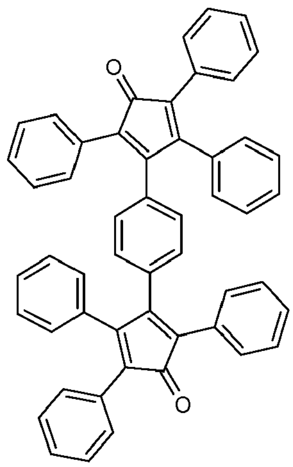 3432-73-3 | 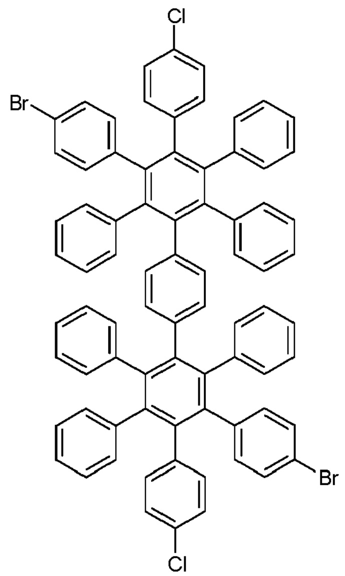 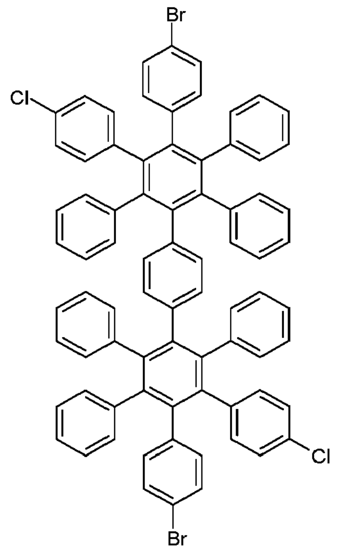 | 61 % |

(fortgesetzt)

| Bsp. | Dien | Produkt | Ausbeute |
|---|---|---|---|
| | | 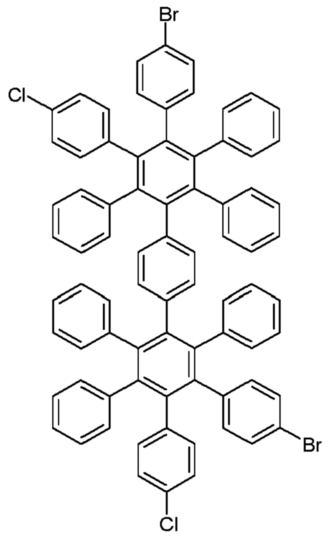 Isomerengemisch | |
| S122 | 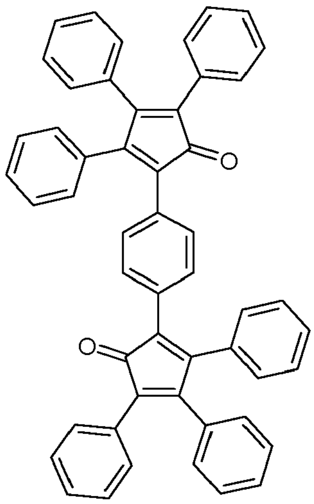 1018691-33-2 | 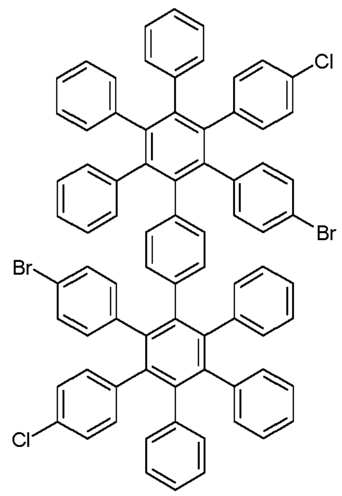 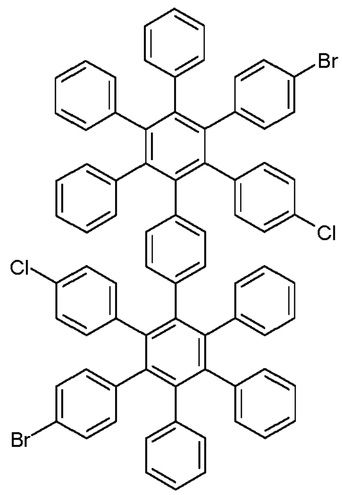 | 63 % |

(fortgesetzt)

| Bsp. | Dien | Produkt | Ausbeute |
|---|---|---|---|
| | | 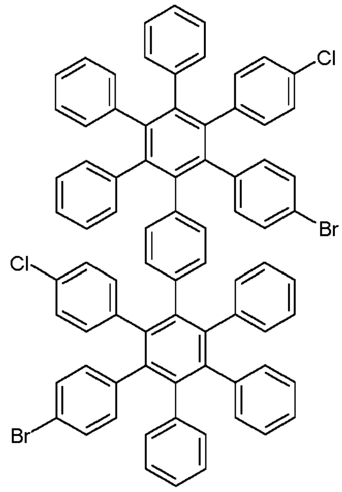 Isomerengemisch | |
| S123 | 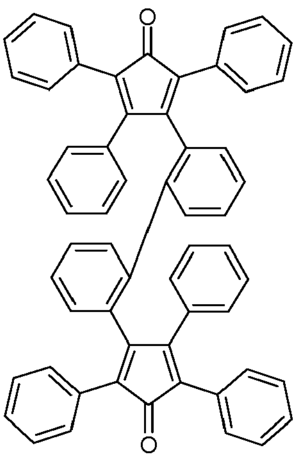 30550-32-04 | 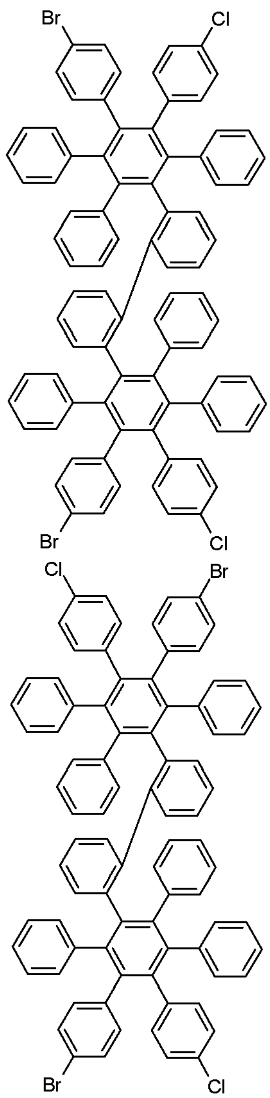 | 48 % |

(fortgesetzt)

| Bsp. | Dien | Produkt | Ausbeute |
|---|---|---|---|
| | | 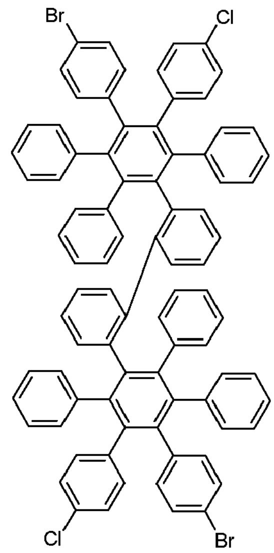 Isomerengemisch | |

**Beispiel S200: Buchwald-Kupplung**

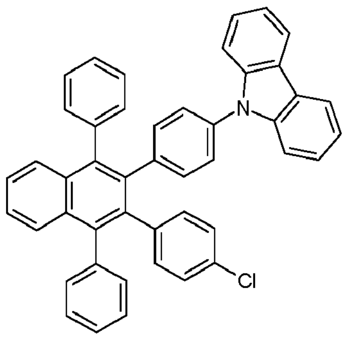

Ein Gemisch aus 54.6 g (100 mmol) S104, 16.7 g (100 mmol) Carbazol [86-74-8], 41.5 g (300 mmol) Kaliumcarbonat, 50 g Glaskugeln, 700 ml Toluol, 405 mg (2 mmol) Tri-tert-butylphosphin und 225 mg (1 mmol) Palladiumacetat wird 24 h unter gutem Rühren unter Rückfluss erhitzt. Nach Erkalten saugt man von den Salzen über ein mit Toluol vorgeschlämmtes Celite-Bett ab, wäscht dreimal mit je 100 ml warmem Toluol nach, wäscht das Filtrat einmal mit 500 ml Wasser, einmal mit 300 ml Kochsalzlösung und trocknet über Magnesiumsulfat. Der nach Abfltrieren des Trockenmittels und Entfernen des Lösungsmittels erhaltene Feststoff wird chromatographiert (Kieselgel, Dichlormethan) und dann aus Dimethylacetamid (DMAC) umkristallisiert. Ausbeute:46.1 g (73 mmol), 73 %; Reinheit: 97 % n. $^1$H-NMR.

Analog können folgende Verbindungen dargestellt werden, wobei für Kupplungen mit sec. Aminen anstelle von Kaliumcarbonat 120 mmol Natrium-tert-butanolal verwendet wird

| Bsp. | Edukte | Produkt | Ausbeute |
|---|---|---|---|
| S201 | S104<br>122-39-4 | 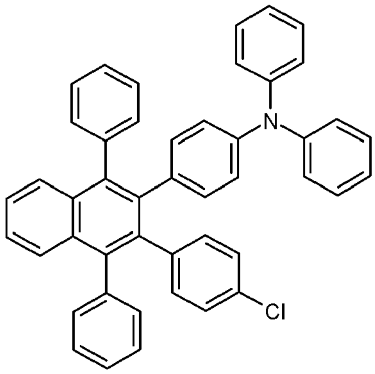 | 58 % |
| S202 | S104<br>1421789-16-3 | 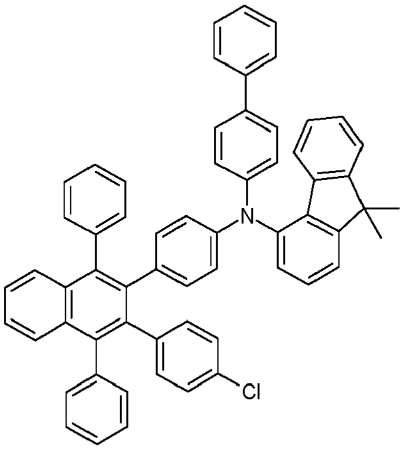 | 70 % |
| S203 | S104<br>205-25-4 | 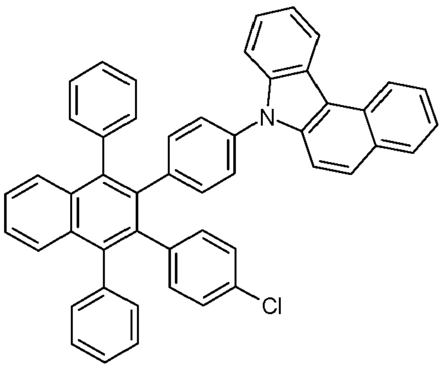 | 68 % |
| S204 | S105<br>56525-79-2 | 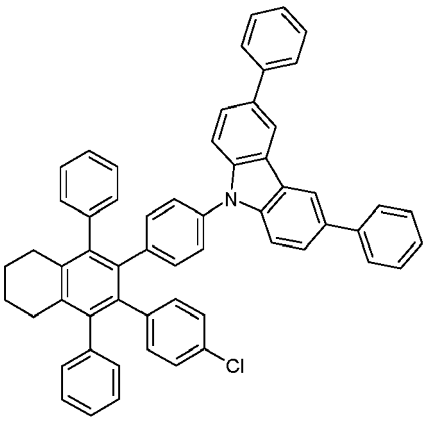 | 67 % |

(fortgesetzt)

| Bsp. | Edukte | Produkt | Ausbeute |
|---|---|---|---|
| S205 | S106 1257220-47-5 | 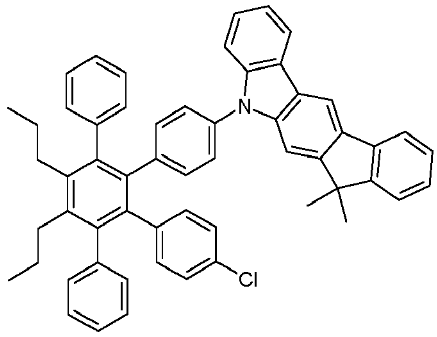 | 72 % |
| S206 | S107 1466521-76-5 | 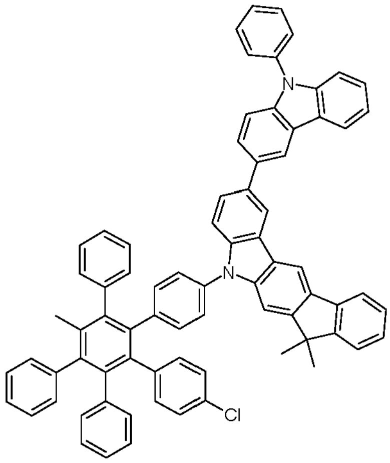 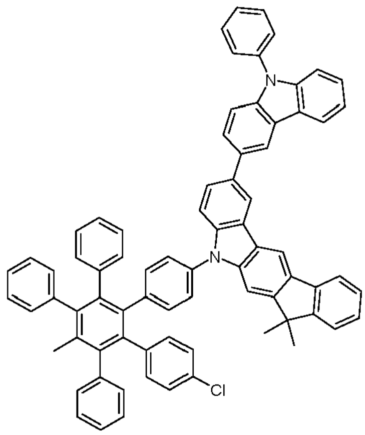 Isomerengemisch | 49 % |
| S207 | S108 1257247-94-1 | 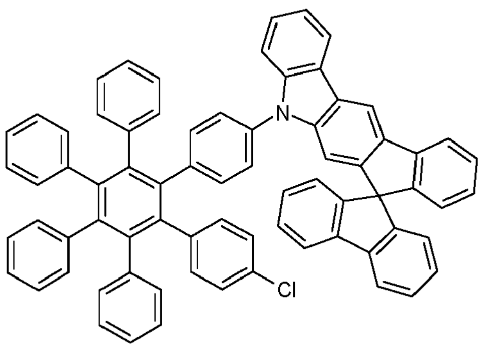 | 59 % |

(fortgesetzt)

| Bsp. | Edukte | Produkt | Ausbeute |
|---|---|---|---|
| S208 | S108<br>1431284-23-9 | 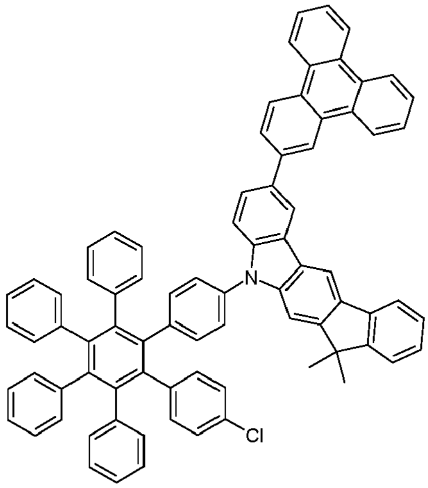 | 55 % |
| S209 | S108<br>1316311-27-9 | 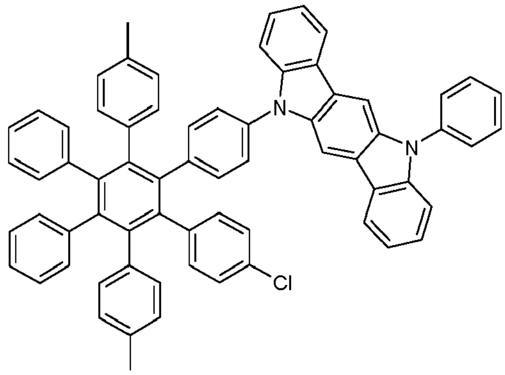 | 60 % |
| S210 | S109<br>1024598-06-8 | 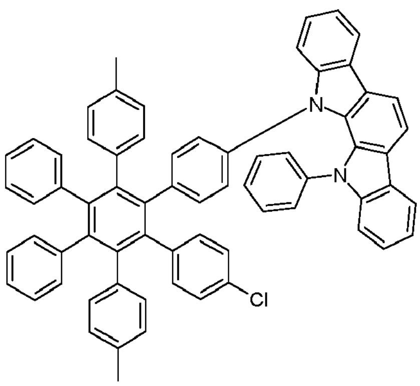 | 38 % |
| S211 | S110<br>1199350-22-5 | 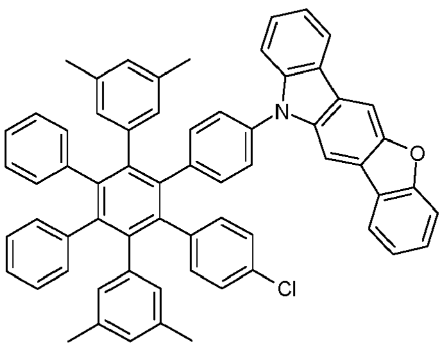 | 70 % |

(fortgesetzt)

| Bsp. | Edukte | Produkt | Ausbeute |
|---|---|---|---|
| S212 | S111<br>1382955-10-3 | 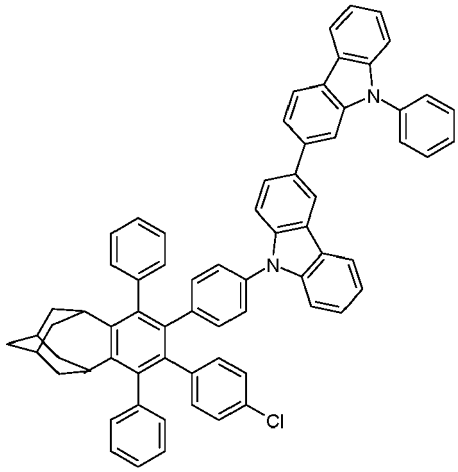 | 64 % |
| S213 | S111<br>1060735-14-9 | 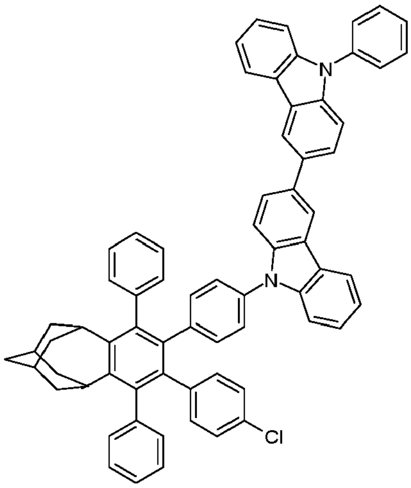 | 60 % |
| S214 | S112<br>1609088-05-2 | 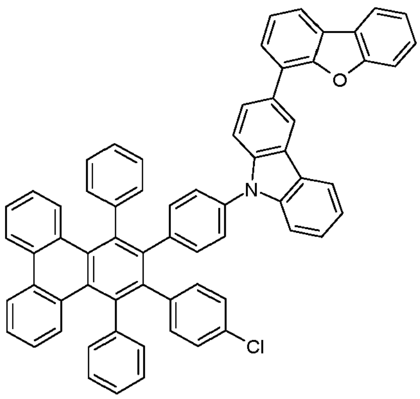 | 59 % |

(fortgesetzt)

| Bsp. | Edukte | Produkt | Ausbeute |
|---|---|---|---|
| S215 | S113 244-76-8 | 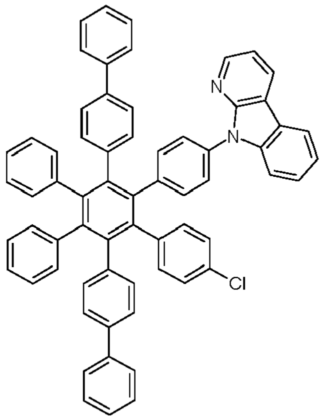 | 68 % |
| S216 | S114 88590-00-5 | 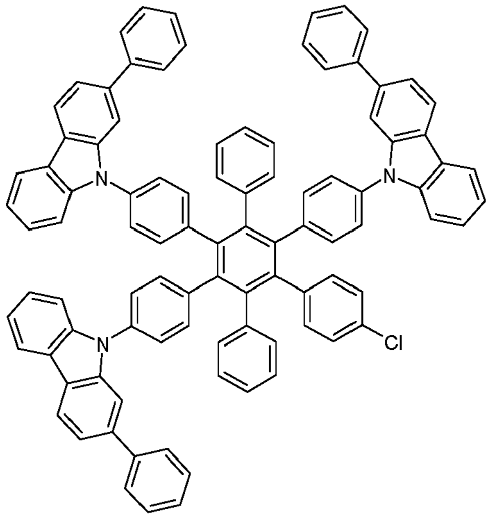 300 mmol 88590-00-5 6 mmol P-tBus / 3 mmol $Pd(ac)_2$ | 45 % |
| S217 | S115 244-63-3 | 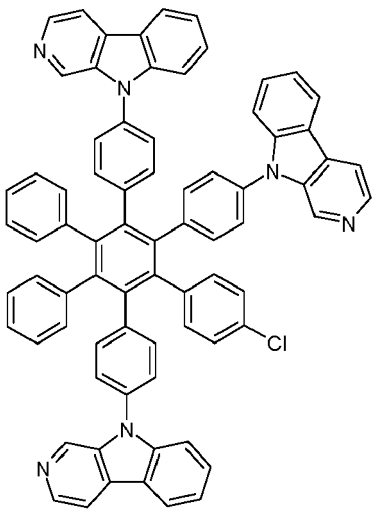 300 mmol 244-63-3 6 mmol P-tBus / 3 mmol $Pd(ac)_2$ | 48 % |

(fortgesetzt)

| Bsp. | Edukte | Produkt | Ausbeute |
|---|---|---|---|
| S218 | S116<br>1365647-82-0 | 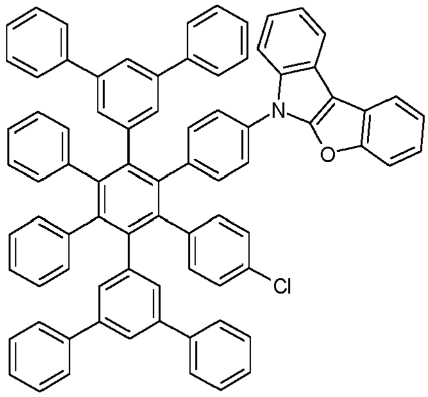 | 58 % |
| S219 | S117<br>135-67-1 | 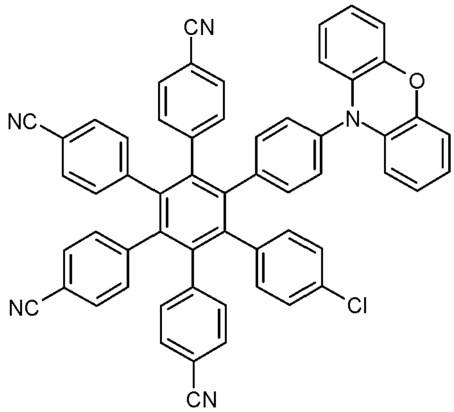 | 62 % |
| S220 | S118<br>1799501-71-5 | 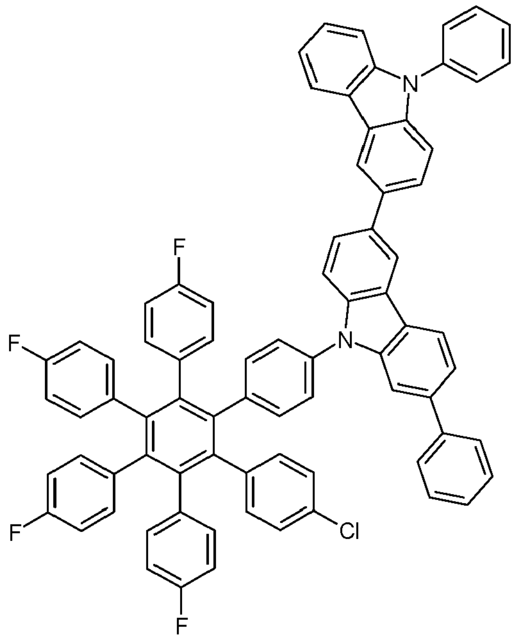 | 60 % |

(fortgesetzt)

| Bsp. | Edukte | Produkt | Ausbeute |
|---|---|---|---|
| S221 | S119<br>955959-89-4 | 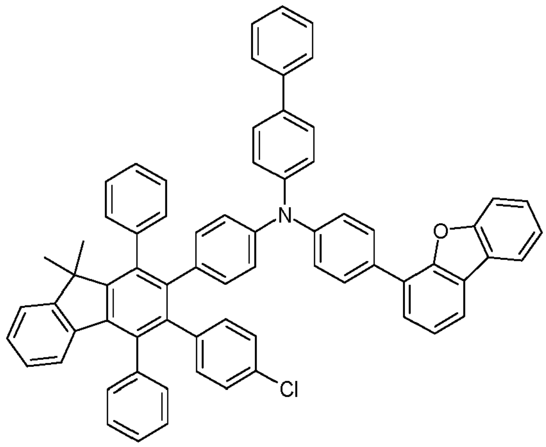<br>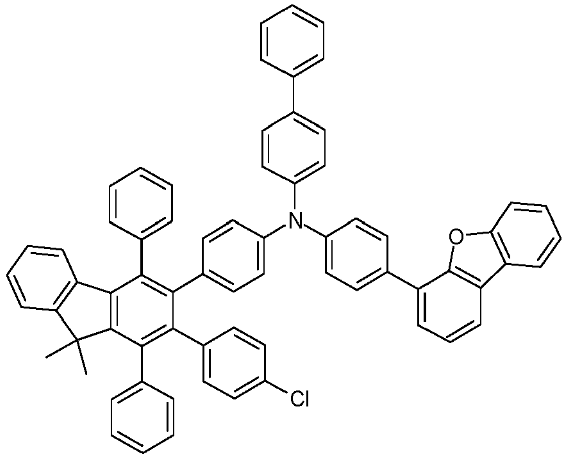<br>Isomerengemisch | 49 % |
| S222 | S120<br>1346669-46-2 | 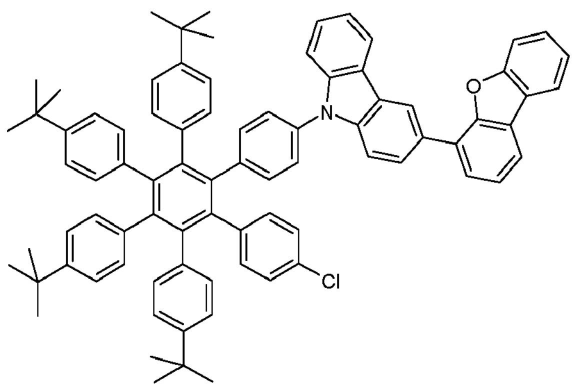 | 55 % |

(fortgesetzt)

| Bsp. | Edukte | Produkt | Ausbeute |
|---|---|---|---|
| S223 | S121<br>1364890-88-9 | 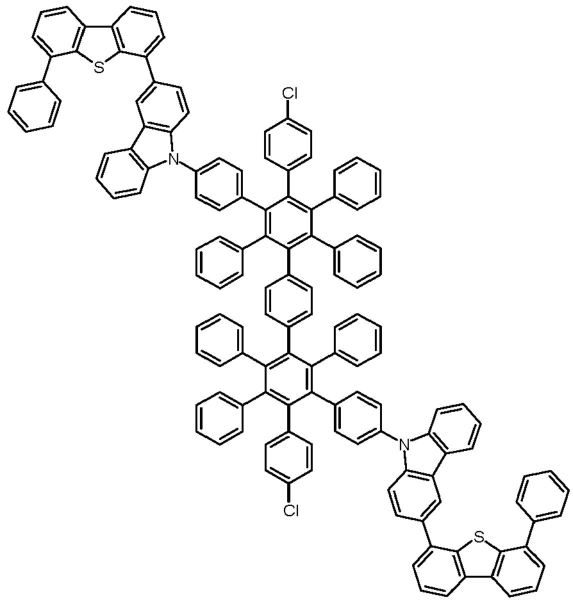<br>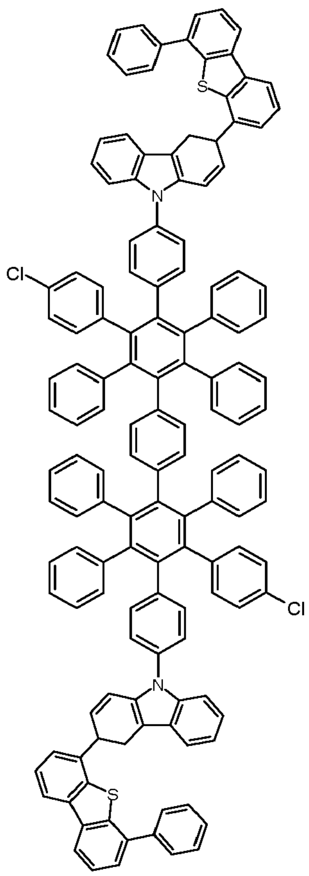 | 47 % |

(fortgesetzt)

| Bsp. | Edukte | Produkt | Ausbeute |
|---|---|---|---|
| | | 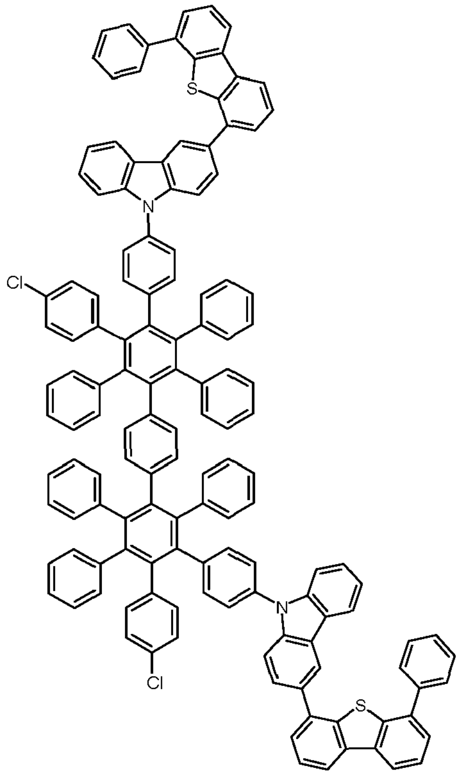 200 mmol 1364890-88-9 4 mmol P-tBus / 2 mmol Pd(ac)$_2$ Isomerengemisch | |
| S224 | S122 1807860-07-6 | 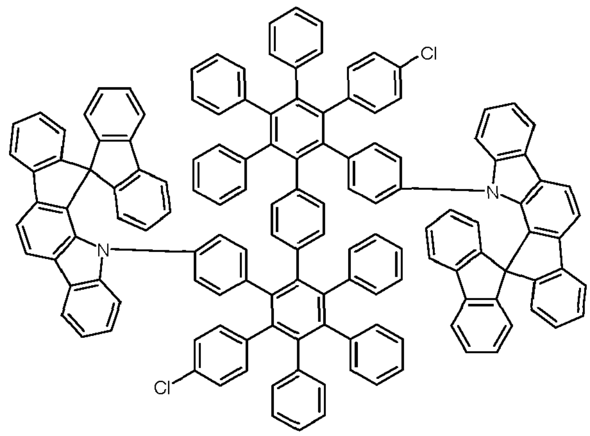 | 51 % |

(fortgesetzt)

| Bsp. | Edukte | Produkt | Ausbeute |
|---|---|---|---|
| | | 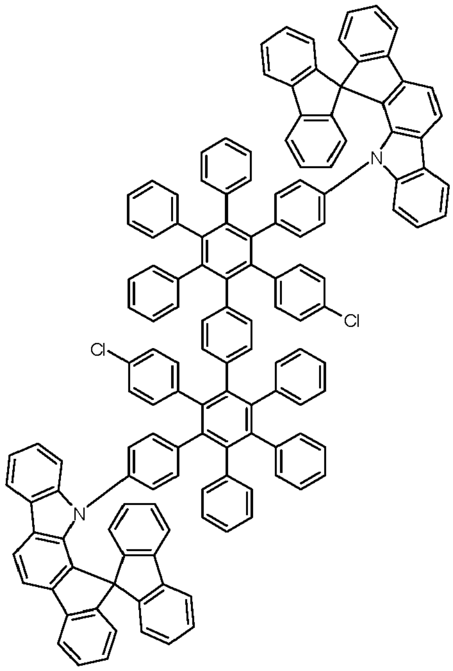 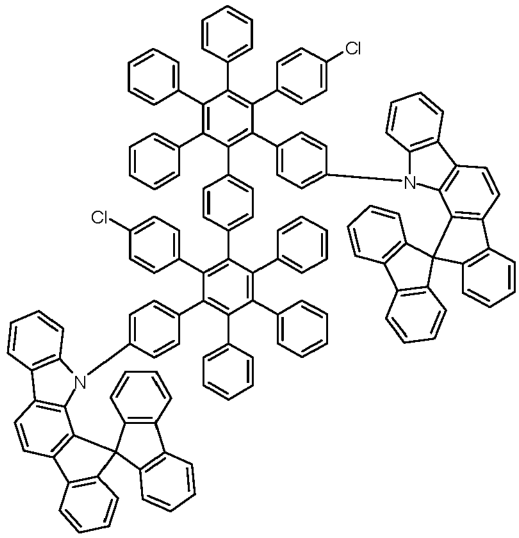 200 mmol 1807860-07-6 4 mmol P-$tBu_3$ / 2 mmol $Pd(ac)_2$ Isomerengemisch | |

(fortgesetzt)

| Bsp. | Edukte | Produkt | Ausbeute |
|---|---|---|---|
| S225 | S123<br>1372775-52-4 | 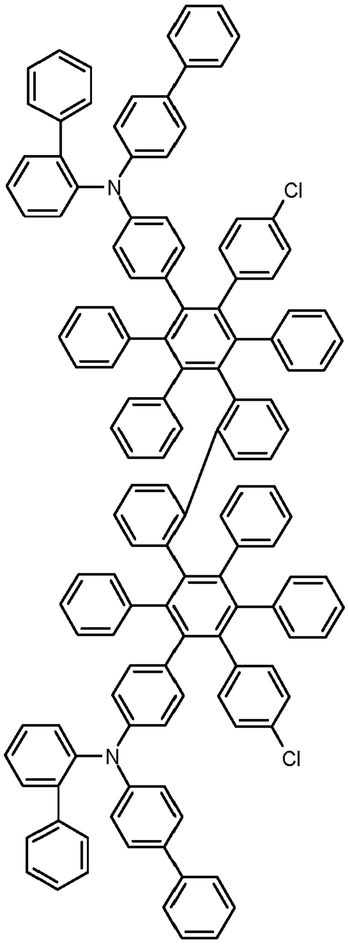<br>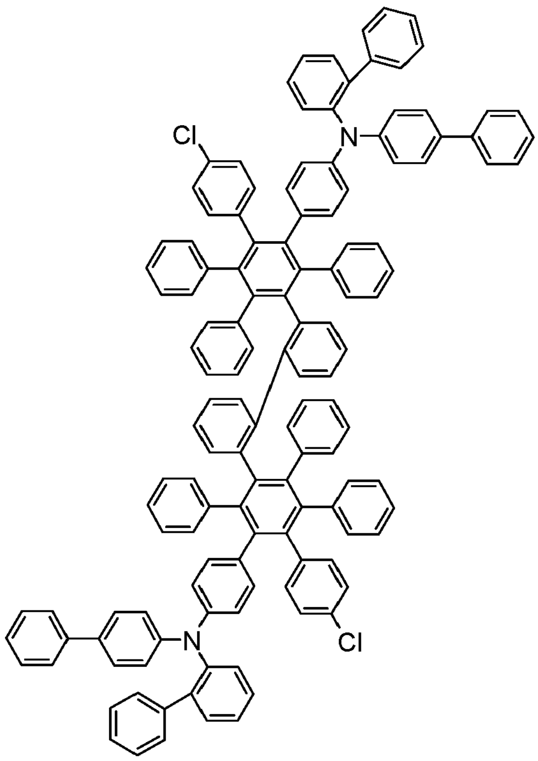 | 45 % |

(fortgesetzt)

| Bsp. | Edukte | Produkt | Ausbeute |
|---|---|---|---|
| | | 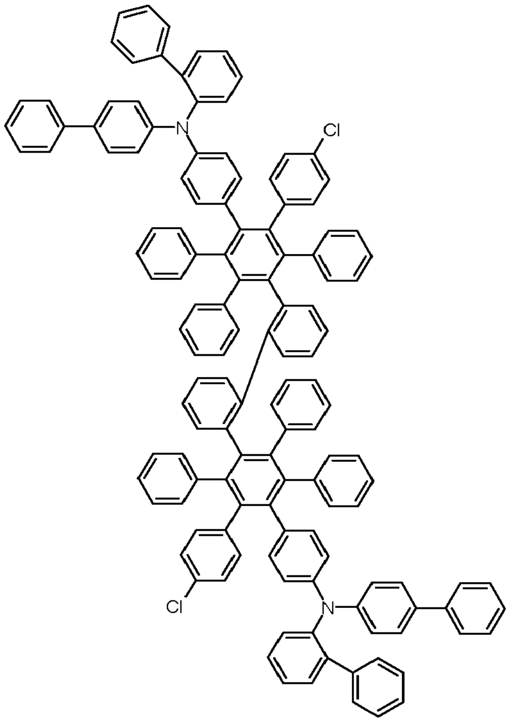 200 mmol 1372775-52-4 4 mmol P-tBu$_3$ / 2 mmol Pd(ac)$_2$ Isomerengemisch | |

**Beispiel S300 : Borylierung**

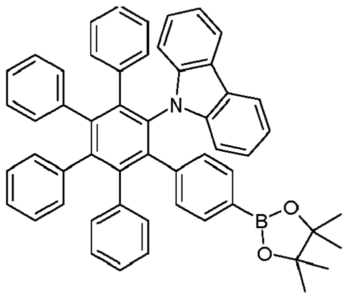

Ein Gemisch aus 70.3 g (100 mmol) S100, 26.7 g (105 mmol) 4,4,4',4',5,5,5',5'-Octamethyl-2,2'-bi-(1,3,2-dioxaborolane) [73183-34-3], 29.5 g (300 mmol) Kaliumacetat, wasserfrei, 50 g Glaskugeln (3 mm Durchmesser) und 700 ml Dioxan wird mit 543 mg (1.3 mmol) S-Phos [657408-07-6] und 225 mg (1 mmol) Palladium(II)acetat versetzt und unter gutem Rühren 16 h auf 90 °C erhitzt. Nach Erkalten filtriert man über ein mit Dioxan vorgeschlämmtes Celite-Bett ab, wäscht mit 300 ml Dioxan nach, engt das Filtrat zur Trockene ein, nimmt den Rückstand in 500 ml Toluol auf, wäscht die Lösung dreimal mit je 100 ml Wasser, einmal mit 200 ml ges. Kochsalzlösung und trocknet dann über Magnesiumsulfat. Der nach Abfltrieren des Trockenmittels und Entfernen des Lösungsmittels erhaltene Schaum wird aus Ethylacetat/Methanol umkristallisiert. Ausbeute: 65.2 g (87 mmol), 87 %; Reinheit: 95 % n. $^1$H-NMR.

Analog können folgende Verbindungen dargestellt werden:

| Bsp. | Edukt | Produkt | Ausbeute |
|---|---|---|---|
| S301 | S101 | 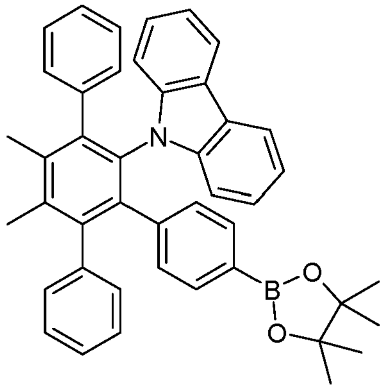 | 89 % |
| S302 | S102 | 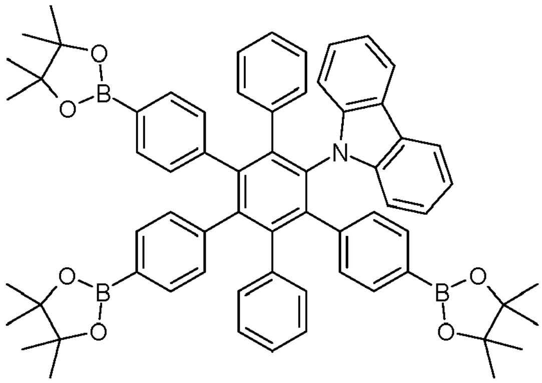 315 mmol 73183-34-3 | 90 % |
| S303 | S103 | 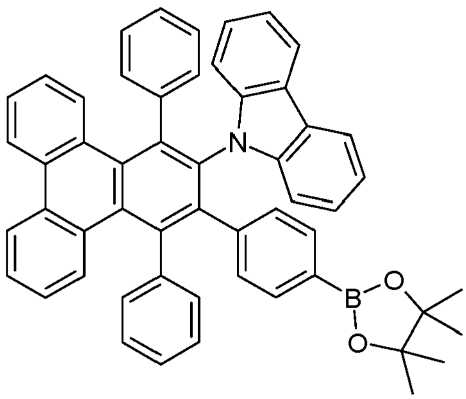 | 85 % |
| S304 | S201 | 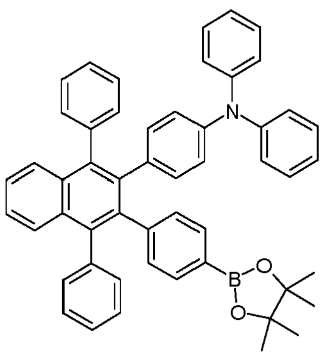 | 86 % |

(fortgesetzt)

| Bsp. | Edukt | Produkt | Ausbeute |
|---|---|---|---|
| S305 | S202 | 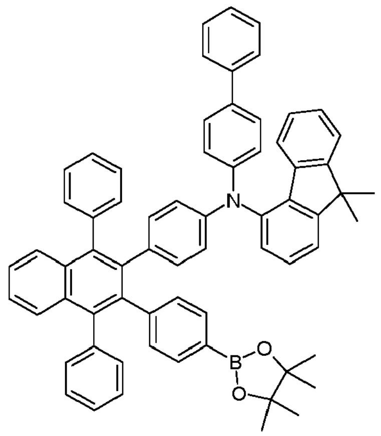 | 83 % |
| S306 | S203 | 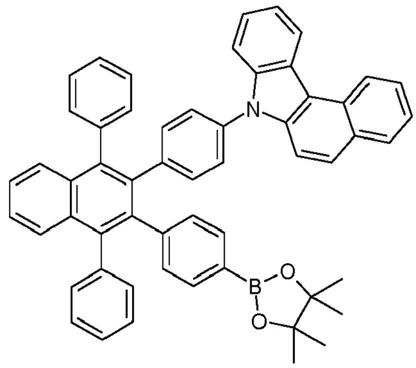 | 90 % |
| S307 | S204 | 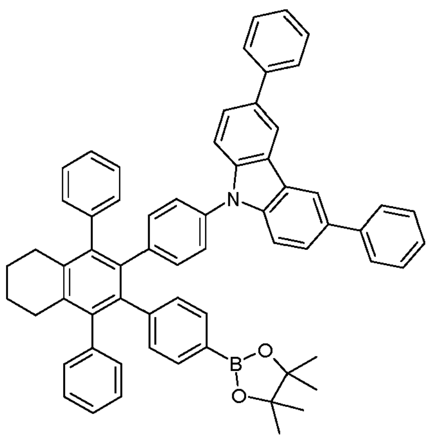 | 86 % |
| S308 | S205 | 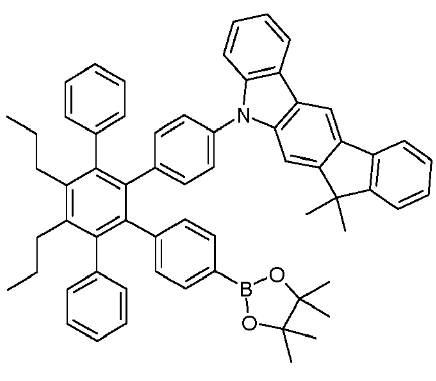 | 87 % |

(fortgesetzt)

| Bsp. | Edukt | Produkt | Ausbeute |
|---|---|---|---|
| S309 | S206 | 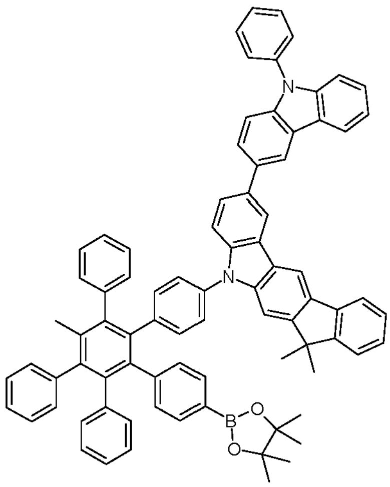 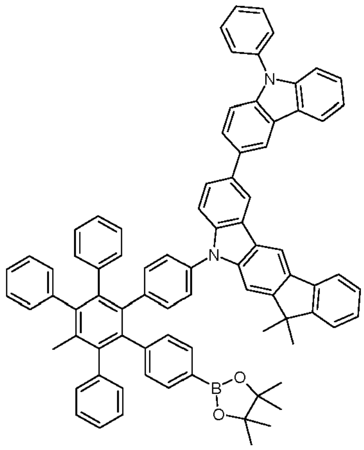 Isomerengemisch | 73 $ |
| S310 | S207 | 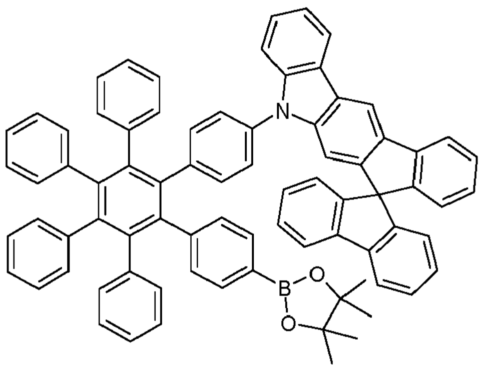 | 79 % |

(fortgesetzt)

| Bsp. | Edukt | Produkt | Ausbeute |
|---|---|---|---|
| S311 | S208 | 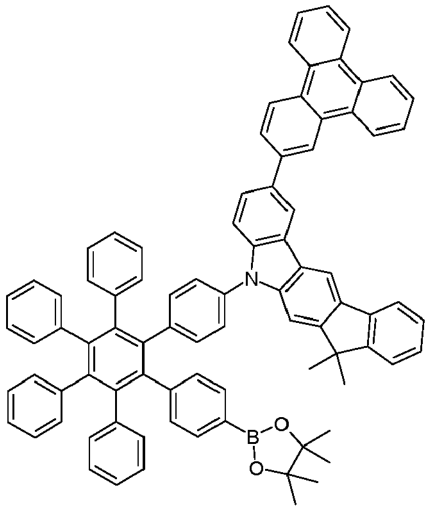 | 80 % |
| S312 | S209 | 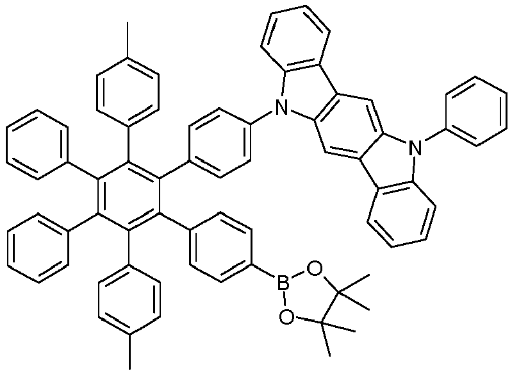 | 85 % |
| S313 | S210 | 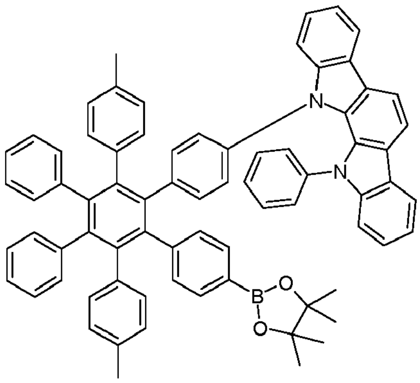 | 85 % |

(fortgesetzt)

| Bsp. | Edukt | Produkt | Ausbeute |
|---|---|---|---|
| S314 | S211 | 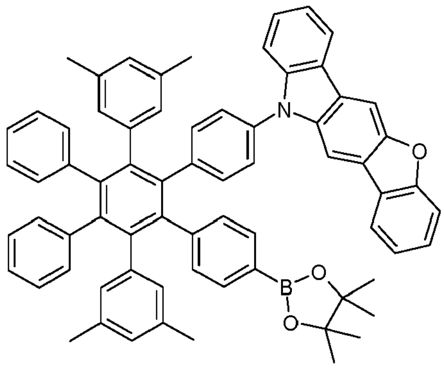 | 87 % |
| S315 | S212 | 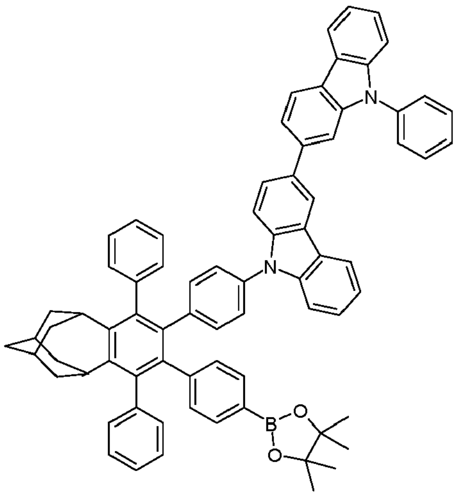 | 86 % |
| S316 | S213 | 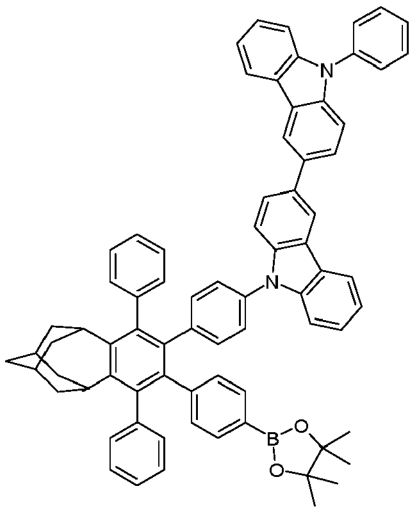 | 88 % |

(fortgesetzt)

| Bsp. | Edukt | Produkt | Ausbeute |
|---|---|---|---|
| S317 | S214 | 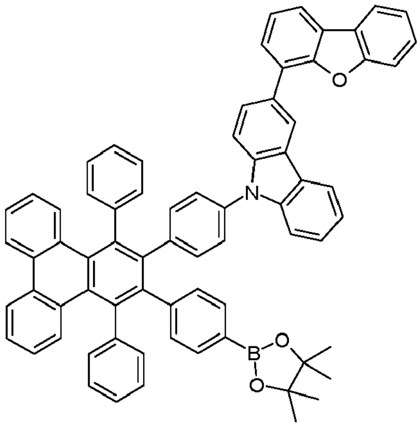 | 79 % |
| S318 | S215 | 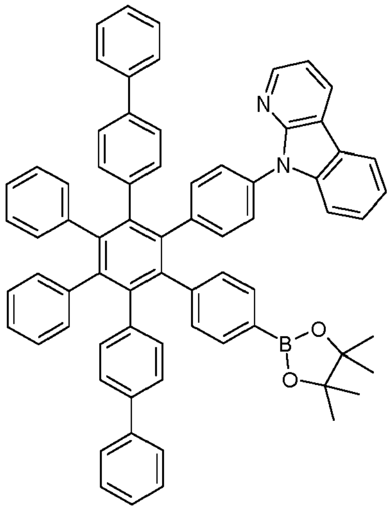 | 81 % |
| S319 | S216 | 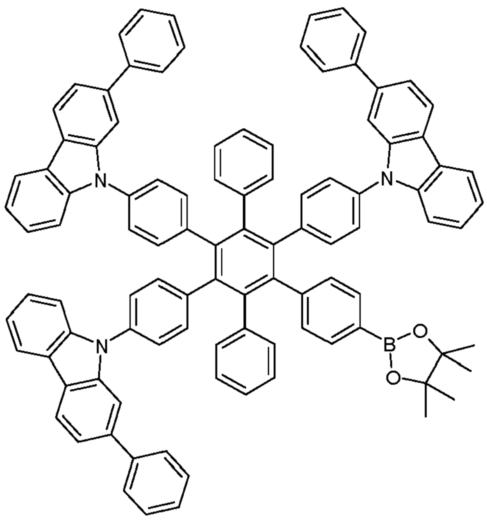 | 90 % |

(fortgesetzt)

| Bsp. | Edukt | Produkt | Ausbeute |
|---|---|---|---|
| S320 | S217 | 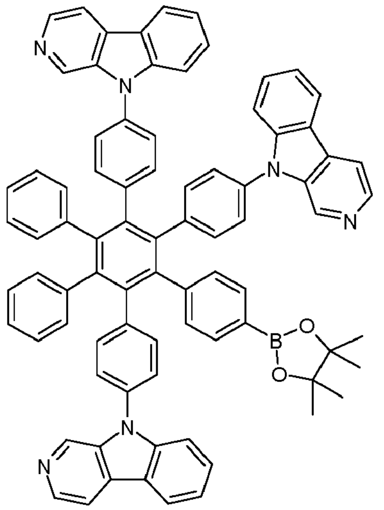 | 80 % |
| S321 | S218 | 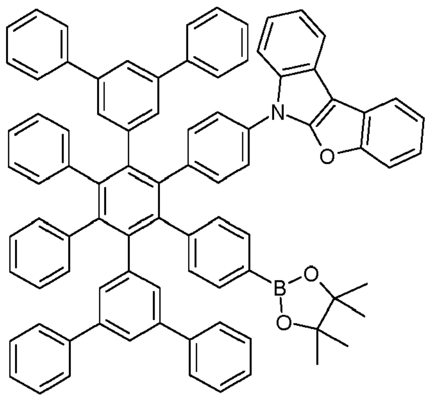 | 88 % |
| S322 | S219 | 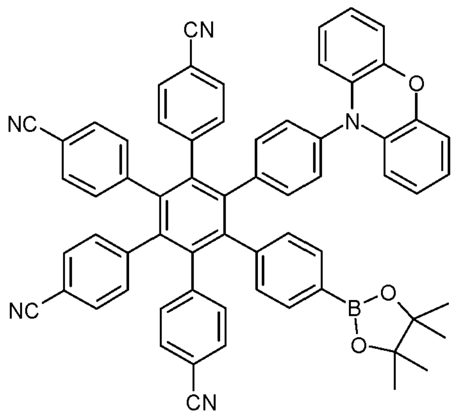 | 74 % |

(fortgesetzt)

| Bsp. | Edukt | Produkt | Ausbeute |
|---|---|---|---|
| S323 | S220 | 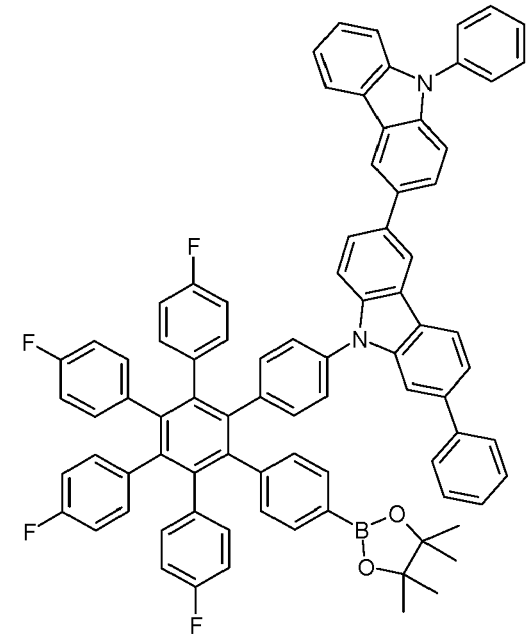 | 82 % |
| S324 | S221 | 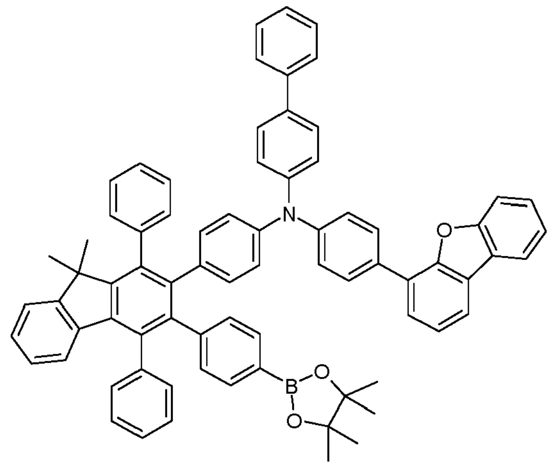 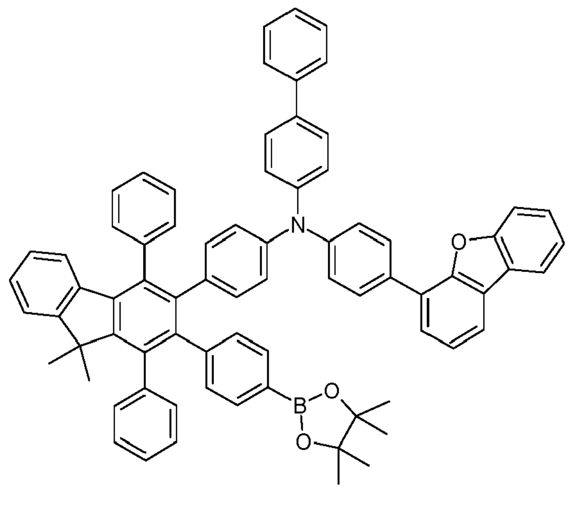 Isomerengemisch | 76 % |

(fortgesetzt)

| Bsp. | Edukt | Produkt | Ausbeute |
|---|---|---|---|
| S325 | S222 | 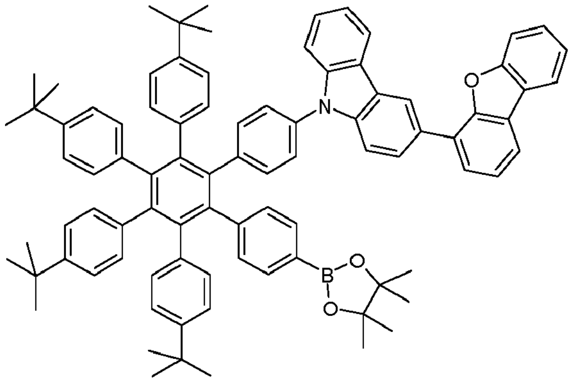 | 69 % |

(fortgesetzt)

| Bsp. | Edukt | Produkt | Ausbeute |
|---|---|---|---|
| S326 | S223 | 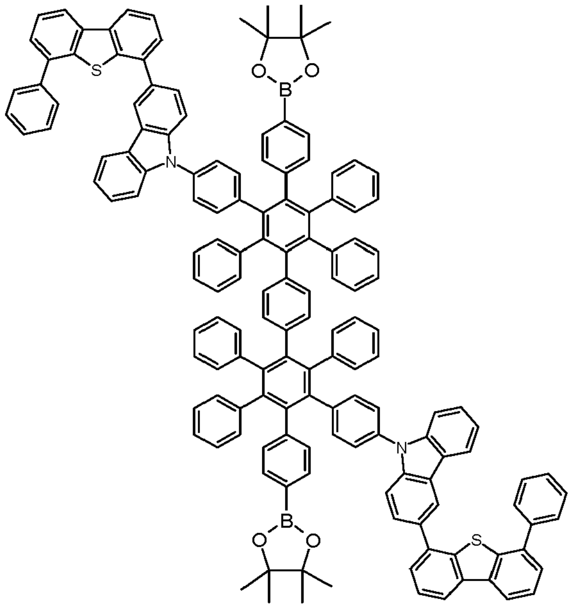 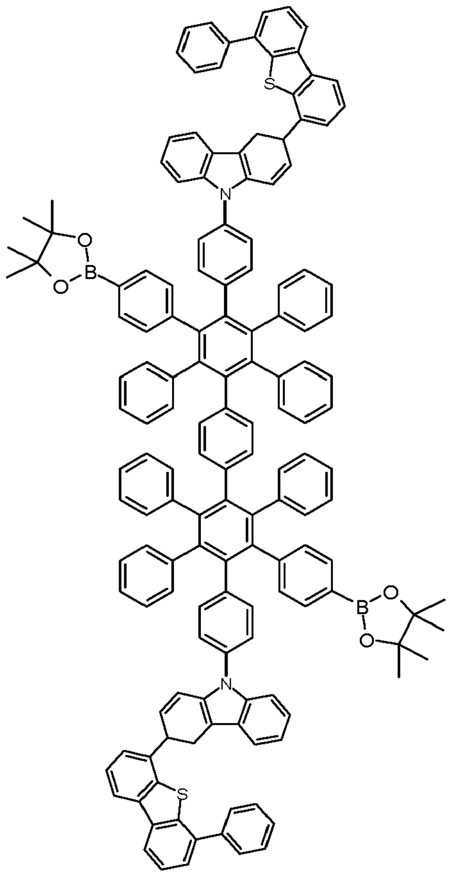 | |

(fortgesetzt)

| Bsp. | Edukt | Produkt | Ausbeute |
|---|---|---|---|
| | | 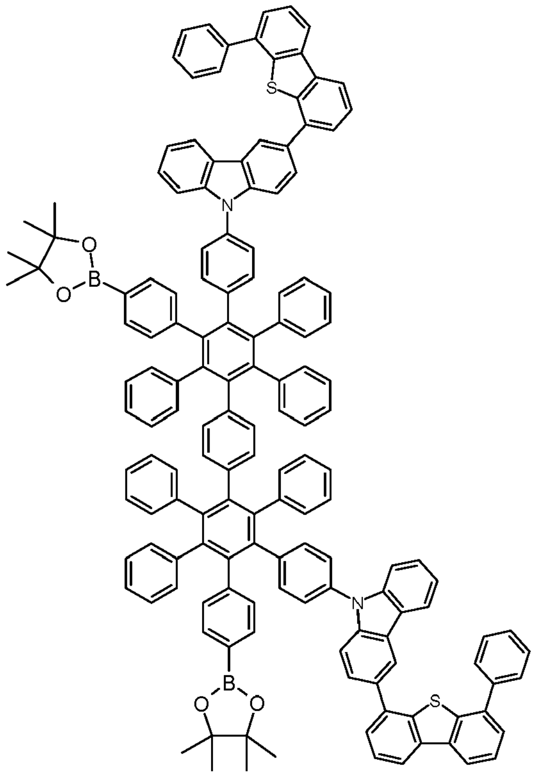 Isomerengemisch 210 mmol 73183-34-3 | |
| S327 | S224 | 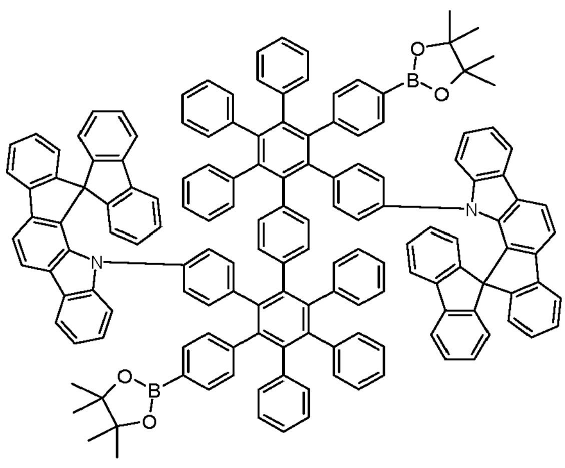 | 67 % |

(fortgesetzt)

| Bsp. | Edukt | Produkt | Ausbeute |
|---|---|---|---|
| | | 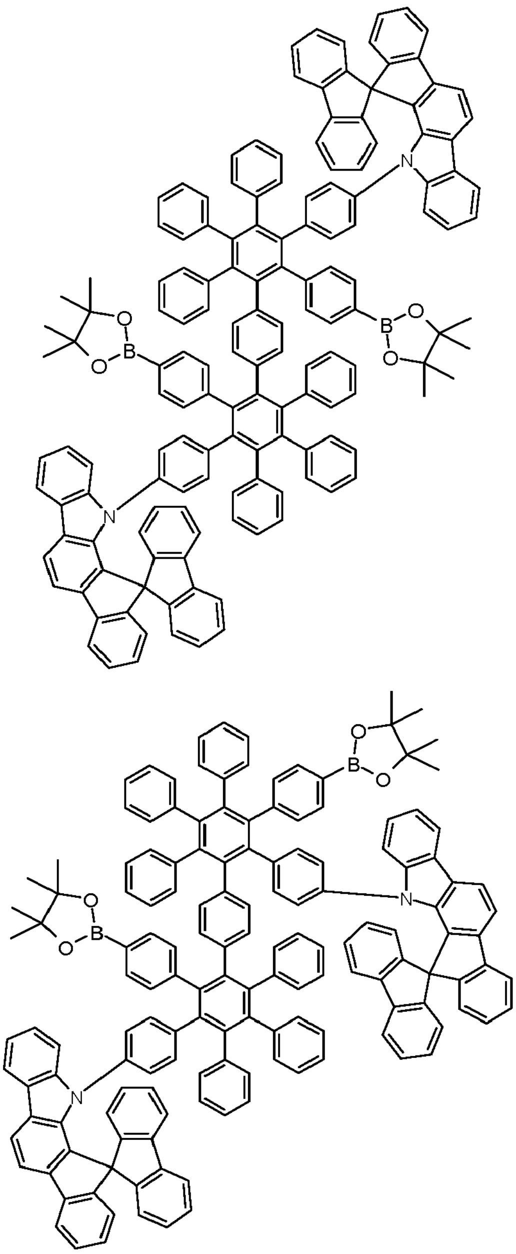 Isomerengemisch 210 mmol 73183-34-3 | |

(fortgesetzt)

| Bsp. | Edukt | Produkt | Ausbeute |
|---|---|---|---|
| S328 | S225 | 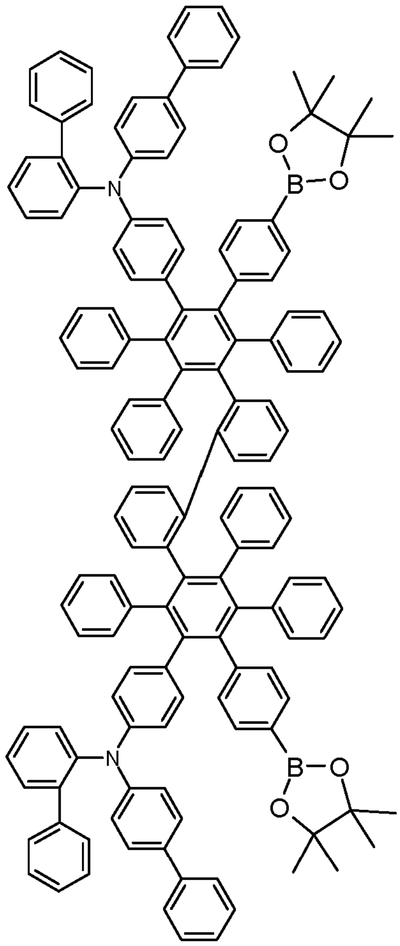 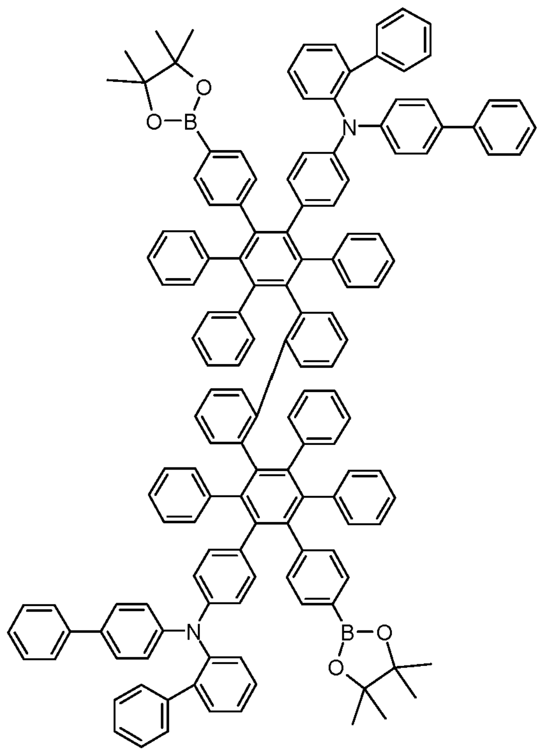 | 71 % |

(fortgesetzt)

| Bsp. | Edukt | Produkt | Ausbeute |
|---|---|---|---|
| | | 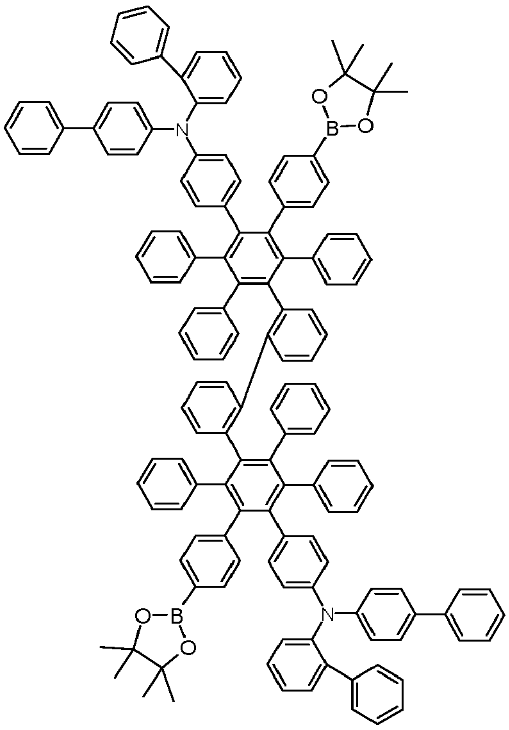 Isomerengemisch 210 mmol 73183-34-3 | |

**Beispiel P1: Suzuki-Kupplung**

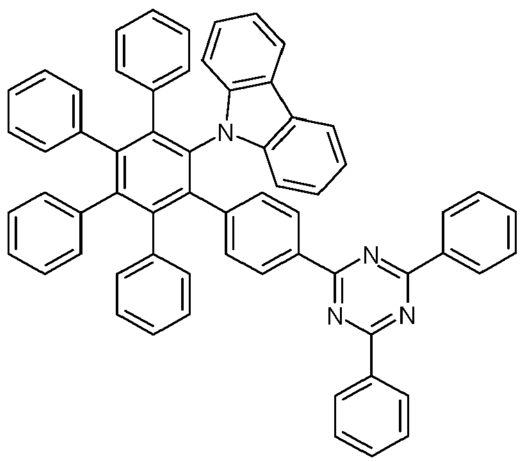

Ein gut gerührtes Gemisch aus 75.0 g (100 mmol) S300, 28.1 g (105 mmol) 1-Chlor-3,5-diphenyltriazin [3842-55-5], 63.7 g (300 mmol) Trikaliumphosphat, 500 ml Toluol, 300 ml Dioxan, 500 ml Wasser wird mit 1.2 g (3 mmol) S-Phos [657408-07-6] und 498 mg (2 mmol) Palladium(II)acetat versetzt und dann 16 h unter Rückfluss erhitzt. Nach Erkalten trennt man die org. Phase ab, wäscht diese zweimal mit je 300 ml Wasser, einmal mit 300 ml ges. Kochsalzlösung und trocknet dann über Magnesiumsulfat. Man filtriert vom Trockenmittel über ein mit Toluol vorgeschlämmtes Celitebett ab, wäscht mit 300 ml Toluol nach, engt das Filtrat zur Trockene ein und kristallisiert den Rückstand zweimal aus Dimethylacetamid um. Die weitere Reinigung erfolgt durch wiederholte Heißextraktion mit n-Butylacetat und anschließende fraktionierte Sublimation (p ca. $10^{-5}$ mbar, T ca. 330 °C). Ausbeute: 47.9 g (56 mmol), 56 %; Reinheit: 99.9 % n. $^1$H-NMR.

Analog können folgende Verbindungen dargestellt werden, wobei Produkte mit Molamasse größer 1200 g/mol typischerweise durch tempern im Hochvakuum von Restlösemitteln befreit werden:

| Bsp. | Edukt | Produkt | Ausbeute |
|---|---|---|---|
| P2 | S101<br>1215596-23-6 | 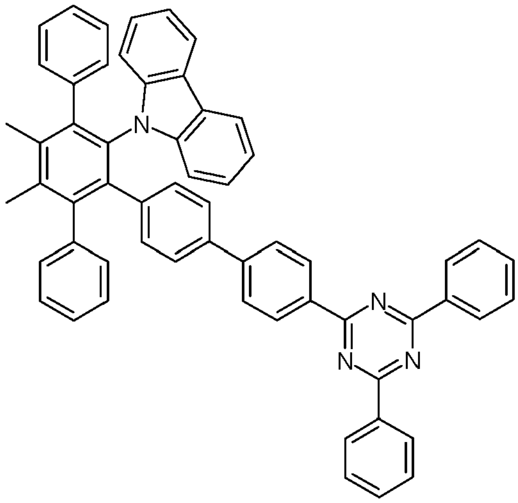 | 54 % |
| P3 | S102<br>1613163-88-4 | 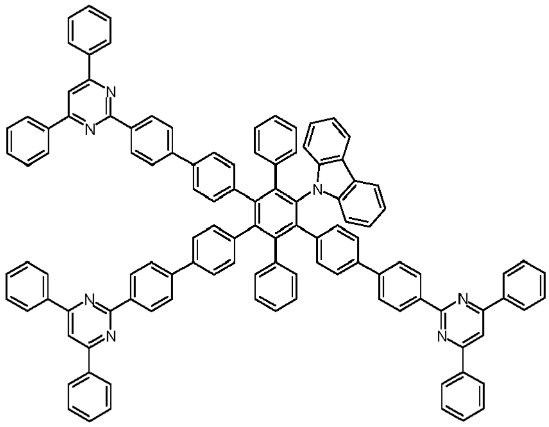<br>315 mmol 1613163-88-4<br>6 mmol S-Phos / 4 mmol $Pd(ac)_2$ | 49 % |
| P4 | S303<br>2915-16-4 | 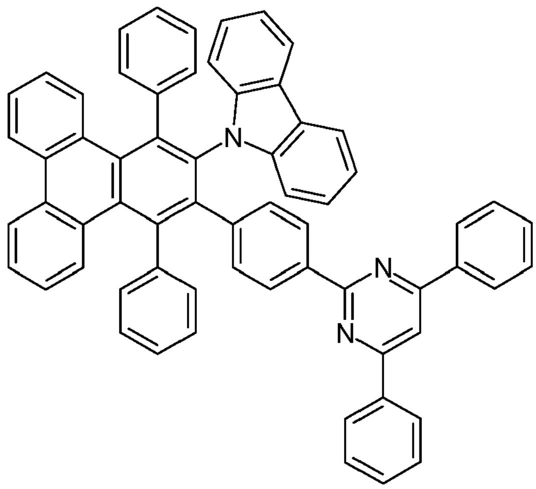 | 59 % |

(fortgesetzt)

| Bsp. | Edukt | Produkt | Ausbeute |
|---|---|---|---|
| P5 | S304<br>3842-55-5 | 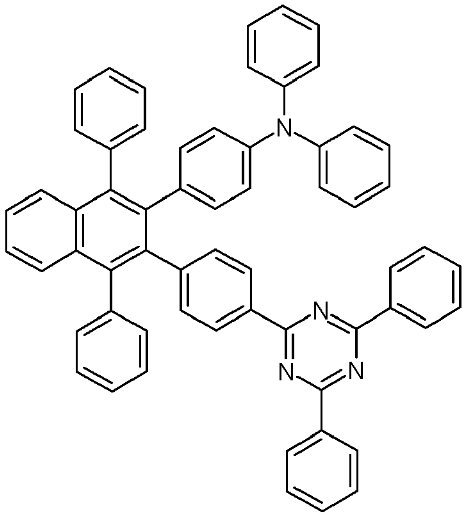 | 57 % |
| P6 | S305<br>2915-16-4 | 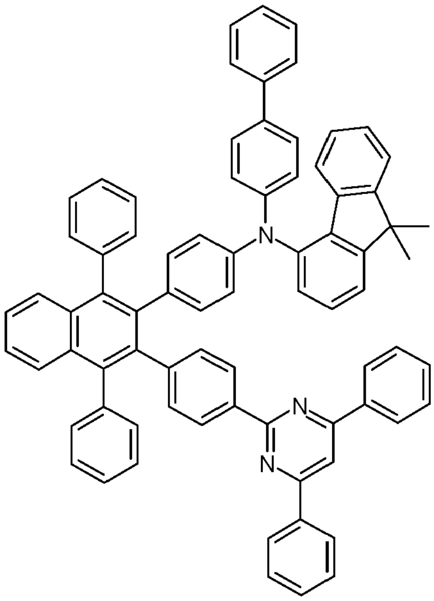 | 53 % |
| P7 | S306<br>29874-83-7 | 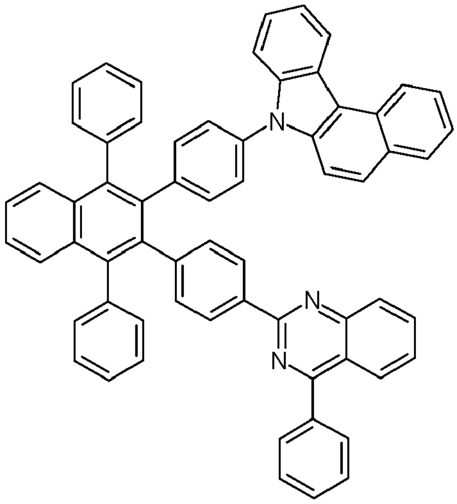 | 55 % |

(fortgesetzt)

| Bsp. | Edukt | Produkt | Ausbeute |
|---|---|---|---|
| P8 | S307<br>1300115-09-6 | 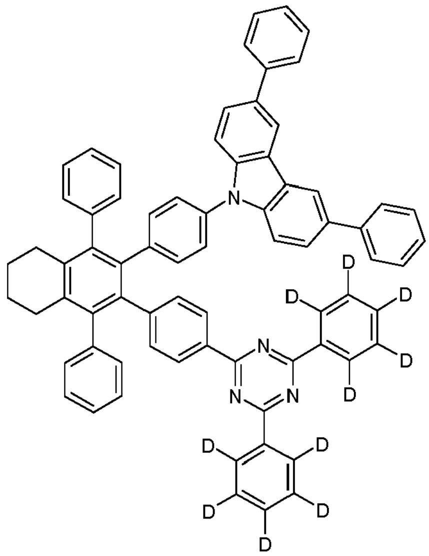 | 51 % |
| P9 | S308<br>6484-25-9 | 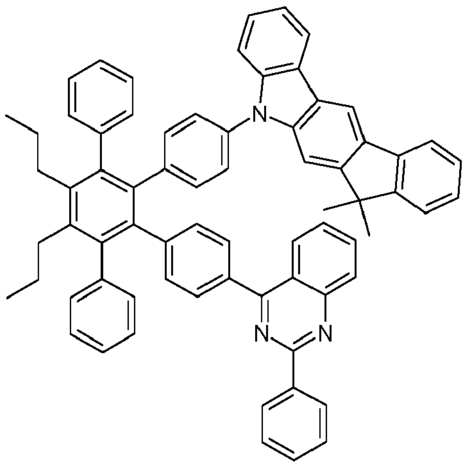 | 49 % |

(fortgesetzt)

| Bsp. | Edukt | Produkt | Ausbeute |
|---|---|---|---|
| P10 | S309<br>24547-45-3 | 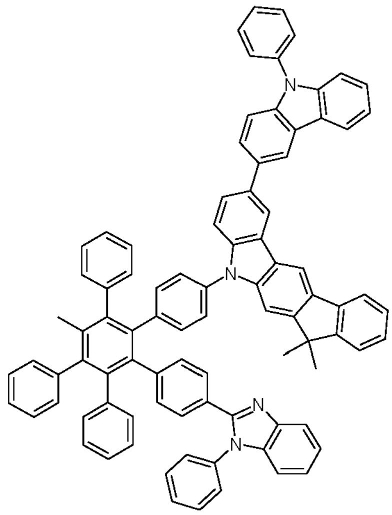<br>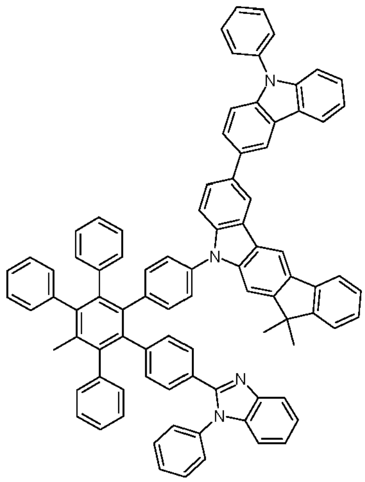<br>Isomerengemisch | 38 % |
| P11 | S310<br>23449-08-3 | 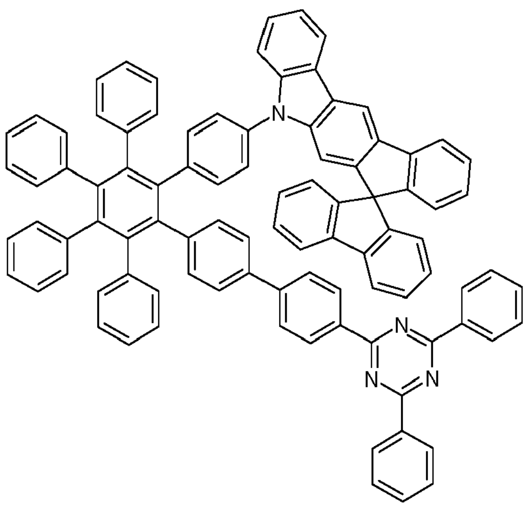 | 55 % |

(fortgesetzt)

| Bsp. | Edukt | Produkt | Ausbeute |
|---|---|---|---|
| P12 | S311 864377-31-1 | 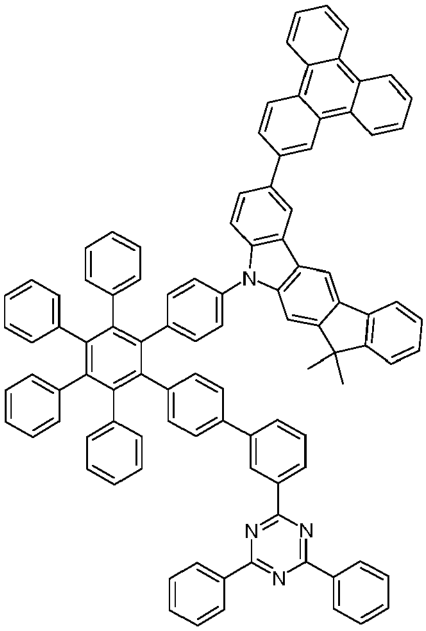 | 58 % |
| P13 | S312 1616231-57-2 | 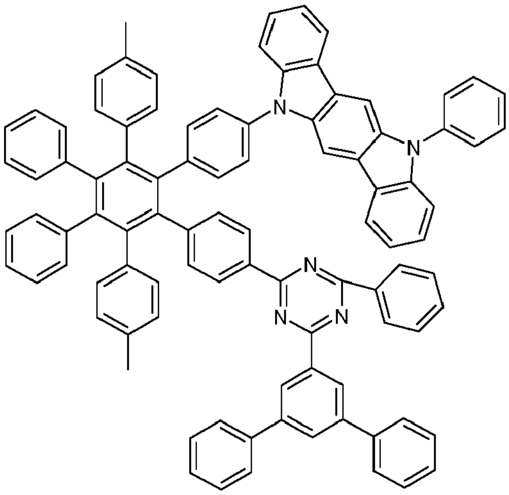 | 54 % |
| P14 | S313 1689576-03-1 | 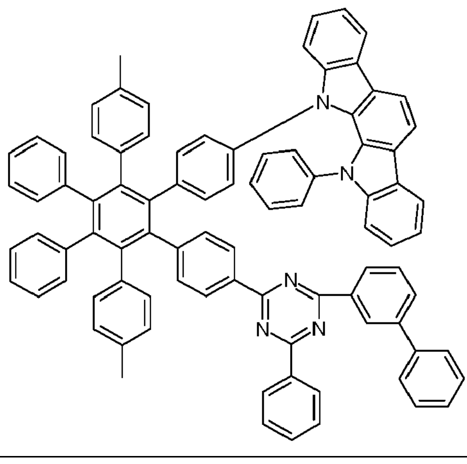 | 50 % |

(fortgesetzt)

| Bsp. | Edukt | Produkt | Ausbeute |
|---|---|---|---|
| P15 | S314<br>55635-65-9 | 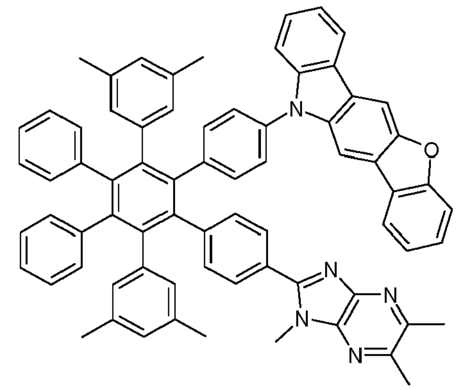 | 57 % |
| P16 | S315<br>1931136-94-5 | 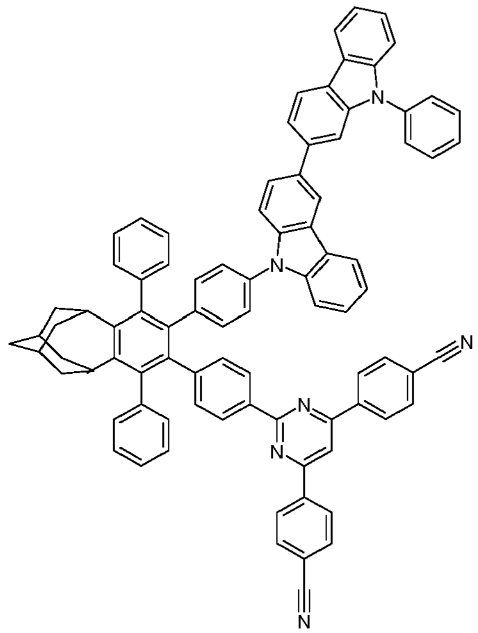 | 51 % |
| P17 | S316<br>3842-55-5 | 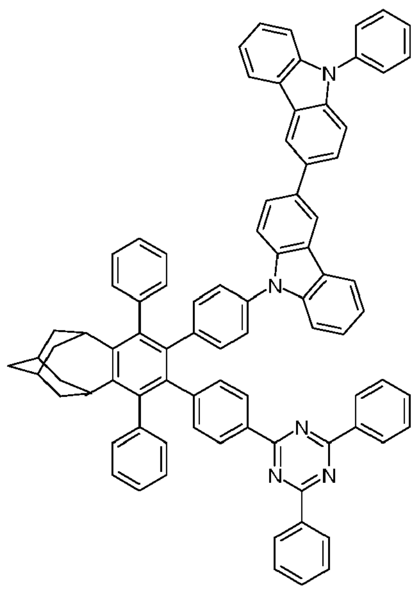 | 53 % |

(fortgesetzt)

| Bsp. | Edukt | Produkt | Ausbeute |
|---|---|---|---|
| P18 | S317<br>3842-55-5 | 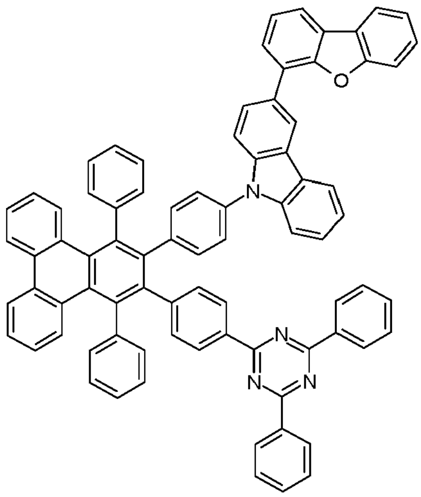 | 59 % |
| P19 | S318<br>1439929-51-7 | 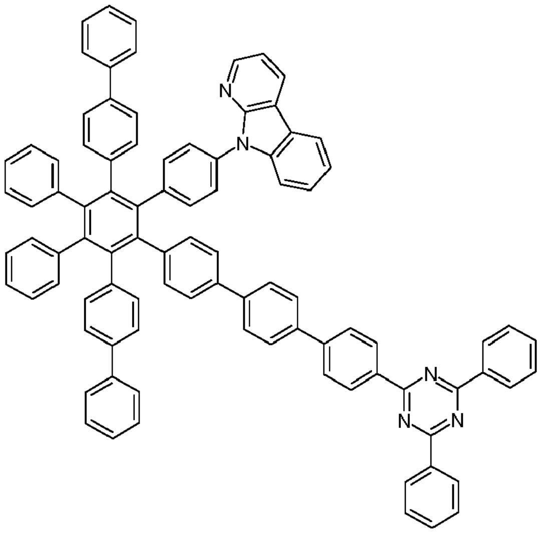 | 55 % |
| P20 | S319<br>334-04-7 | 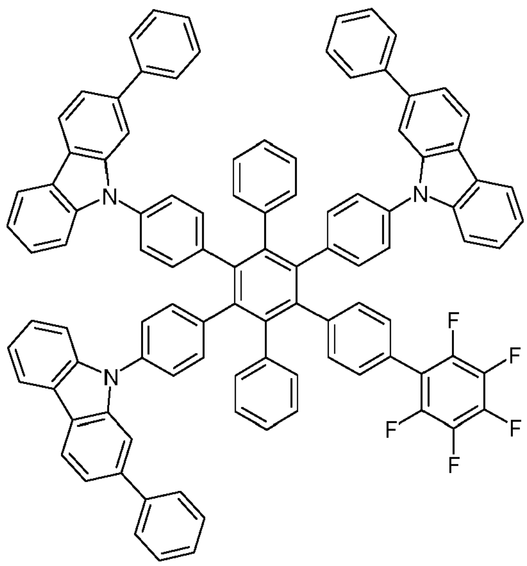 | 52 % |

(fortgesetzt)

| Bsp. | Edukt | Produkt | Ausbeute |
|---|---|---|---|
| P21 | S320 371-88-6 | 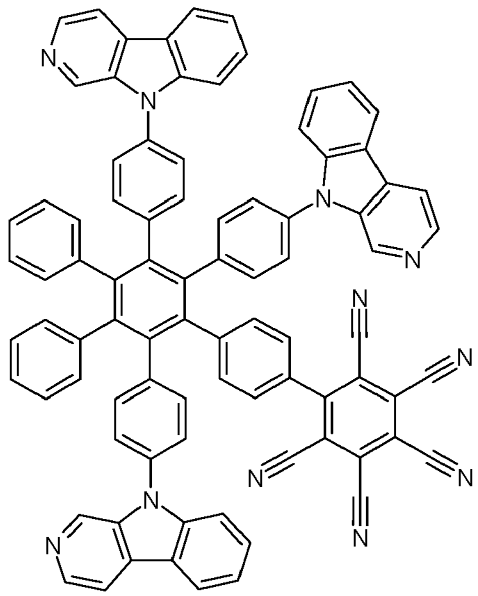 | 56 % |
| P22 | S321 80587-76-4 | 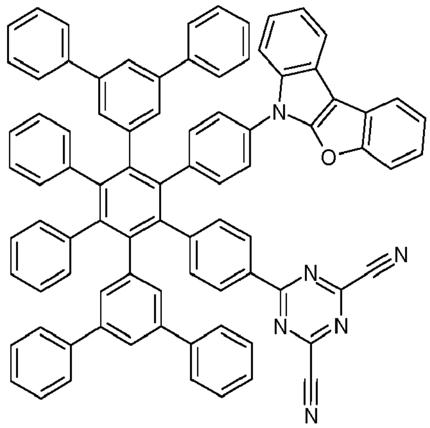 | 49 % |
| P23 | S322 37084-03-4 | 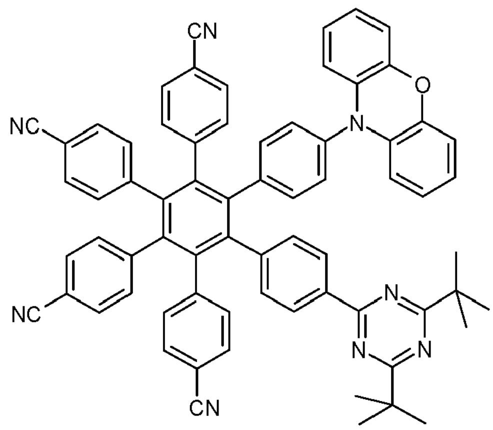 | 44 % |

(fortgesetzt)

| Bsp. | Edukt | Produkt | Ausbeute |
|---|---|---|---|
| P24 | S323<br>334-04-7 | 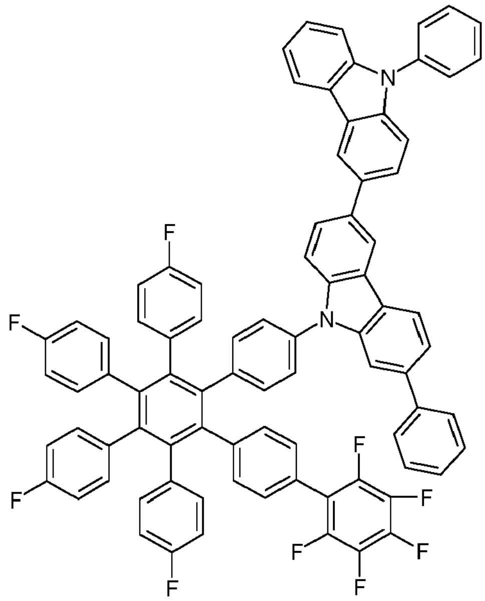 | 47 % |

(fortgesetzt)

| Bsp. | Edukt | Produkt | Ausbeute |
|---|---|---|---|
| P25 | S324<br>3842-55-5 | 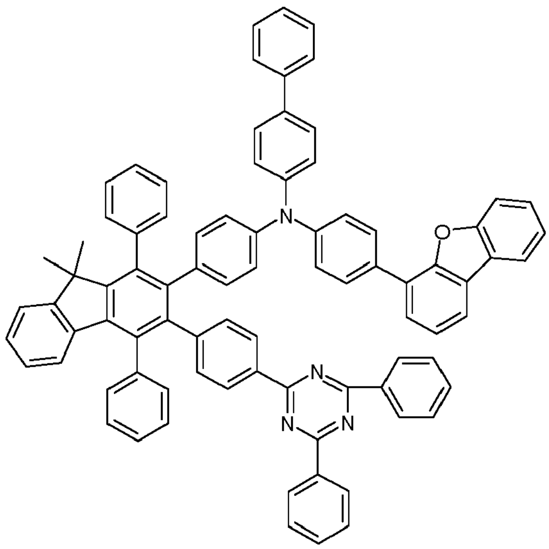<br>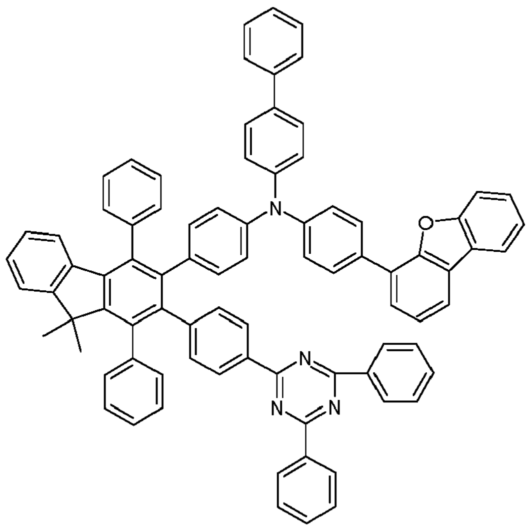<br>Isomerengemisch | 30 % |
| P26 | S325<br>3842-55-5 | 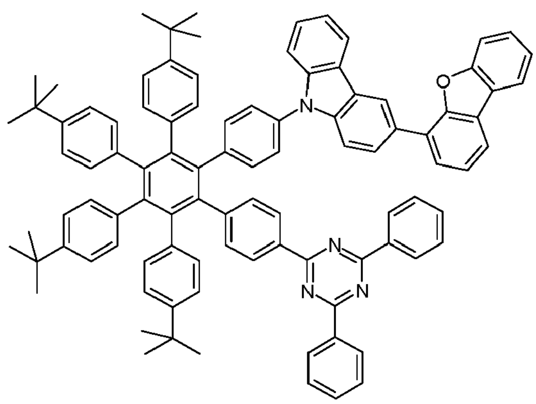 | |

(fortgesetzt)

| Bsp. | Edukt | Produkt | Ausbeute |
|---|---|---|---|
| P27 | S326<br>15679-03-5 | 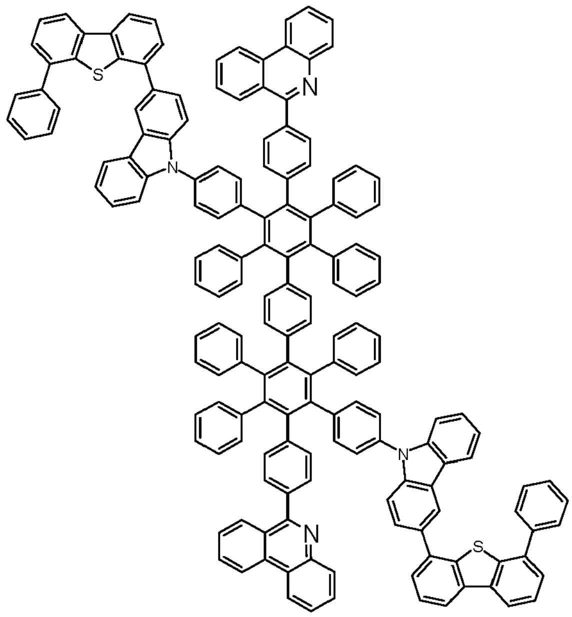 | 32 % |

(fortgesetzt)

| Bsp. | Edukt | Produkt | Ausbeute |
|---|---|---|---|
| | | 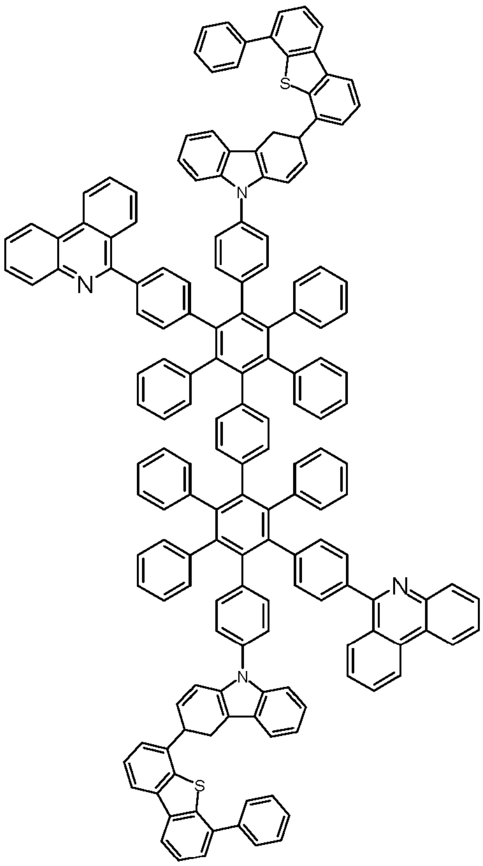<br>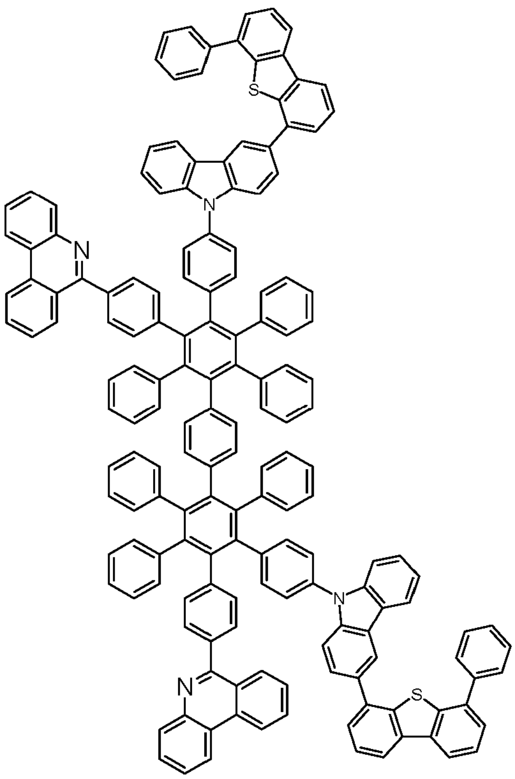<br>Isomerengemisch 210 mmol 73183-34-3 | |

(fortgesetzt)

| Bsp. | Edukt | Produkt | Ausbeute |
|---|---|---|---|
| P28 | S327<br>3842-55-5 | 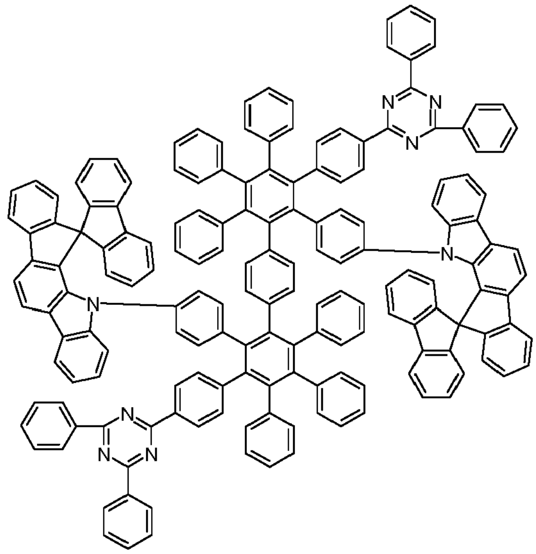<br>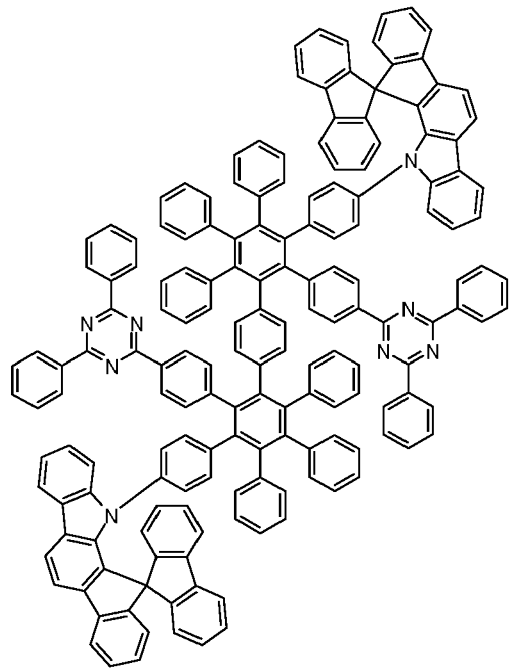 | 27 % |

(fortgesetzt)

| Bsp. | Edukt | Produkt | Ausbeute |
|---|---|---|---|
| | | 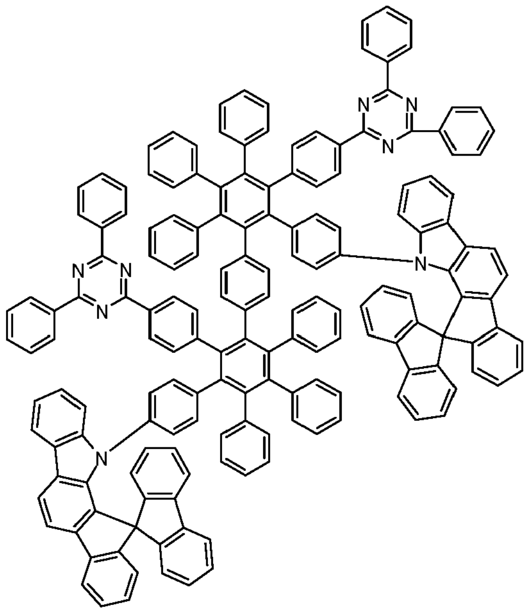 Isomerengemisch 210 mmol 73183-34-3 | |
| P29 | S328 3842-55-5 | 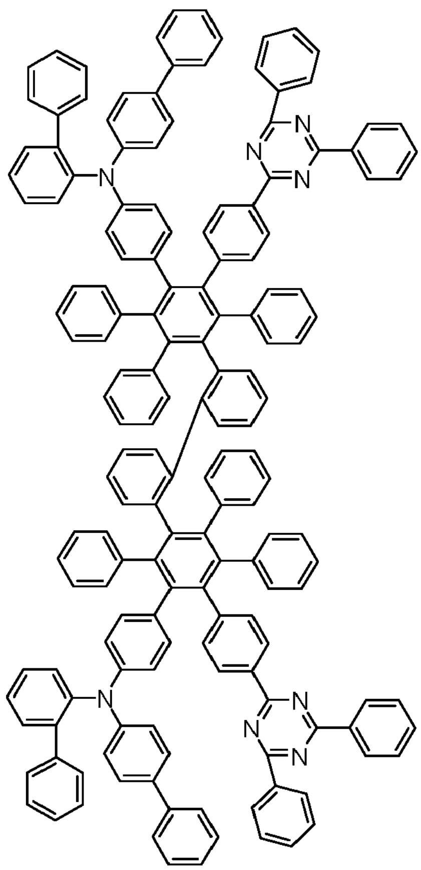 | 29 % |

(fortgesetzt)

| Bsp. | Edukt | Produkt | Ausbeute |
|---|---|---|---|
| | | 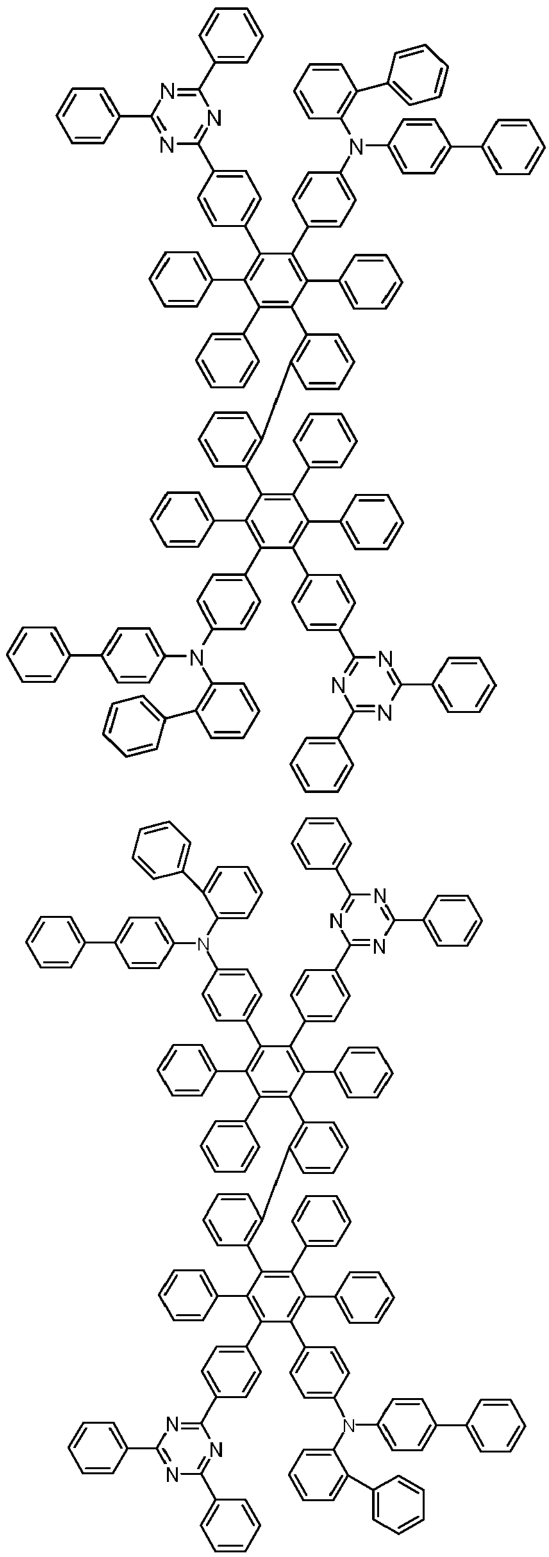 Isomerengemisch 210 mmol 73183-34-3 | |

**Beispiel: Herstellung der OLEDs**

**1) Vakuum-prozessierte Devices:**

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, verwendete Materialien) angepasst wird.

In den folgenden Beispielen werden die Ergebnisse verschiedener OLEDs vorgestellt. Glasplättchen, mit strukturiertem ITO (50 nm, Indium-Zinn-Oxid) bilden die Substrate, auf welche die OLEDs aufgebracht werden. Die OLEDs haben prinzipiell folgenden Schichtaufbau: Substrat / Lochtransportschicht 1 (HTL1) bestehend aus HTM dotiert mit 5 % NDP-9 (kommerziell erhältlich von der Fa. Novaled), 20 nm / Lochtransportschicht 2 (HTL2) / optionale Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet.

Zunächst werden vakuum-prozessierte OLEDs beschrieben. Hierfür werden alle Materialien in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Co Verdampfung in einem bestimmten Volumenanteil beigemischt wird. Eine Angabe wie M3:M2:Ir(L2) (55%:35%:10%) bedeutet hierbei, dass das Material M3 in einem Volumenanteil von 55%, M2 in einem Anteil von 35% und Ir(L2) in einem Anteil von 10% in der Schicht vorliegt. Analog kann auch die Elektronentransportschicht aus einer Mischung zweier Materialien bestehen. Der genaue Aufbau der OLEDs ist Tabelle 1 zu entnehmen. Die zur Herstellung der OLEDs verwendeten Materialien sind in Tabelle 4 gezeigt.

Die OLEDs werden standardmäßig charakterisiert. Hierfür werden die Elektrolumineszenzspektren, die Stromeffizienz (gemessen in cd/A) und die Spannung (gemessen bei 1000 cd/m$^2$ in V) bestimmt aus Strom-Spannungs-Helligkeits-Kennlinien (IUL-Kennlinien). Für ausgewählte Versuche wird die Lebensdauer bestimmt. Als Lebensdauer wird die Zeit definiert, nach der die Leuchtdichte von einer bestimmten Startleuchtdichte aus auf einen gewissen Anteil abgesunken ist. Die Angabe LD50 bedeutet, dass es sich bei der genannten Lebensdauer um die Zeit handelt, bei der die Leuchtdichte auf 50% der Startleuchtdichte abgefallen ist, also von z.B. 1000 cd/m$^2$ auf 500 cd/m$^2$. Je nach Emissionsfarbe wurden unterschiedliche Starthelligkeiten gewählt. Die Werte für die Lebensdauer können mit Hilfe dem Fachmann bekannten Umrechnungsformeln auf eine Angabe für andere Startleuchtdichten umgerechnet werden. Hierbei ist die Lebensdauer für eine Startleuchtdichte von 1000 cd/m$^2$ eine übliche Angabe.

**Verwendung von erfindungsgemäßen Verbindungen als Emittermaterialien in phosphoreszierenden OLEDs**

Die erfindungsgemäßen Verbindungen lassen sich unter anderem als Emittermaterialien (TADF) in der Emissionsschicht in OLEDs einsetzen. Außerdem sind sie als Matrix / Hostmaterial für phosphoreszente Emitter einsetzbar. Als Vergleich gemäß dem Stand der Technik werden die Verbindungen gemäß Tabelle 4 verwendet. Die Ergebnisse der OLEDs sind in Tabelle 2 zusammengefasst.

**Tabelle 1: Aufbau der OLEDs**

| Bsp. | HTL2 Dicke | EBL Dicke | EML Dicke | HBL Dicke | ETL Dicke |
|---|---|---|---|---|---|
| Ref.-D1 | HTM 40 nm | --- | M1 :IrRef1 (88%:12%) 35 nm | HBM1 10 nm | ETM 1: ETM2 (50%:50%) 30 nm |
| D1 | HTM 40 nm | --- | P1 :IrRef1 (88%:12%) 35 nm | HBM1 10 nm | ETM 1: ETM2 (50%:50%) 30 nm |
| D2 | HTM 40 nm | --- | P4:IrRef1 (88%:12%) 35 nm | HBM1 10 nm | ETM 1: ETM2 (50%:50%) 30 nm |
| D3 | HTM 40 nm | --- | P4:IrRef1 (80%:20%) 35 nm | HBM1 10 nm | ETM 1: ETM2 (50%:50%) 30 nm |
| D4 | HTM 40 nm | --- | P4:M3:IrRef1 (60%:30%:10%) 35 nm | HBM1 10 nm | ETM 1: ETM2 (50%:50%) 30 nm |
| D5 | HTM 40 nm | --- | P18:M3 (60%:40%) 40 nm | HBM1 10 nm | ETM 1: ETM2 (50%:50%) 30 nm |

(fortgesetzt)

| Bsp. | HTL2 Dicke | EBL Dicke | EML Dicke | HBL Dicke | ETL Dicke |
|---|---|---|---|---|---|
| D6 | HTM 40 nm | --- | P18:M3:SER1 (60%:35%:5%) 40 nm | HBM1 10 nm | ETM 1: ETM2 (50%:50%) 30 nm |

**Tabelle 2: Ergebnisse der Vakuum-prozessierten OLEDs**

| Bsp. | EQE (%) 1000 cd/m$^2$ | Spannung (V) 1000 cd/m$^2$ | CIE x/y 1000 cd/m$^2$ | LD50 (h) 1000 cd/m$^2$ |
|---|---|---|---|---|
| Ref.-D1 | 18.8 | 3.2 | 0.34/0.62 | 190000 |
| D1 | 18.9 | 3.0 | 0.33/0.62 | 240000 |
| D2 | 19.2 | 2.9 | 0.34/0.62 | 230000 |
| D3 | 19.4 | 2.9 | 0.33/0.63 | 340000 |
| D4 | 19.0 | 3.1 | 0.34/0.62 | 360000 |
| **Bsp.** | **EQE (%) 500 cd/m$^2$** | **Spannung (V) 500 cd/m$^2$** | **CIE x/y 500 cd/m$^2$** | **LD50 (h) 500 cd/m$^2$** |
| D5 | 12.3 | 3.4 | 0.26/0.54 | 20000 |
| D6 | 15.7 | 3.5 | 0.51/0.47 | 90000 |

**Lösungs-prozessierte Devices:**

**Aus niedermolekularen löslichen Funktionsmaterialien**

Die erfindungsgemäßen Iridium-Komplexe können auch aus Lösung verarbeitet werden und führen dort zu prozesstechnisch wesentlich einfacheren OLEDs, im Vergleich zu den vakuumprozessierten OLEDs, mit dennoch guten Eigenschaften. Die Herstellung solcher Bauteile lehnt sich an die Herstellung polymerer Leuchtdioden (PLEDs) an, die in der Literatur bereits vielfach beschrieben ist (z. B. in der WO 2004/037887). Der Aufbau setzt sich aus Substrat / ITO / Lochinjektionsschicht (60 nm) / Interlayer (20 nm) / Emissionsschicht (60 nm) / Lochblockierschicht (10 nm) / Elektronentransportschicht (40 nm) / Kathode zusammen. Dazu werden Substrate der Firma Technoprint (Sodalimeglas) verwendet, auf welche die ITO-Struktur (Indium-Zinn-Oxid, eine transparente, leitfähige Anode) aufgebracht wird. Die Substrate werden im Reinraum mit DI Wasser und einem Detergens (Deconex 15 PF) gereinigt und dann durch eine UV/Ozon-Plasmabehandlung aktiviert. Danach wird ebenfalls im Reinraum eine 20 nm Lochinjektionsschicht durch Spin-Coating aufgebracht. Die benötigte Spinrate hängt vom Verdünnungsgrad und der spezifischen Spin-Coater-Geometrie ab. Um Restwasser aus der Schicht zu entfernen, werden die Substrate für 30 Minuten bei 200 °C auf einer Heizplatte ausgeheizt. Die verwendete Interlayer dient dem Lochtransport, in diesem Fall wird HL-X von Merck verwendet. Die Interlayer kann alternativ auch durch eine oder mehrere Schichten ersetzt werden, die lediglich die Bedingung erfüllen müssen, durch den nachgelagerten Prozessierungsschritt der EML-Abscheidung aus Lösung nicht wieder abgelöst zu werden. Zur Herstellung der Emissionsschicht werden die erfindungsgemäßen Triplettemitter zusammen mit den Matrixmaterialien in Toluol oder Chlorbenzol gelöst. Der typische Feststoffgehalt solcher Lösungen liegt zwischen 16 und 25 g/L, wenn, wie hier, die für eine Device typische Schichtdicke von 60 nm mittels Spincoating erzielt werden soll. Die lösungsprozessierten grünen Devices vom Typ1 enthalten eine Emissionsschicht aus P:M:IrRef2 (X%:Y%:Z%), die roten vom Typ2 enthalten eine Emissionsschicht aus P:M:IrRef2:IrRef3 (X%:Y%:Z%:5%), d.h. sie enthalten zwei verschiedene Ir-Komplexe. Die Emissionsschicht wird in einer Inertgasatmosphäre, im vorliegenden Fall Argon, aufgeschleudert und 10 min bei 160 °C ausgeheizt. Darüber wird die Lochblockierschicht (10nm ETM1) und die Elektronentransportschicht (40nm ETM1 (50%) / ETM2 (50%)) aufgedampft (Aufdampfanlagen von Lesker o.a., typischer Aufdampfdruck $5 \times 10^{-6}$ mbar). Zuletzt wird eine Kathode aus Aluminium (100 nm) (hochreines Metall von Aldrich) aufgedampft. Um das Device vor Luft und Luftfeuchtigkeit zu schützen, wird die Vorrichtung abschließend verkapselt und dann charakterisiert. Die genannten OLED-Beispiele sind noch nicht optimiert, Tabelle 3 fasst die erhaltenen Daten zusammen.

**Tabelle 3: Ergebnisse mit aus Lösung prozessierten Materialien**

| Bsp. | P:M:IrRef | EQE (%) 1000 cd/m$^2$ | Spannung (V) 1000 cd/m$^2$ | CIE x/y | LD50 (h) 1000 cd/m$^2$ |
|---|---|---|---|---|---|
| Rote Devices vom Typ2 | | | | | |
| Sol-Ref-Red1 | M4:M5: IrRef2 30%:40%:25% | 17.4 | 6.2 | 0.66/0.34 | 240000 |
| Sol-RedD1 | P11:M5:IrRef2 70%:0%:25% | 18.3 | 5.8 | 0.66/0.34 | 290000 |
| Sol-RedD2 | P11:M5:IrRef2 50%:20%:25% | 18.6 | 5.9 | 0.66/0.34 | 320000 |
| Sol-RedD3 | P28:M5:IrRef2 70%:0%:25% | 18.8 | 6.1 | 0.66/0.34 | 370000 |
| **Grüne Devices vom Typ1** | | | | | |
| Sol-Ref-Green1 | M4:M5: IrRef2 20%:60%:20% | 20.1 | 5.3 | 0.33/0.62 | 200000 |
| Sol-GreenD1 | P10:M5:IrRef2 80%:0%:20% | 20.6 | 5.4 | 0.33/0.62 | 230000 |
| Sol-GreenD2 | P10:M5:IrRef2 50%:30%:20% | 20.9 | 5.3 | 0.33/0.62 | 220000 |
| Sol-GreenD3 | P11:M5:IrRef2 60%:20%:20% | 20.8 | 5.1 | 0.33/0.62 | 240000 |
| Sol-GreenD4 | P13:M5:IrRef2 50%:30%:20% | 20.5 | 5.2 | 0.33/0.62 | 240000 |
| Sol-GreenD5 | P14:M4:IrRef2 45%:30%:25% | 20.2 | 5.0 | 0.34/0.61 | 250000 |
| Sol-GreenD6 | P18:M3:IrRef2 50%:35%:15% | 20.0 | 5.2 | 0.32/0.63 | 310000 |
| Sol-GreenD7 | P25:M5:IrRef2 80%:0%:20% | 19.9 | 5.1 | 0.33/0.62 | 260000 |
| Sol-GreenD8 | P28:M5:IrRef2 75%:0%:25% | 20.3 | 5.0 | 0.32/0.62 | 240000 |

**Tabelle 4: Strukturformeln der verwendeten Materialien**

| | |
|---|---|
| 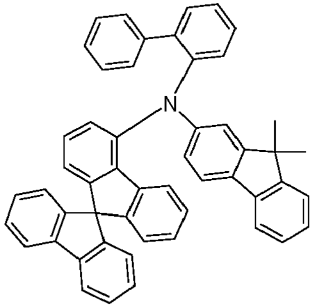 HTM = M9 1450933-44-4 | 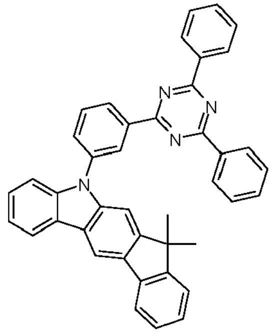 M1 1257248-13-7 |
| 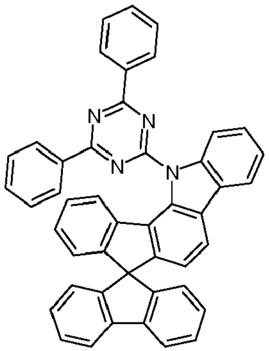 M2 1615703-29-1 | 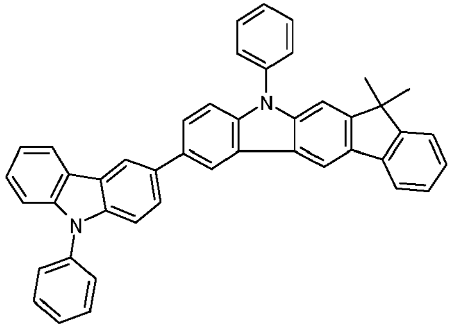 M3 1357150-54-9 |

(fortgesetzt)

| | |
|---|---|
| 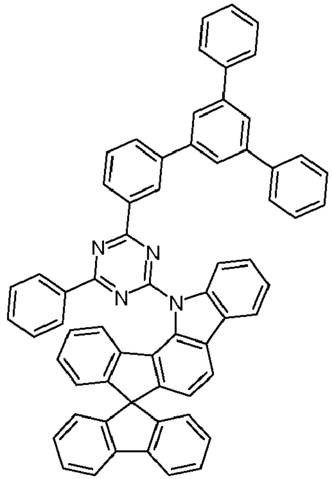 M4 1616231-60-7 | 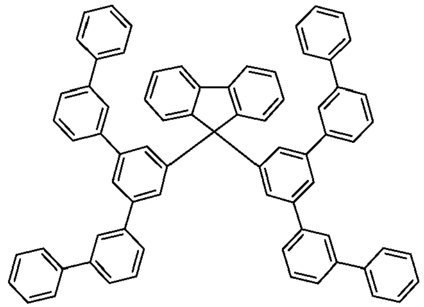 M5 1246496-85-4 |
| 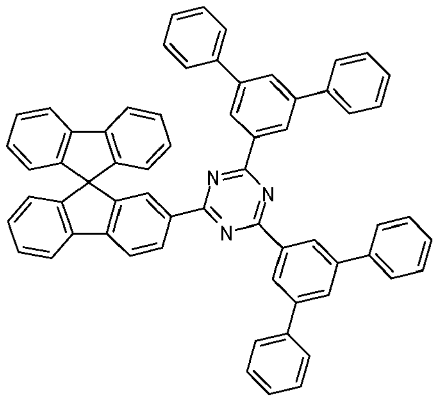 ETM1 = HBM1 = M10 1233200-52-6 | 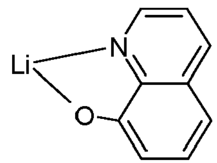 ETM2 25387-93-3 |

(fortgesetzt)

| | |
|---|---|
| 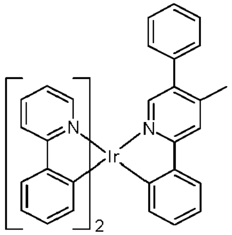 IrRef1 1215692-34-4 | 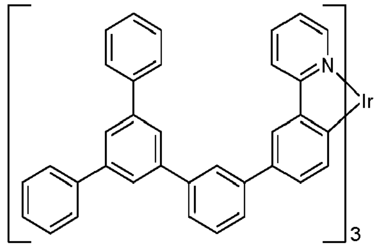 IrRef2 1269508-30-6 |
| 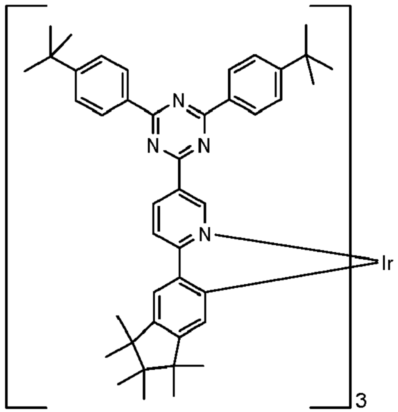 IrRef3 1870013-87-8 | 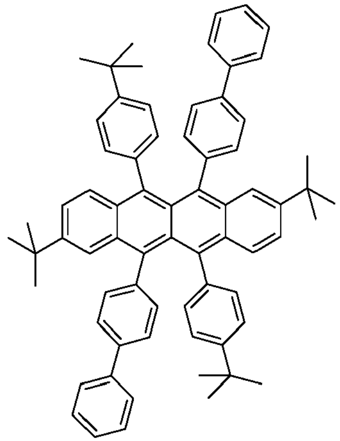 SER1 850797-15-8 |

## Patentansprüche

1. Verbindung, umfassend mindestens eine Struktur der Formel (I):

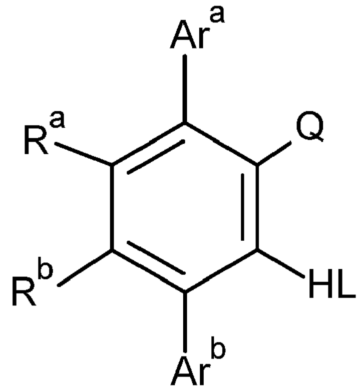

Formel (I)

wobei für die verwendeten Symbole gilt:

Q ist eine Akzeptorgruppe, umfassend ein aromatisches oder heteroaromatisches Ringsystem mit 5 bis 60 aromatischen Ringatomen, welches durch einen oder mehrere Reste $R^1$ substituiert sein kann;
HL ist eine Donorgruppe;
$Ar^3$, $Ar^b$ ist gleich oder verschieden ein aromatisches oder heteroaromatisches Ringsystem mit 5 bis 60 aromatischen Ringatomen, welches durch einen oder mehrere Reste $R^1$ substituiert sein kann;
$R^a$, $R^b$ ist gleich oder verschieden H, D, F, Cl, Br, I, $B(OR^1)_2$, CHO, $C(=O)R^1$, $CR^1=C(R^1)_2$, CN, $C(=O)OR^1$, $C(=O)N(R^1)_2$, $Si(R^1)_3$, $N(R^1)_2$, $NO_2$, $P(=O)(R^1)_2$, $OSO_2R^1$, $OR^1$, $S(=O)R^1$, $S(=O)_2R^1$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^1$ substituiert sein kann, wobei eine oder mehrere nicht benachbarte $CH_2$-Gruppen durch $-R^1C=CR^1-$, -C≡C-, $Si(R^1)_2$, $Ge(R^1)_2$, $Sn(R^1)_2$, C=O, C=S, C=Se, $C=NR^1$, $NR^1$, $P(=O)(R^1)$, -C(=O)O-, $-C(=O)NR^1-$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^1$ substituiert sein kann, oder eine Aryloxy- oder Heteroaryloxygruppe mit 5 bis 60 aromatischen Ringatomen, die durch einen oder mehrere Reste $R^1$ substituiert sein kann, oder eine Aralkyl- oder Heteroaralkylgruppe mit 5 bis 60 aromatischen Ringatomen, die durch einen oder mehrere Reste $R^1$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^1$ substituiert sein kann; oder eine Kombination dieser Systeme, dabei bilden die Reste $R^a$ und $R^b$ miteinander oder mit der Gruppe $Ar^a$ oder $Ar^b$ ein mono- oder polycyclisches, aliphatisches, heteroaliphatisches, aromatisches oder heteroaromatisches Ringsystem;
$R^1$ ist bei jedem Auftreten gleich oder verschieden H, D, F, Cl, Br, I, $B(OR^2)_2$, CHO, $C(=O)R^2$, $CR^2=C(R^2)_2$, CN, $C(=O)OR^2$, $C(=O)N(R^2)_2$, $Si(R^2)_3$, $N(R^2)_2$, $NO_2$, $P(=O)(R^2)_2$, $OSO_2R^2$, $OR^2$, $S(=O)R^2$, $S(=O)_2R^2$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, -C≡C-, $Si(R^2)_2$, $Ge(R^2)_2$, $Sn(R^2)_2$, C=O, C=S, C=Se, $C=NR^2$, $NR^2$, $P(=O)(R^2)$, -C(=O)O-, $-C(=O)NR^2-$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^2$ substituiert sein kann, oder eine Aralkyl- oder Heteroaralkylgruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann; oder eine Kombination dieser Systeme; dabei können zwei oder mehrere Reste $R^1$ miteinander ein mono- oder polycyclisches, aliphatisches, heteroaliphatisches, aromatisches oder heteroaro-

matisches Ringsystem bilden;

$R^2$ ist bei jedem Auftreten gleich oder verschieden H, D, F, Cl, Br, I, $B(OR^3)_2$, CHO, $C(=O)R^3$, $CR^3=C(R^3)_2$, CN, $C(=O)OR^3$, $C(=O)N(R^3)_2$, $Si(R^3)_3$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, -C≡C-, $Si(R^3)_2$, $Si(R^3)_2$, $Ge(R^3)_2$, $Sn(R^3)_2$, C=O, C=S, C=Se, $C=NR^3$, $NR^3$, $P(=O)(R^3)$, -C(=O)O-, $-C(=O)NR^3-$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^3$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Aralkyl- oder Heteroaralkylgruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere Substituenten $R^2$ auch miteinander ein mono- oder polycyclisches, aliphatisches, heteroaliphatisches, aromatisches oder heteroaromatisches Ringsystem bilden;

$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere Substituenten $R^3$ auch miteinander ein mono- oder polycyclisches, aliphatisches, heteroaliphatisches, aromatisches oder heteroaromatisches Ringsystem bilden.

2. Verbindung gemäß Anspruch 1, **dadurch gekennzeichnet, dass** sich die Gruppe $Ar^a$ und/oder $Ar^b$ in Formel (I) von einer Donorgruppe gemäß Symbol HL unterscheidet.

3. Verbindung gemäß Anspruch 1 oder 2, **dadurch gekennzeichnet, dass** die Donorgruppe HL eine Gruppe umfasst, bevorzugt für eine Gruppe steht, die ausgewählt ist aus den Formeln (H-1) bis (H-3)

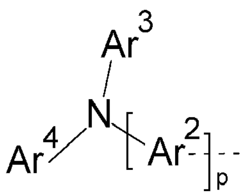

Formel (H-1)

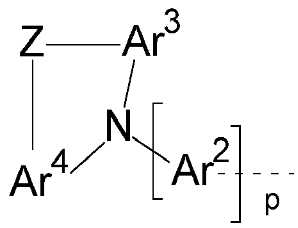

Formel (H-2)

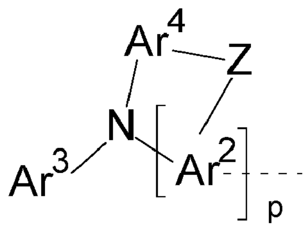

Formel (H-3)

, wobei die gestrichelte Bindung die Anbindungsposition markiert und $Ar^2$, $Ar^3$, $Ar^4$ jeweils unabhängig eine Arylgruppe mit 6 bis 40 C-Atomen oder eine Heteroarylgruppe mit 3 bis 40 C-Atomen ist, welche jeweils durch einen oder mehrere Reste $R^1$ substituiert sein kann;

p 0, 1, 2, 3, 4, 5 oder 6, vorzugsweise 0, 1, 2 oder 3 und besonders bevorzugt 0, 1 oder 2 ist und

Z für $CR^1_2$, $SiR^1_2$, C=O, N-$Ar^1$, $BR^1$, $PR^1$, $POR^1$, SO, $SO_2$, Se, O oder S, vorzugsweise $CR^1_2$, N-$Ar^1$, O oder S steht, wobei der Rest $R^1$ die in Anspruch 1 genannte Bedeutung aufweist und $Ar^1$ ein aromatisches oder heteroaromatisches Ringsystem mit 5 bis 60 aromatischen Ringatomen, das jeweils mit einem oder mehreren Resten $R^1$ substituiert sein kann, eine Aryloxygruppe mit 5 bis 60 aromatischen Ringatomen, die mit einem

oder mehreren Resten $R^1$ substituiert sein kann, oder einer Aralkylgruppe mit 5 bis 60 aromatischen Ringatomen, die jeweils mit einem oder mehreren Resten $R^1$ substituiert sein kann, darstellt, wobei optional zwei oder mehr, vorzugsweise benachbarte Substituenten $R^1$ ein mono- oder polycyclisches, aliphatisches, heteroaliphatisches, aromatisches oder heteroaromatisches Ringsystem m bilden können, das mit einem oder mehreren Resten $R^3$ substituiert sein kann.

**4.** Verbindung gemäß mindestens einem der vorhergehenden Ansprüche, **dadurch gekennzeichnet, dass** die Donorgruppe HL eine Gruppe umfasst, bevorzugt für eine Gruppe steht, die ausgewählt ist aus den Formeln (H-4) bis (H-26)

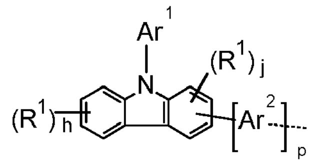

Formel (H-4)

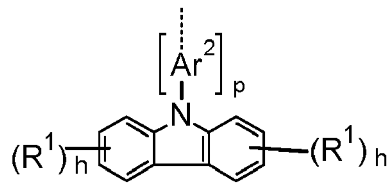

Formel (H-5)

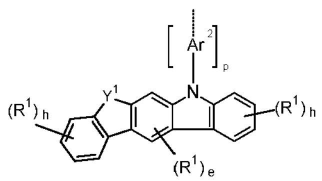

Formel (H-6)

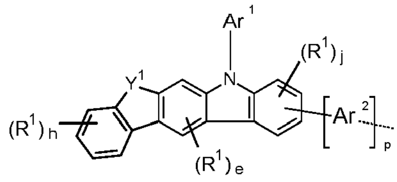

Formel (H-7)

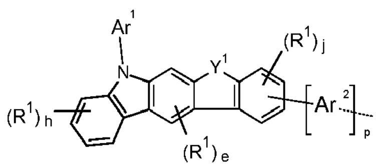

Formel (H-8)

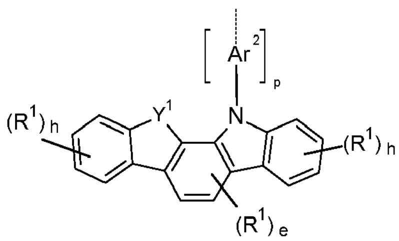

Formel (H-9)

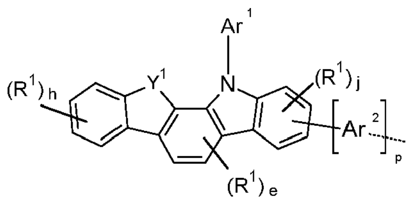

Formel (H-10)

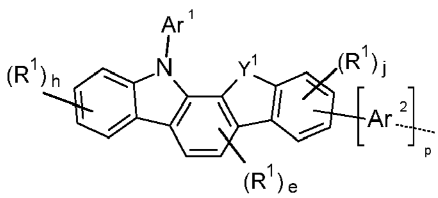

Formel (H-11)

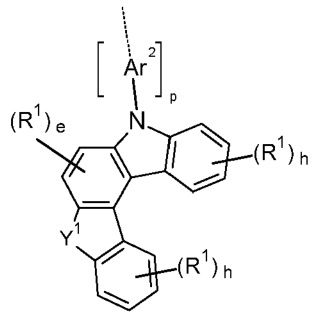

Formel (H-12)

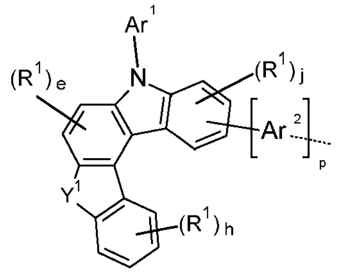

Formel (H-13)

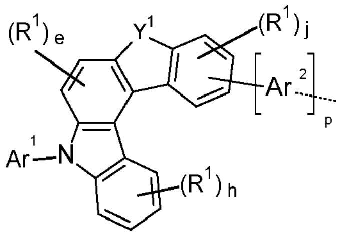

Formel (H-14)

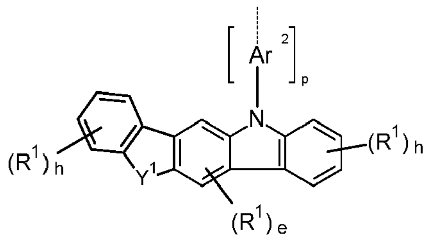

Formel (H-15)

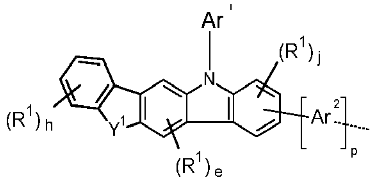

Formel (H-16)

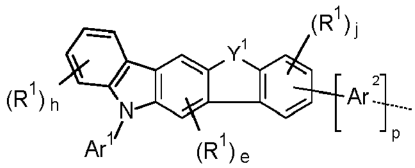

Formel (H-17)

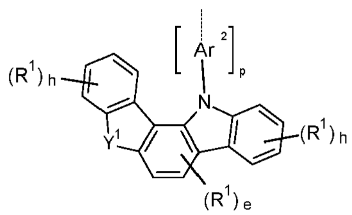

Formel (H-18)

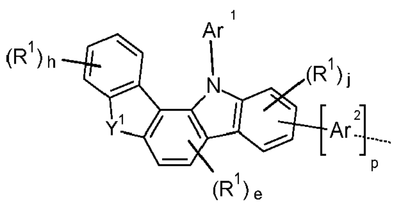

Formel (H-19)

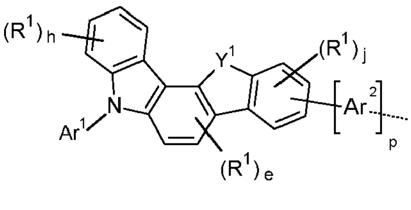

Formel (H-20)

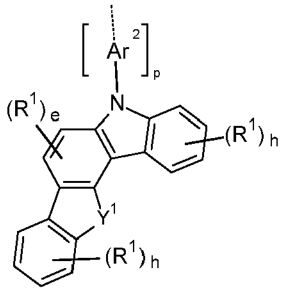

Formel (H-21)

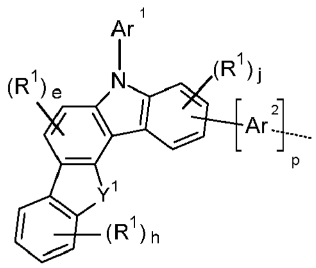

Formel (H-22)

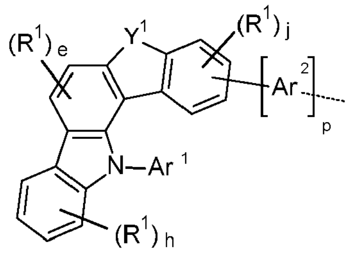

Formel (H-23)

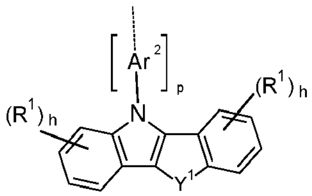

Formel (H-24)

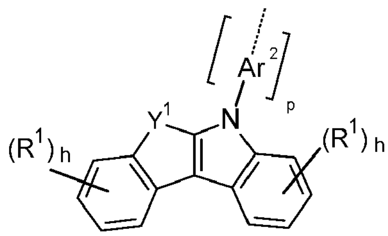

Formel (H-25)

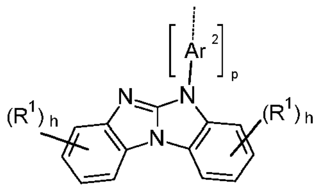

Formel (H-26)

, wobei $Y^1$ O, S, $C(R^1)_2$ oder $NAr^1$ darstellt, die gestrichelte Bindung die Anbindungsposition markiert, e 0, 1 oder 2 ist, j 0, 1, 2 oder 3 ist, h 0, 1, 2, 3 oder 4 ist, p 0, 1, 2, 3, 4, 5 oder 6, vorzugsweise 0, 1, 2 oder 3 und besonders bevorzugt 0, 1 oder 2 ist, $Ar^1$ und $Ar^2$ die in Anspruch 2 und $R^1$ die in Anspruch 1 genannte Bedeutung aufweist.

**5.** Verbindung gemäß Anspruch 3 oder Anspruch 4, **dadurch gekennzeichnet, dass** die Gruppe $Ar^2$ für eine verbindende Struktur der Formel (LAr-1) steht

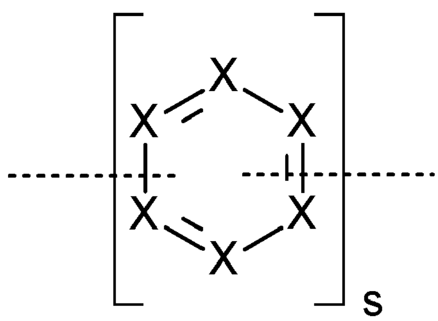

Formel (LAr-1)

wobei X bei jedem Auftreten gleich oder verschieden N oder $CR^1$, vorzugsweise $CR^1$, oder C ist falls an X eine Gruppe bindet;

die gestrichelte Bindung die Anbindungsposition markiert und s für 0, 1, 2, 3, 4, 5 oder 6, vorzugsweise für 0, 1, 2 oder 3, besonders bevorzugt für 0, 1 oder 2 und speziell bevorzugt für 0 oder 1 steht, wobei der Rest $R^1$ die in Anspruch 1 genannte Bedeutung aufweist.

**6.** Verbindungen gemäß mindestens einem der vorhergehenden Ansprüche, **dadurch gekennzeichnet, dass** die Akzeptorgruppe für eine Gruppe steht, die durch die Formel (QL) darstellbar ist,

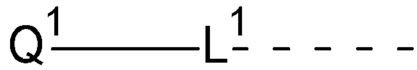

Formel (QL)

worin L' eine Bindung oder ein aromatisches oder heteroaromatisches Ringsystem mit 5 bis 60 aromatischen Ringatomen darstellt, welches durch einen oder mehrere Reste $R^1$ substituiert sein kann, und $Q^1$ eine elektronenziehende Gruppe ist, wobei der Rest $R^1$ die in Anspruch 1 genannte Bedeutung aufweist.

**7.** Verbindung gemäß Anspruch 5, **dadurch gekennzeichnet, dass** die Gruppe L' für eine verbindende Struktur der Formel (LAr-2) steht

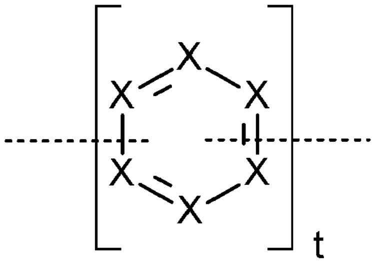

Formel (LAr-2)

wobei X bei jedem Auftreten gleich oder verschieden N oder $CR^1$, vorzugsweise $CR^1$, oder C ist falls an X eine Gruppe bindet;
die gestrichelte Bindung die Anbindungsposition markiert und t für 0, 1, 2, 3, 4, 5 oder 6, vorzugsweise für 0, 1, 2 oder 3, besonders bevorzugt für 0, 1 oder 2 und speziell bevorzugt für 0 oder 1 steht, wobei der Rest $R^1$ die in Anspruch 1 genannte Bedeutung aufweist.

**8.** Verbindungen gemäß Anspruch 6 oder 7, **dadurch gekennzeichnet, dass** die Gruppe $Q^1$ ausgewählt ist aus Strukturen der Formeln (Q-1), (Q-2), (Q-4), (Q-4), (Q-5), (Q-6), (Q-7), (Q-8), (Q-9) und/oder (Q-10)

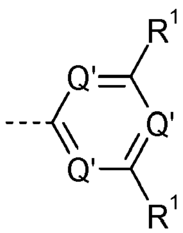

Formel (Q-1)

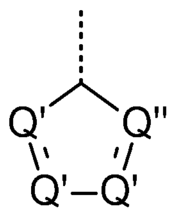

Formel (Q-2)

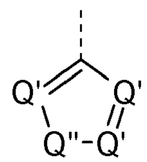

Formel (Q-3)

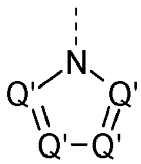

Formel (Q-4)

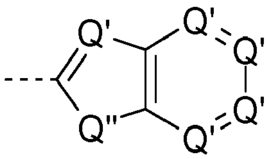

Formel (Q-5)

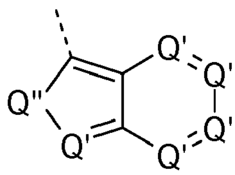

Formel (Q-6)

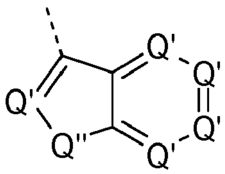

Formel (Q-7)

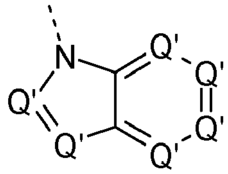

Formel (Q-8)

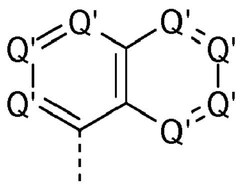

Formel (Q-9)

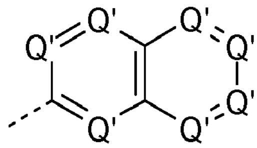

Formel (Q-10),

aufweist, wobei die gestrichelte Bindung die Anbindungsposition markiert,

Q’ bei jedem Auftreten gleich oder verschieden $CR^1$ oder N darstellt, und
Q" $NR^1$, O oder S darstellt;

wobei wenigstens ein Q’ gleich N und/oder wenigstens ein Q" gleich $NR^1$ ist und

$R^1$ wie in Anspruch 1 definiert ist.

**9.** Verbindungen gemäß mindestens einem der Ansprüche 6 bis 8, **dadurch gekennzeichnet, dass** die Gruppe $Q^1$ ausgewählt ist aus Strukturen der Formeln (Q-11), (Q-12), (Q-13), (Q-14) und/oder (Q-15)

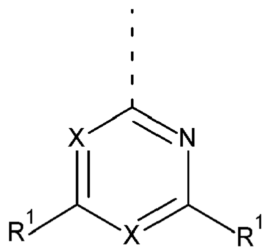

Formel (Q-11)

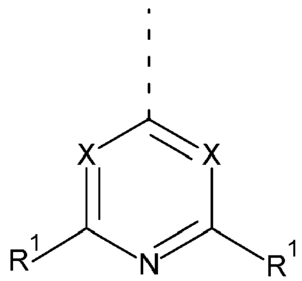

Formel (Q-12)

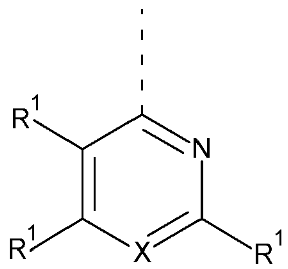

Formel (Q-13)

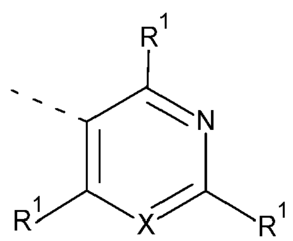

Formel (Q-14)

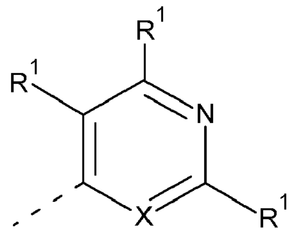

Formel (Q-15)

wobei das Symbol $R^1$ die zuvor in Anspruch 1 genannte Bedeutung aufweist, X N oder $CR^1$ ist und die gestrichelte Bindung die Anbindungsposition markiert, wobei X vorzugsweise ein Stickstoffatom darstellt.

**10.** Verbindungen gemäß mindestens einem der vorhergehenden Ansprüche 6 bis 9, **dadurch gekennzeichnet, dass** die Gruppe $Q^1$ ausgewählt ist aus Strukturen der Formeln (Q-26), (Q-27), (Q-28), (Q-29) und/oder (Q-30),

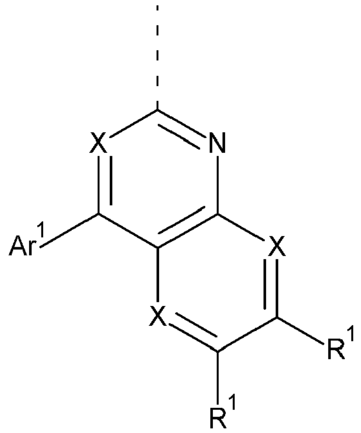

Formel (Q-16)

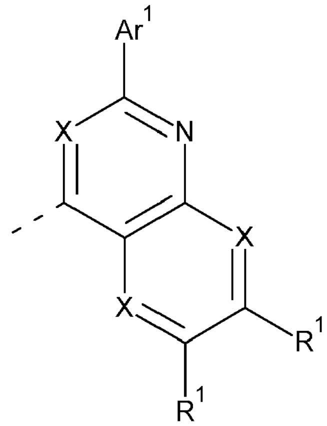

Formel (Q-17)

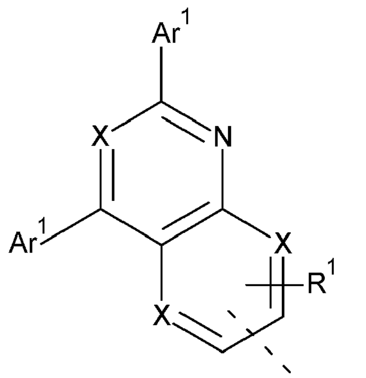

Formel (Q-28)

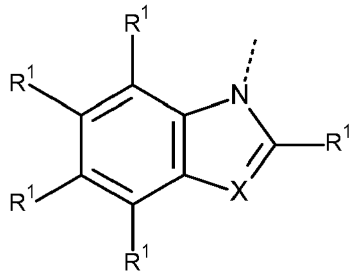

Formel (Q-29)

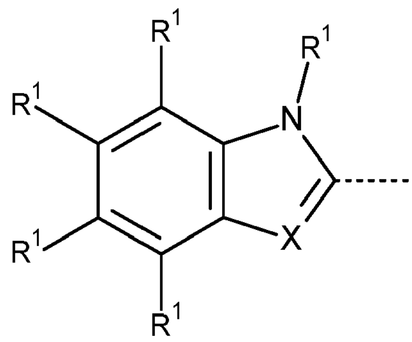

Formel (Q-30)

wobei X N oder $CR^1$ ist, das Symbol $R^1$ die zuvor in Anspruch 1 genannte Bedeutung aufweist und die gestrichelte Bindung die Anbindungsposition markiert, wobei X vorzugsweise ein Stickstoffatom darstellt und $Ar^1$ ein aromatisches oder heteroaromatisches Ringsystem mit 5 bis 60 aromatischen Ringatomen, das jeweils mit einem oder mehreren Resten $R^1$ substituiert sein kann, eine Aryloxygruppe mit 5 bis 60 aromatischen Ringatomen, die mit einem oder mehreren Resten $R^1$ substituiert sein kann, oder einer Aralkylgruppe mit 5 bis 60 aromatischen Ringatomen, die jeweils mit einem oder mehreren Resten $R^1$ substituiert sein kann, darstellt, wobei optional zwei oder mehr, vorzugsweise benachbarte Substituenten $R^1$ ein mono- oder polycyclisches, aliphatisches, heteroaliphatisches, aromatisches oder heteroaromatisches Ringsystem bilden können, das mit einem oder mehreren Resten $R^2$ substituiert sein kann.

**11.** Verbindungen gemäß mindestens einem der Ansprüche 6 oder 10, **dadurch gekennzeichnet, dass** die elektronenziehende Gruppe $Q^1$ ein aromatisches oder heteroaromatisches Ringsystem mit 5 bis 60 aromatischen Ringatomen ist, welches einen oder mehrere elektronenziehende Substituenten aufweist.

**12.** Verbindungen gemäß Anspruch 11, **dadurch gekennzeichnet, dass** der elektronenziehende Substituent ausgewählt ist aus F, fluorierte Alkylgruppen, $CF_3$, $C_nF_{2n+1}$, $C(=O)OR^1$, $C(=O)N(R^1)_2$, $NO_2$, CHO, $C(=O)R^1$, $S(=O)R^1$, $S(=O)_2R^1$ und/oder CN, vorzugsweise CN, F, Fluorierte Alkylgruppen, $CF_3$, $C_nF_{2n+1}$, wobei $R^1$ die in Anspruch 1 genannte Bedeutung aufweist und n eine ganze Zahl im Bereich von 1 bis 20, vorzugsweise 1 bis 10 und besonders bevorzugt 1 bis 5 darstellt.

**13.** Verbindungen gemäß mindestens einem der Ansprüche 5 bis 12, **dadurch gekennzeichnet, dass** die Differenz zwischen dem Index s in Formel (LAr-1) und dem Index t in Formel (LAr-2) höchstens 2, vorzugsweise höchstens 1 und speziell bevorzugt 0 ist.

**14.** Oligomer, Polymer oder Dendrimer enthaltend eine oder mehrere Verbindungen nach einem der Ansprüche 1 bis 13, wobei statt eines Wasserstoffatoms oder eines Substituenten ein oder mehrere Bindungen der Verbindungen zum Polymer, Oligomer oder Dendrimer vorhanden sind.

**15.** Zusammensetzung enthaltend wenigstens eine Verbindung nach einem oder mehreren der Ansprüche 1 bis 13 oder ein Oligomer, Polymer oder Dendrimer nach Anspruch 14 und wenigstens eine weitere Verbindung ausgewählt aus der Gruppe bestehend aus fluoreszierenden Emittern, phosphoreszierenden Emittern, Host-Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien.

**16.** Formulierung, enthaltend mindestens eine Verbindung nach einem oder mehreren der Ansprüche 1 bis 13 oder ein Oligomer, Polymer oder Dendrimer nach Anspruch 14 oder eine Zusammensetzung nach Anspruch 15 und mindestens ein Lösemittel.

**17.** Verwendung einer Verbindung nach einem oder mehreren der Ansprüche 1 bis 13, eines Oligomers, Polymers oder Dendrimers nach Anspruch 14 oder einer Zusammensetzung nach Anspruch 15 in einer elektronischen Vorrichtung als TADF-Material, Hostmaterial, Elektronentransportmaterial oder Lochleitmaterial.

**18.** Verfahren zur Herstellung einer Verbindung nach einem oder mehreren der Ansprüche 1 bis 13 oder eines Oligomers, Polymers und/oder Dendrimers nach Anspruch 14, **dadurch gekennzeichnet, dass** in einer Kupplungsreaktion

eine Verbindung, umfassend mindestens eine Donorgruppe, mit einer Akzeptorgruppe verbunden wird.

**19.** Elektronische Vorrichtung, enthaltend mindestens eine Verbindung nach einem oder mehreren der Ansprüche 1 bis 13 ein Oligomer, Polymer oder Dendrimer nach Anspruch 14 oder eine Zusammensetzung gemäß Anspruch 15, wobei die elektronische Vorrichtung bevorzugt ausgewählt ist aus der Gruppe bestehend aus organischen integrierten Schaltungen, organischen Feld-Effekt-Transistoren, organischen Dünnfilmtransistoren, organischen Solarzellen, organischen optischen Detektoren, organischen Photorezeptoren, organischen Feld-Quench-Devices, organischen Elektrolumineszenzvorrichtungen, wobei organische Elektrolumineszenzvorrichtungen ganz bevorzugt sind und wobei organische Elektrolumineszenzvorrichtungen aus der Gruppe der organischen lichtemittierenden Transistoren, organischen lichtemittierenden Dioden, organischen lichtemittierenden elektrochemischen Zellen und organischen Laserdioden ganz besonders bevorzugt sind; insbesondere bevorzugte organische Elektrolumineszenzvorrichtungen sind organische lichtemittierende Dioden und organische lichtemittierende elektrochemische Zellen.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2013173845 A
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- EP 676461 A
- WO 2004081017 A
- US 20070092753 A1
- WO 2004058911 A
- WO 2005084081 A
- WO 200508408 A
- WO 2006048268 A
- WO 2006117052 A
- DE 102007024850
- US 20070252517 A1
- US 20080193796 A1
- US 5121029 A
- US 20030027016 A1
- US 7326371 B2
- US 2006043858 A
- WO 2007114358 A
- WO 2008145239 A
- JP 3148176 B
- EP 1009044 A
- US 2004018383 A
- WO 2005061656 A1
- EP 0681019 B1
- WO 2004013073 A1
- US 5077142 A
- WO 2007065678 A
- US 20070205412 A1
- EP 0676461 A
- US 6562485 B
- US 7294849 B
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- WO 2016124304 A
- WO 2016015815 A
- WO 2016000803 A
- WO 2015117718 A
- WO 2015104045 A
- WO 2015036074 A
- WO 2005011013 A
- WO 2004013080 A
- WO 2004093207 A
- WO 2006005627 A
- WO 2010006680 A
- WO 2014015935 A
- WO 2005039246 A
- US 20050069729 A
- JP 2004288381 A
- EP 1205527 A
- WO 2008086851 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 005111172 A

- WO 2010015306 A
- EP 652273 A
- WO 2009062578 A
- WO 2010054729 A
- WO 2010054730 A
- US 20090136779 A
- WO 2010050778 A
- WO 2011042107 A
- WO 2011088877 A
- WO 2012143080 A
- WO 2012048781 A
- WO 2011116865 A
- WO 2011137951 A
- WO 2013064206 A
- WO 2014094963 A
- EP 14002104
- WO 2010108579 A
- WO 2005053051 A
- WO 2009030981 A
- WO 2004037887 A


**In der Beschreibung aufgeführte Nicht-Patentliteratur**


- **B. H. UOYAM et al.** *Nature,* 2012, vol. 492, 234
- **OUIBING PEI et al.** *Science,* 1995, vol. 296, 1086-1088
- **D. M. KOLLER et al.** *Nature Photonics,* 2008, 1-4
- **B. M. S. ARNOLD et al.** *Appl. Phys. Lett.,* 2008, vol. 92, 053301
- **A. SAGADEVAN et al.** *Green Chemistry,* 2015, vol. 17 (2), 1113-1119